# EUROPEAN PATENT SPECIFICATION

(11) **EP 3 723 777 B1**
(45) Date of publication and mention of the grant of the patent: **31.03.2021**
(21) Application number: 19755806.7
(22) Date of filing: 25.06.2019
(51) Int. Cl.: A61K 35/74, A61K 35/741, A61K 9/19, A61P 1/00, A61P 43/00

(54) **COMPOSITIONS COMPRISING BACTERIAL STRAINS**
ZUSAMMENSETZUNGEN MIT BAKTERIENSTÄMMEN
COMPOSITIONS COMPRENANT DES SOUCHES BACTÉRIENNES

(30) Priority: 25.06.2018 EP 18179641; 12.09.2018 GB 201814836; 09.04.2019 GB 201905000
(43) Date of publication of application: 21.10.2020
(73) Proprietor: 4D Pharma Research Limited, Aberdeen, Aberdeenshire AB25 2ZS (GB)
(72) Inventor: YUILLE, Samantha, Aberdeen, Aberdeenshire AB21 9LF (GB); REICHARDT, Nicole, Aberdeen, Aberdeenshire AB25 2ZS (GB); REID, Sarah, Aberdeen, Aberdeenshire AB25 2ZS (GB); AHMED, Suaad, Aberdeen, Aberdeenshire AB25 2ZS (GB); SAVIGNAC, Helene, Aberdeen, Aberdeenshire AB25 2ZS (GB); MULDER, Imke Elisabeth, Aberdeen, Aberdeenshire AB25 2ZS (GB); ETTORRE, Anna, Aberdeen, Aberdeenshire AB25 2ZS (GB)
(74) Representative: Brand Murray Fuller LLP
(86) International application number: PCT/EP2019/066905
(87) International publication number: WO 2020/002370

(56) References cited:
- WO-A1-2017/160711
- WO-A2-2017/091783
- BESSIS S ET AL: ""Bariatricus massiliensis" as a new bacterial species from human gut microbiota", NEW MICROBES AND NEW INFECTIONS, ELSEVIER LTD, GB , vol. 12 1 July 2016 (2016-07-01), pages 54-55, XP009509412, ISSN: 2052-2975, DOI: 10.1016/J.NMNI.2016.04.003 Retrieved from the Internet: URL:http://www.journals.elsevier.com/new-m icrobes-and-new-infections/ cited in the application
- LEY RUTH E ET AL: "Microbial ecology: human gut microbes associated with obesity", NATURE, LONDON, LONDON, vol. 444, no. 7122, 21 December 2006 (2006-12-21), pages 1022-1023, XP002492886, ISSN: 0028-0836, DOI: 10.1038/4441022A
- ELLEN LI ET AL: "Inflammatory Bowel Diseases Phenotype, C. difficile and NOD2 Genotype Are Associated with Shifts in Human Ileum Associated Microbial Composition", PLOS ONE, vol. 7, no. 6, 13 June 2012 (2012-06-13), page e26284, XP055524407, DOI: 10.1371/journal.pone.0026284
- YUILLE SAMANTHA ET AL: "Human gut bacteria as potent class I histone deacetylase inhibitors in vitro through production of butyric acid and valeric acid", PLOS ONE, PUBLIC LIBRARY OF SCIENCE, US , vol. 13, no. 7 27 July 2018 (2018-07-27), page e0201073, XP009508155, ISSN: 1932-6203, DOI: 10.1371/JOURNAL.PONE.0201073 Retrieved from the Internet: URL:https://epo.summon.serialssolutions.co m/2.0.0/link/0/eLvHCXMwtV1Nb9QwELWoeuGCKCC 6UCQfOICiFCdx4uQIFbSVEK3KcugpshO7jVTtrrpZp L3x05mxHW8KC6IHLlEySRwn8-KZ8ccbQl4rnuucFzr OVSVjLksWKyN43DSZaRJTMGNH8C-P-PRYXBxjtoYht eRG9l81DTLQNa6cvYe2Q6EggH3QOWxB67D9J727Xvm rVR8pR8QsMZXMYt7joH-DvnJ06liGZ7hkSja6X4MUu UOuO9XZ5Dv

## Description

### TECHNICAL FIELD

This invention is in the field of compositions comprising bacterial strains isolated from the mammalian digestive tract and the use of such compositions in the treatment of disease, as defined in the claims.

### BACKGROUND TO THE INVENTION

The human intestine is thought to be sterile *in utero*, but it is exposed to a large variety of maternal and environmental microbes immediately after birth. Thereafter, a dynamic period of microbial colonization and succession occurs, which is influenced by factors such as delivery mode, environment, diet and host genotype, all of which impact upon the composition of the gut microbiota, particularly during early life. Subsequently, the microbiota stabilizes and becomes adult-like [1]. The human gut microbiota contains more than 500-1000 different phylotypes belonging essentially to two major bacterial divisions, the Bacteroidetes and the Firmicutes [2]. The successful symbiotic relationships arising from bacterial colonization of the human gut have yielded a wide variety of metabolic, structural, protective and other beneficial functions. The enhanced metabolic activities of the colonized gut ensure that otherwise indigestible dietary components are degraded with release of by-products providing an important nutrient source for the host. Similarly, the immunological importance of the gut microbiota is well-recognized and is exemplified in germfree animals which have an impaired immune system that is functionally reconstituted following the introduction of commensal bacteria [3-5].

Dramatic changes in microbiota composition have been documented in gastrointestinal disorders such as inflammatory bowel disease (IBD). For example, the levels of *Clostridium* cluster XIVa bacteria are reduced in IBD patients whilst numbers of *E. coli* are increased, suggesting a shift in the balance of symbionts and pathobionts within the gut [6-9]. Interestingly, this microbial dysbiosis is also associated with imbalances in T effector cell populations.

In recognition of the potential positive effect that certain bacterial strains may have on the animal gut, various strains have been proposed for use in the treatment of various diseases (see, for example, [10-13]). Also, certain strains, including mostly *Lactobacillus* and *Bifidobacterium* strains, have been proposed for use in treating various inflammatory and autoimmune diseases that are not directly linked to the intestines (see [14] and [15] for reviews). However, the relationship between different diseases and different bacterial strains, and the precise effects of particular bacterial strains on the gut and at a systemic level and on any particular types of diseases, are poorly characterised.

There is a requirement in the art for new methods of treating diseases. There is also a requirement for the potential effects of gut bacteria to be characterised so that new therapies using gut bacteria can be developed.

WO2017/160711 describes modulation of the gut microbiome to treat mental disorders or diseases of the central nervous system.

Bessis et al. (2016, New Microbe and New Infect 12: 54-55) describes *Bariatricus massiliensis* as a new bacterial species from the human gut microbiota.

### SUMMARY OF THE INVENTION

The inventors have developed new compositions comprising a bacterial strain of the genus *Bariatricus* that can be used in therapy. In particular, the inventors have developed new compositions comprising a strain of the genus *Bariatricus* for use in treating and preventing diseases or conditions mediated by elevated histone deacetylase (HDAC) activity. The inventors have identified that bacterial strains from the genus *Bariatricus* can be effective for reducing histone deacetylase activity. Histone deacetylase activity has been shown to mediate pathological symptoms in an array of diseases and conditions including but not limited to autoimmune or inflammatory diseases and conditions including, but not limited to, Graft-versus-host disease (GVHD), inflammatory bowel diseases, such as Crohn's disease, neurodegenerative diseases, such as Parkinson's disease, brain injury, such as stroke, and a range of cancers. As such, the compositions of the invention may have pleiotropic benefits in the treatment or prevention of multiple diseases mediated at least in part by HDAC activity. In some embodiments, the compositions of the invention are for use in the treatment of prevention of diseases mediated by increased HDAC activity.

As described in the examples, oral administration of compositions comprising *Bariatricus* may reduce the activity of histone deacetylase in models of disease. Also, as described in the examples, oral administration of compositions comprising *Bariatricus* may reduce hyperactivity in mice models of disease. In certain embodiments, the compositions of the invention may be for use in the treatment or prevention of a disease or condition associated with hyperactivity. The compositions may be for use in the treatment or prevention of hyperactivity. The compositions may be for use in the treatment or prevention of hyperactivity associated with behavioural disorders, such attention deficit hyperactive disorder. Therefore, the inventors have identified compositions effective in the prevention or treatment of diseases mediated by HDAC activity and compositions effective in the treatment or prevention of behavioural disorders. Behavioural disorders suitable for treatment with compositions of the invention may or may not be mediated in part by HDAC activity.

In a first embodiment, the invention provides a composition comprising a bacterial strain of the genus *Bariatricus*, for use in therapy.

In particular embodiments, the invention provides a composition comprising a bacterial strain of the genus *Bariatricus*, for use in treating and preventing diseases mediated by elevated HDAC activity. The inventors have identified that treatment with bacterial strains from this genus can reduce the activity of HDAC, which can provide clinical benefits in the treatment of diseases mediated by HDAC activity. In some embodiments, the compositions of the invention have been found to be particularly beneficial in reducing Class I HDAC activity. Class I HDACs are ubiquitously expressed and most commonly reside in the nucleus. Class I HDACs deacetylate histone lysine residues to restore positive charge to the histone, thereby increasing electrostatic binding between histones and DNA. HDAC activity therefore increases chromatin compaction causing downregulation of the expression of genes at the underlying DNA sequence. HDACs also have additional regulatory effects by modifying non-histone protein targets. The inhibition of the acetylation of non-histone protein targets may be beneficial in the treatment or prevention of other aspects of disease not directly related to the control of gene expression by chromatin expansion. In certain embodiments, the compositions of the invention can therefore be used to regulate target gene expression.

In particular embodiments, the invention provides a composition comprising a bacterial strain of the genus *Bariatricus*, for use in a method of treating or preventing a disease or condition selected from the group consisting of: a neurodegenerative disease, such as Alzheimer's disease, Huntington's disease or Parkinson's disease; brain injury, such as stroke; behavioural or psychiatric disorders, such as attention deficit hyperactivity disorder, obsessive compulsive disorder, anxiety disorder, bipolar disorder, or post-traumatic stress disorder; an inflammatory or autoimmune disease, such as asthma, arthritis, psoriasis, multiple sclerosis, diabetes, allograft rejection, graft-versus-host disease, or an inflammatory bowel disease, such as Crohn's disease; or cancer, such as prostate cancer, colorectal cancer, breast cancer, lung cancer, liver cancer or gastric cancer. The effect shown for the bacterial strains from the genus *Bariatricus* on HDAC activity may provide therapeutic benefits for diseases and conditions mediated by aberrant HDAC activity, such as those listed above. In certain embodiments, the compositions of the invention may provide therapeutic benefits in the treatment of diseases or conditions with increased HDAC expression. In certain embodiments, the compositions of the invention may provide therapeutic benefits in the treatment of diseases or conditions with increased HDAC activity. Furthermore, the inventors have identified that treatment with a bacterial strain of the genus *Bariatricus* can reduce the activation of proinflammatory molecules, such as IL-6, by LPS. Chronic inflammation induced by IL-6 can ultimately lead to cell death. Therefore, the bacterial strains of the invention may be particularly useful in the treatment or prevention of inflammatory or autoimmune disorders. In some embodiments, the bacterial strains are useful in the treatment of inflammatory or autoimmune disorders characterised by the enhanced activation of IL-6. Furthermore, the inventors have identified that treatment with a bacterial strain of the genus *Bariatricus* can increase MAP2 (Microtubule-associated protein 2) activation. MAP2 is a gene associated with neuronal differentiation of MAP2 and is thought to be essential for microtubule formation in neuritogenesis, so compositions of the invention may be particularly useful for treating neurodegenerative diseases or brain injuries. In some embodiments, the compositions of the invention are for use in treating neurodegenerative diseases by activating or increasing the levels of MAP2. Furthermore, the inventors have identified that treatment with a bacterial strain of the genus *Bariatricus* can alter the expression of IDO1 in the intestine. IDOl expression in the colon is associated with amelioration of disease in a mouse model of colitis. Therefore, the bacterial strains of the invention may be particularly useful in the treatment or prevention of inflammatory bowel diseases. Furthermore, the inventors have identified that treatment with a bacterial strain of the genus *Bariatricus* can alter the expression of a number of genes in the brain, e.g. increase the expression of BDNF in the hippocampus and prefrontal cortex. BDNF is essential for adult synaptic plasticity and the formation of memories and a decrease in the levels of BDNF is observed in Alzheimer's and Huntington's patients. Therefore, the bacterial strains of the invention may be particularly useful in the treatment or prevention of neurodegenerative diseases, e.g. Alzheimer's and Huntington's disease.

In some embodiments, bacterial strains from the genus *Bariatricus* may provide therapeutic benefits in the treatment of behavioural disorders selected from the list consisting of: attention deficit hyperactive disorder, oppositional defiant disorder and conduct disorder. The inventors have identified that treatment with *Bariatricus* strains reduce hyperactivity in mice, which is a symptom of behavioural disorders such as ADHD. The strains of the invention may therefore be useful in the treatment or prevention of behavioural disorders, in particular in the treatment or prevention of behavioural disorders associated with hyperactivity, such as ADHD. In certain embodiments, the compositions of the invention are for use in the treatment or prevention of hyperactivity in a subject. In certain embodiments, the invention provides a composition comprising a bacterial strain of the species *Bariatricus massiliensis* for use in the treatment or prevention of behavioural disorders. In preferred embodiments, the invention provides a composition comprising a bacterial strain of the species *Bariatricus massiliensis* for use in the treatment or prevention of ADHD

In some embodiments, bacterial strains from the genus *Bariatricus* may provide therapeutic benefits in the treatment or prevention of GVHD. The inventors have identified that treatment with *Bariatricus* strains increase survival from GVHD in mice. The strains of the invention may therefore be useful in the treatment or prevention of GVHD. In certain embodiments, the compositions of the invention are for use in the treatment or prevention of GVHD mediated at least in part by HDAC activity. In certain embodiments, the compositions of the invention are for use in the treatment or prevention of GVHD in a subject.

In some embodiments, the invention provides a composition comprising a bacterial strain of the genus *Bariatricus*, for use in a method of treating or preventing a neurodegenerative disease mediated by elevated HDAC activity. In some embodiments, the compositions of the invention may be useful in the treatment or prevention of symptoms of neurodegenerative diseases mediated by HDAC activity. The inventors have identified that the strains of the invention inhibit HDAC activity. Histone acetylation and deacetylation are important epigenetic regulators of gene expression. Histone acetylation imbalance has been implicated in the pathogenesis of neurodegenerative diseases such as Alzheimer's disease, Huntington's disease and Parkinson's disease. In some embodiments, the strains of the invention are for use in the treatment or prevention of age-associated neurodegenerative diseases. In some embodiments, the compositions of the invention are for use in the treatment or prevention of age-onset neurodegenerative diseases, such as age-onset Parkinson's disease or age-onset Alzheimer's disease. In certain embodiments, the invention provides a composition comprising a bacterial strain of the species *Bariatricus massiliensis* for use in the treatment or prevention of neurodegenerative disease. In preferred embodiments, the invention provides a composition comprising a bacterial strain of the species *Bariatricus massiliensis* for use in the treatment or prevention of Alzheimer's disease, Huntington's disease or Parkinson's disease.

In some embodiments the invention provides a composition comprising a bacterial strain of the genus *Bariatricus* for use in a method of treating or preventing an inflammatory bowel disease mediated by elevated HDAC activity. Inhibition of HDAC activity has been shown to suppress the production of proinflammatory cytokines in the gastrointestinal tract. Thus, the compositions of the invention may be useful in the treatment of inflammatory diseases. In particular, the compositions of the invention may be useful in the treatment or prevention of conditions associated with increased colonic proinflammatory cytokine pathogenesis. In some embodiments, the compositions of the invention are for use in the treatment or prevention of inflammatory bowel disease. In some embodiments, the compositions of the invention are for use in the treatment or prevention of ulcerative colitis. In some embodiments, the compositions of the invention are for use in the treatment or prevention of Crohn's disease. In certain embodiments, the invention provides a composition comprising a bacterial strain of the species *Bariatricus massiliensis* for use in the treatment or prevention of inflammatory disease. In preferred embodiments, the invention provides a composition comprising a bacterial strain of the species *Bariatricus massiliensis* for use in the treatment or prevention of colitis.

In certain embodiments of the invention, the compositions are for use in treating brain injury. The neuroprotective activity of the compositions of the invention and their ability to reduce levels of histone deacetylase activity (HDAC) may make them useful for treating brain injury. In preferred embodiments, the compositions of the invention are for use in treating stroke, such as treating brain injury resulting from a stroke. In certain embodiments, the invention provides a composition comprising a bacterial strain of the species *Bariatricus massiliensis* for use in the treatment or prevention of brain injury, in particular stroke.

In some embodiments, the compositions of the invention are for use in the treatment or prevention of cancer. Dysregulation of acetylation pathways in cancer have been implicated in cancer cell survival and tumour immune evasion. For example, HDAC mediated deacetylation of p53 reduces the stability and half-life of p53. Acetylated p53 binds and regulates the expression of cell cycle regulatory and pro-apoptotic genes with greater efficacy, reducing cancer cell growth and promoting apoptosis. Deacetylation of p53 may therefore inhibit apoptosis in cancer cells, increasing cancer cell survival. In certain embodiments, the compositions of the invention are for use in the treatment or prevention of cancers. In some embodiments, the compositions of the invention are for use in the treatment of cancers with non-mutated p53. In some embodiments, the compositions of the invention are for use in a method of increasing apoptosis in cancer cells. In some embodiments, the compositions of the invention are for use in a method of decreasing tumour immune evasion. In some embodiments, the compositions of the invention are for use in the treatment or prevention of cancers with increased HDAC-activity. In some embodiments, the compositions are for use as pro-apoptotic medicaments, for example for use in the treatment or prevention of cancers. In certain embodiments, the invention provides a composition comprising a bacterial strain of the species *Bariatricus massiliensis* for use in the treatment or prevention of cancer.

In further preferred embodiments, the invention provides a composition comprising a bacterial strain of the genus *Bariatricus*, for use in a method of treating or preventing cancer, such as breast, lung or liver cancer. In certain embodiments, the composition is for use in a method of reducing tumour size or preventing tumour growth in the treatment of cancer. In certain embodiments, the invention provides a composition comprising a bacterial strain of the species *Bariatricus massiliensis*, for use in the treatment of cancer.

In certain embodiments, the compositions of the invention are for use in a method of reducing histone deacetylase activity in the treatment or prevention of a disease or condition mediated by elevated histone deacetylase activity.

In certain embodiments, the composition is for use in a patient with elevated histone deacetylase activity. In certain embodiments, the composition is for use in a patient with elevated Class I HDAC activity. The effect on histone deacetylase activity shown for *Bariatricus* strains may be particularly beneficial for such patients.

The bacterial strain in the composition is of *Bariatricus.* In certain embodiments of the invention, the bacterial strain in the composition is of *Bariatricus massiliensis.* Closely related strains may also be used, such as bacterial strains that have a 16s rRNA gene sequence that is at least 95%, 96%, 97%, 98%, 99%, 99.5% or 99.9% identical to SEQ ID NO:1 or SEQ ID NO:2. Preferably, the bacterial strain for use in the invention has the 16s rRNA gene sequence represented by SEQ ID NO: 1. Preferably, the bacterial strain for use in the invention has the 16s rRNA gene sequence represented by SEQ ID NO: 2.

In certain embodiments, the composition of the invention is for oral administration. Oral administration of the strains of the invention can be effective for treating diseases and conditions mediated by HDAC activity. In certain embodiments, oral administration of the strains of the invention can be effective for treating diseases and conditions mediated by elevated Class I HDAC activity. Also, oral administration is convenient for patients and practitioners and allows delivery to and /or partial or total colonisation of the intestine.

In certain embodiments, the composition of the invention comprises one or more pharmaceutically acceptable excipients or carriers.

In certain embodiments, the composition of the invention comprises a bacterial strain that has been lyophilised. Lyophilisation is an effective and convenient technique for preparing stable compositions that allow delivery of bacteria.

In certain embodiments, the invention provides a food product comprising the composition as described above.

Additionally, a method of treating or preventing a disease or condition mediated by HDAC activity, comprising administering a composition comprising a bacterial strain of the genus *Bariatricus* is disclosed.

In developing the above invention, the inventors have identified and characterised bacterial strains that are particularly useful for therapy. The *Bariatricus massiliensis* strains of the invention are shown to be effective for treating the diseases described herein, such as stroke, ADHD and GVHD. Therefore, in another aspect, the invention provides a cell of the *Bariatricus massiliensis* strain deposited under accession number NCIMB 43042. The invention also provides compositions comprising such cells, or biologically pure cultures of such cells. The invention also provides a cell of the *Bariatricus massiliensis* strain deposited under accession number NCIMB 43042 for use in therapy, in particular for the diseases described herein. Disclosed is a cell of the *Bariatricus massiliensis* strain deposited under accession number NCIMB 43171. Disclosed are also compositions comprising such cells, or biologically pure cultures of such cells. The invention also provides a cell of the *Bariatricus massiliensis* strain deposited under accession number NCIMB 43171 for use in therapy, in particular for the diseases described herein.

### BRIEF DESCRIPTION OF DRAWINGS

**Figure 1** Whole-cell histone deacetylase activity (Figure 1A), Cell lysate histone deacetylase activity (Figure 1B)
**Figure 2** Levels of metabolite production in *Bariatricus massiliensis* strain 43042
**Figure 3** Inhibition of Class I HDACs (Figure 3A); inhibition of HDAC1 (Figure 3B); inhibition of HDAC2 (Figure 3C); inhibition of HDAC3 (Figure 3D)

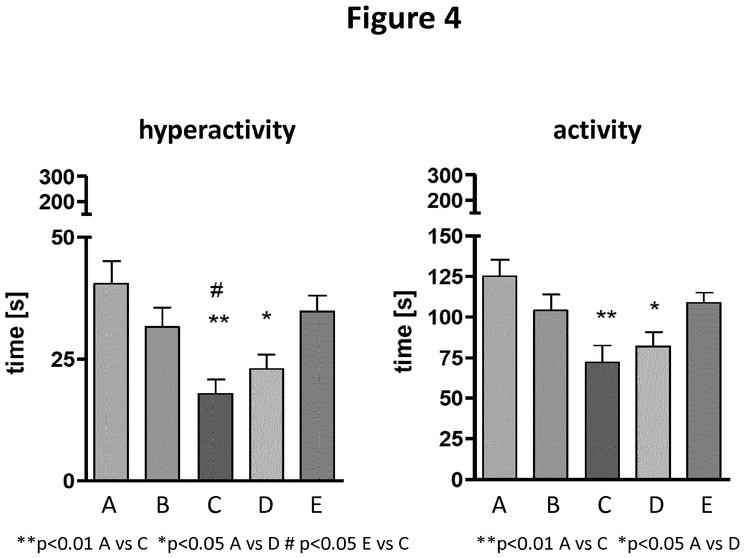

**Figure 4** Strain 43042 reduces hyperactivity in mice
**Figure 5** GVHD body weight data in mice models administered *Bariatricus massiliensis* strain 43171. Animals were weighed daily for the duration of the study Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 6** GVHD body weight data in mice models administered *Bariatricus massiliensis* strain 43171. Animals were weighed daily for the duration of the study, and percent body weight change relative to Day -14 is shown. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 7** GVHD body weight data in mice models administered *Bariatricus massiliensis* strain 43171. Animals were weighed daily for the duration of the study, and percent body weight change relative to Day 0 is shown. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 8** GVHD body weight data in mice models administered *Bariatricus massiliensis* strain 43171, accounting for group attrition, the body weight with which an animal died was carried forward for the duration of the study for animals found dead or euthanized for all groups except Group 2. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 9** GVHD body weight data in mice models administered tacrolimus (FK506) ***: p≤0.005.
**Figure 10** Animal survival in mice models administered with *Bariatricus massiliensis* strain 43171
**Figure 11** Animal survival in mice models administered with tacrolimus (FK506)
**Figure 12** GVHD clinical scores in mice models administered *Bariatricus massiliensis* strain 43171. Animals were assigned a clinical GVHD score daily from Days 0 to 30. Area under the curve (AUC) was calculated using the trapezoidal transformation rule and is shown in figure inset. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.

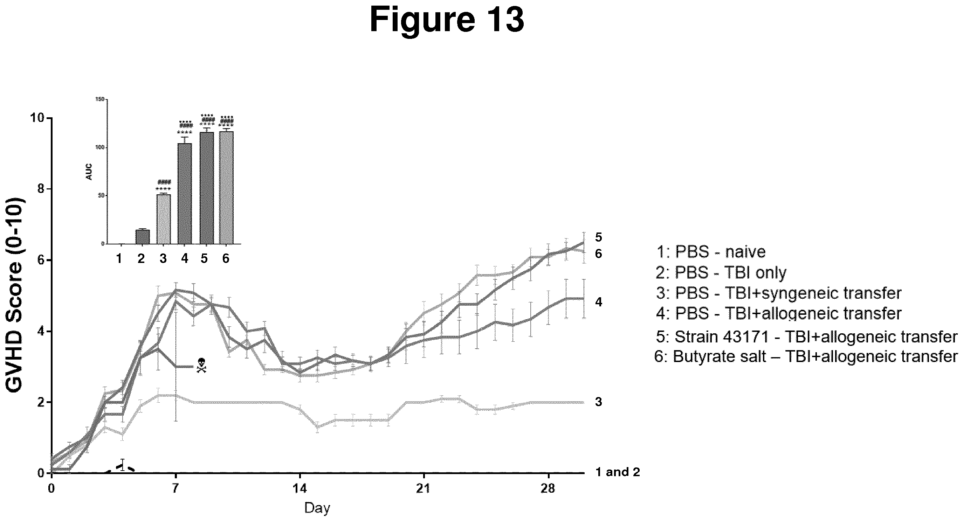

**Figure 13** GVHD clinical scores in mice models administered *Bariatricus massiliensis* strain 43171. Animals were assigned a clinical GVHD score daily from Days 0 to 30. To account for group attrition, the GVHD score with which an animal died was carried forward for the duration of the study for animals found dead or euthanized for all groups except Group 2. Area under the curve (AUC) was calculated using the trapezoidal transformation rule and is shown in figure inset. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 14** (A) posture, (B) activity, (C) fur texture, (D) skin integrity, and (E) weight loss used in composite GVHD scores in mice models administered *Bariatricus massiliensis* strain 43171.
**Figure 15** GVHD clinical scores in mice models administered tacrolimus (FK506)
**Figure 16** Colitis severity scores in mice models administered *Bariatricus massiliensis* strain 43171. Animals underwent video endoscopy on Day 29 to assess colon inflammation. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 17** Representative colon endoscopy images.
**Figure 18** Plasma citrulline levels in mice administered *Bariatricus massiliensis* strain 43171. Blood was collected prior to euthanasia from all surviving animals and was processed for plasma; plasma citrulline was assessed in duplicate by ELISA. Plasma was diluted 1:10 for analysis. Asterisk indicates significance as compared to Group 1; hash indicates significance as compared to Group 2; and dot indicates significance as compared to Group 3; unless otherwise indicated. *p<0.05, **p<0.01, ***p<0.005, ****p<0.001. Data is presented as mean ± SEM. n=8-12 per group.
**Figure 19** Levels of IL-6 secretion.
**Figure 20** Activation of MAP2.
**Figure 21** IDOl expression in the ileum (A) and colon (B) of mice administered *Bariatricus.* *p<0.05.
**Figure 22** Expression of glucocorticoid receptor (A), mineralocorticoid receptor (B), BDNF (C), Grin 2B (D), CRH (E), CFR1 (F), CD11b (G) and GABA A2 (H) in the hippocampus of mice administered *Bariatricus.* *p<0.05.
**Figure 23** Expression of oxytocin receptor (A), glucocorticoid receptor (B), mineralocorticoid receptor (C), Grin 2A (D) and Grin 2B (E) in the amygdala of mice administered *Bariatricus.* *p<0.05.
**Figure 24** Expression of BDNF (A), CRFR1 (B) and mineralocorticoid receptor (C) in the prefrontal cortex of mice administered *Bariatricus.* *p<0.05, **p<0.01.

### DISCLOSURE OF THE INVENTION

### Bacterial strains

The compositions of the invention comprise a bacterial strain of the genus *Bariatricus*, as defined in the claims. The examples demonstrate that bacteria of this genus are useful for treating or preventing diseases and conditions mediated by HDAC activity. The preferred bacterial strains are of the species *Bariatricus massiliensis.*

An example of a *Bariatricus* strain for use in the invention is a strain of the species *Bariatricus massiliensis.* The *Bariatricus* are Gram-reaction-positive, rod-shaped obligate anaerobes (Bessis et al 2016 New Microbe and New Infect 12: 54-55). *Bariatricus massiliensis* may be isolated from the human gut. The 16S rRNA gene sequences of the *Bariatricus massiliensis* strains used in the examples are disclosed herein as SEQ ID NO:1 and SEQ ID NO:2. A further exemplary *Bariatricus massiliensis* strain is described in Bessis et al (2016 New Microbe and New Infect 12: 54-55).

The *Bariatricus massiliensis* bacteria deposited under accession number NCIMB 43042 was tested in the Examples and is referred to herein as strain 43042. A 16S rRNA gene sequence for the 43042 strain that was tested is provided in SEQ ID NO:1. Strain 43042 was deposited with the international depositary authority NCIMB, Ltd. (Ferguson Building, Aberdeen, AB21 9YA, Scotland) by 4D Pharma Research Limited (Life Sciences Innovation Building, Aberdeen, AB25 2ZS, Scotland) on 18/05/2018 as "*Bariatricus massiliensis*" and was assigned accession number NCIMB 43042. The *Bariatricus massiliensis* bacteria deposited under accession number NCIMB 43171 was tested in the Examples and is referred to herein as strain 43171. A 16S rRNA gene sequence for strain 43171 that was tested is provided in SEQ ID NO:2. Strain 43171 was deposited with the international depositary authority NCIMB, Ltd. (Ferguson Building, Craibstone Estate, Bucksburn, Aberdeen, AB21 9YA Scotland) by 4D Pharma Research Limited (Life Sciences Innovation Building, Cornhill Road, Aberdeen, AB25 2ZS, Scotland) on 20/08/2018 as "*Bariatricus massiliensis*" and was assigned accession number NCIMB 43171.

Bacterial strains closely related to the strains tested in the Examples are also expected to be effective for treating or preventing diseases and conditions mediated by HDAC activity. In certain embodiments, the bacterial strain for use in the invention has a 16s rRNA gene sequence that is at least 95%, 96%, 97%, 98%, 99%, 99.5% or 99.9% identical to SEQ ID NO:1. Preferably, the bacterial strain for use in the invention has the 16s rRNA gene sequence represented by SEQ ID NO:1. In certain embodiments, the bacterial strain for use in the invention has a 16s rRNA gene sequence that is at least 95%, 96%, 97%, 98%, 99%, 99.5% or 99.9% identical to SEQ ID NO:2. Preferably, the bacterial strain for use in the invention has the 16s rRNA gene sequence represented by SEQ ID NO:2.

Bacterial strains that are biotypes of the bacterium deposited under accession number NCIMB 43042 or NCIMB 43171 are also expected to be effective for treating or preventing diseases and conditions mediated HDAC activity. A biotype is a closely related strain that has the same or very similar physiological and biochemical characteristics.

Strains that are biotypes of a bacterium deposited under accession number NCIMB 43042 or NCIMB 43171 and that are suitable for use in the invention may be identified by sequencing other nucleotide sequences for a bacterium deposited under accession number NCIMB 43042 or NCIMB 43171. For example, substantially the whole genome may be sequenced and a biotype strain for use in the invention may have at least 95%, 96%, 97%, 98%, 99%, 99.5% or 99.9% sequence identity across at least 80% of its whole genome (e.g. across at least 85%, 90%, 95% or 99%, or across its whole genome). Other suitable sequences for use in identifying biotype strains may include hsp60 or repetitive sequences such as BOX, ERIC, (GTG)₅, or REP [16]. Biotype strains may have sequences with at least 95%, 96%, 97%, 98%, 99%, 99.5% or 99.9% sequence identity to the corresponding sequence of a bacterium deposited under accession number NCIMB 43042 or NCIMB 43171.

Alternatively, strains that are biotypes of a bacterium deposited under accession number NCIMB 43042 or NCIMB 43171 and that are suitable for use in the invention may be identified by using the accession number NCIMB 43042 or NCIMB 43171, and restriction fragment analysis and/or PCR analysis, for example by using fluorescent amplified fragment length polymorphism (FAFLP) and repetitive DNA element (rep)-PCR fingerprinting, or protein profiling, or partial 16S or 23S rDNA sequencing. In preferred embodiments, such techniques may be used to identify other *Bariatricus* strains.

In certain embodiments, strains that are biotypes of a bacterium deposited under accession number NCIMB 43042 or NCIMB 43171 and that are suitable for use in the invention are strains that provide the same pattern as a bacterium deposited under accession number NCIMB 43042 or NCIMB 43171 when analysed by amplified ribosomal DNA restriction analysis (ARDRA), for example when using Sau3AI restriction enzyme (for exemplary methods and guidance see, for example, [17]. Alternatively, biotype strains are identified as strains that have the same carbohydrate fermentation patterns as a bacterium deposited under accession number NCIMB 43042 or NCIMB 43171.

Other *Bariatricus* strains that are useful in the compositions and uses of the invention, such as biotypes of a bacterium deposited under accession number NCIMB 43042 or NCIMB 43171, may be identified using any appropriate method or strategy, including the assays described in the examples. For instance, strains for use in the invention may be identified by administering the bacteria to the HDAC activity assay and assessing HDAC activity inhibition. Bacterial strains with comparable HDAC inhibitory activity to NCIMB 43042 are suitable for use in the invention. In particular, bacterial strains that have similar growth patterns, metabolic type and/or surface antigens to a bacterium deposited under accession number NCIMB 43042 may be useful in the invention. A useful strain will have comparable HDAC inhibitory activity and/or comparable effects on the reduction of hyperactivity in the assays used in the Examples to the NCIMB 43042 strain, which may be identified by using the culturing and administration protocols described in the Examples. In other instances, strains for use in the invention may be identified by administering the bacteria to the GVHD models described in the Examples and assessing efficacy in the treatment of GVHD. Bacterial strains with comparable activity to NCIMB 43171 with respect to the treatment of GVHD under conditions set out in the Examples are suitable for use in the invention.

A particularly preferred strain of the invention is the *Bariatricus massiliensis* strain deposited under accession number NCIMB 43042. This is the exemplary 43042 strain tested in the examples and shown to be effective for reducing HDAC activity and reducing hyperactivity. Therefore, the invention provides a cell, such as an isolated cell, of the *Bariatricus massiliensis* strain deposited under accession number NCIMB 43042. The invention also provides a composition comprising a cell of the *Bariatricus massiliensis* strain deposited under accession number NCIMB 43042. The invention also provides a biologically pure culture of the *Bariatricus massiliensis* strain deposited under accession number NCIMB 43042. The invention also provides a cell of the *Bariatricus massiliensis* strain deposited under accession number NCIMB 43042 for use in therapy, in particular for the diseases described herein.

A derivative of the strain deposited under accession number NCIMB 43042 may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable HDAC inhibitory activity to the original NCIMB 43042 strain. In particular, a derivative strain will elicit comparable effects on HDAC inhibitory activity or hyperactivity models shown in the Examples, which may be identified by using the culturing and administration protocols described in the Examples. A derivative of the NCIMB 43042 strain will generally be a biotype of the NCIMB 43042 strain.

Another particularly preferred strain of the invention is the *Bariatricus massiliensis* strain deposited under accession number NCIMB 43171. The exemplary strain 43171 tested in the examples is shown to be effective in the treatment of GVHD. Disclosed is a cell, such as an isolated cell, of the *Bariatricus massiliensis* strain 43171, or a derivative thereof. Disclosed is also a composition comprising a cell of the *Bariatricus massiliensis* strain 43171, or a derivative thereof. Disclosed is also a biologically pure culture of the *Bariatricus massiliensis* strain 43171. The invention also provides a cell of the *Bariatricus massiliensis* strain 43171, for use in therapy, in particular for the diseases described herein.

A derivative of the strain deposited under accession number NCIMB 43171 may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable activity to the original NCIMB 43171 strain with respect to the treatment of GVHD under conditions set out in the Examples. A derivative of the NCIMB 43171 strain will generally be a biotype of the NCIMB 43171 strain.

In preferred embodiments, the bacterial strains in the compositions of the invention are viable and capable of partially or totally colonising the intestine.

### Therapeutic uses

As demonstrated in the examples, the bacterial compositions of the invention are effective for reducing the HDAC activity. In particular, treatment with compositions of the invention achieves a reduction in Class 1 HDAC activity. In particular, treatment with the compositions of the invention achieves a reduction in HDAC2 activity. The compositions of the invention also show clinical improvements in animal models of hyperactivity. Therefore, the compositions of the invention may be useful for treating or preventing diseases or conditions mediated by HDAC activity. A condition may be a symptom of a disease. In particular, the compositions of the invention may be useful for reducing or preventing diseases or conditions mediated by elevated levels of HDAC activity. In particular, the compositions of the invention may be useful for reducing or preventing diseases or conditions mediated by elevated levels of Class I HDAC activity. In particular, the compositions of the invention may be useful for reducing or preventing diseases or conditions mediated by elevated levels of HDAC2 activity.

Histone deacetylases are a class of enzymes that remove acetyl groups from protein targets. The most abundant HDAC target are histones, but HDACs are known to deacetylate lysine residues of non-histone protein targets to temporally regulate protein activity. As such, HDACs are sometimes referred to as lysine deacetylases. There are currently 13 known HDACs which are categorised into four main classes class I (HDACs 1, 2, 3 and 8), class IIa (HDACs 4,5,7 and 9) and class IIb (HDACs 6 and 10), Class III (sirt1-sirt7) and class IV (HDAC 11) [7]. Each class generally has a different tissue expression pattern and subcellular localisation.

Protein acetylation/deacetylation is generally used as a mechanism of post-translational control of protein activity. Histone acetylation/deacetylation is a well-established mechanism of transcriptional regulation. Genetic regulation is caused by histone deacetylase-mediated cleavage of an acetyl group from a ε-N-acetyl of a lysine amino acid in a histone tail. Removal of the acetyl group restores positive charge to the histone tail, leading to more favourable binding to the negative charged phosphodiester DNA backbone. Improved binding leads to tighter chromosome compaction and an overall reduction in gene expression at the site of histone deacetylation.

Histone deacetylase activity has been implicated in a wide array of diseases and conditions. Inhibition of histone deacetylase activity can be used to alleviate or ameliorate these diseases or conditions. Pan-inhibitors of histone deacetylases may be useful in the treatment or prevention of HDAC-mediated diseases. Isoform specific HDAC inhibitors may be useful in the treatment or prevention of diseases mediated by specific HDAC isoform activity.

Inhibition of HDAC activity is an established treatment modality and a number of HDAC inhibitors are approved medicines, including: Vorinostat (CTCL), Romidepsin (CTCL), Chidamide (PTCL), Panobinostat (multiple myeloma), Belinostat (T cell lymphoma), and many are in clinical trials, including: Panobinostat (CTCL), valproic acid (cervical cancer and ovarian cancer, spinal muscular atrophy), Mocetinostat (follicular lymphoma, Hodgkin lymphoma and acute myeloid leukemia), Abexinostat (sarcoma), Entinostat (Hodgkin lymphoma, lung cancer and breast cancer), SB939 (Recurrent or Metastatic Prostate Cancer), Resminostat (Hodgkin lymphoma), Givinostat (refractory leukemias and myelomas), HBI-800 (Advanced Solid Tumors Including Melanoma, Renal Cell Carcinoma (RCC), and Non-Small Cell Lung Cancer (NSCLC)), Kevetrin (ovarian cancer), CUDC-101, AR-42 (relapsed or treatment-resistant multiple myelom, chronic lymphocytic leukemia or lymphoma), CHR-2845, CHR-3996, 4SC-202 (advanced haematological indications), CG200745 (solid tumours), ACY-1215 (multiple myeloma), ME-344 (solid refractory tumours), sulforaphane, and Trichostatin (anti-inflammatory).

Examples of diseases or conditions mediated by HDAC activity include neurodegenerative diseases, such as Alzheimer's disease, Huntington's disease or Parkinson's disease, brain injury, such as stroke, behavioural disorders, such as attention deficit hyperactivity disorder, inflammatory bowel diseases, such as Crohn's disease, cancer, such as prostate cancer, colorectal cancer, breast cancer, lung cancer, liver cancer or gastric cancer. In certain embodiments the compositions of the invention are used to treat or prevent one of these diseases or conditions. In certain embodiments, the compositions of the invention are used to treat or prevent one of these diseases or conditions mediated by elevated HDAC activity. In certain embodiments, the compositions of the invention are used to treat or prevent one of these diseases or conditions mediated by elevated Class I HDAC activity. In certain embodiments, the compositions of the invention are used to treat or prevent one of these diseases or conditions mediated by elevated HDAC2 activity.

In certain embodiments, the compositions of the invention are for use in therapy. In certain embodiments, the compositions of the invention are for use in the treatment of prevention of a disease or condition mediated by elevated HDAC activity. In certain embodiments, the compositions of the invention are for use in a method of reducing HDAC activity in the treatment or prevention of a disease or condition mediated by elevated HDAC activity. In some embodiments, the compositions of the invention are for use in treating or preventing a disease or condition mediated by elevated Class I HDAC activity. In certain embodiments, the compositions of the invention are for use in a method of inhibiting Class I HDAC activity. In certain embodiments, the compositions of the invention are for use in a method of selectively inhibiting Class I HDAC activity in the treatment or prevention of a disease mediated by elevated Class I HDAC activity. The inventors have identified that certain compositions of the invention selectively inhibit Class I HDACs. As used herein "selective" refers to compositions that have the greatest inhibitory effect on Class I HDACs, for example, in comparison to their inhibitory effect of HDACs from other classes. Selective inhibition of HDACs is advantageous for the treatment of diseases that require long-term administration of a therapeutic agent, for example where a disease or condition needs to be treated throughout the lifetime of a patient. In certain embodiments, the compositions of the invention that are Class I HDAC selective inhibitors are for use in the palliative treatment or prevention of a disease or condition mediated by elevated Class I HDAC activity. Selective inhibitors are advantageous over pan-inhibitors known in the art by reducing side effects associated with the unwanted inhibition of other classes of HDACs. In certain embodiments, the compositions of the invention are HDAC2 selective inhibitors. In certain embodiments, the compositions of the invention are for use in a method of selectively reducing HDAC2 activity. In certain embodiments, the compositions of the invention are for use in the treatment or prevention of a disease mediated by elevated HDAC2 activity.

### Brain injury

The examples demonstrate that the compositions of the invention are neuroprotective and have HDAC inhibitory activity. HDAC2 is a crucial target for functional recovery from stroke [18] and HDAC inhibition can prevent white matter injury [19], so the compositions of the invention may be useful in the treatment of brain injury.

In certain embodiments, the compositions of the invention are for use in treating brain injury. In some embodiments, the brain injury is a traumatic brain injury. In some embodiments, the brain injury is an acquired brain injury. In some embodiments, the compositions of the invention are for use in treating brain injury resulting from trauma. In some embodiments, the compositions of the invention are for use in treating brain injury resulting from a tumour. In some embodiments, the compositions of the invention are for use in treating brain injury resulting from a stroke. In some embodiments, the compositions of the invention are for use in treating brain injury resulting from a brain haemorrhage. In some embodiments, the compositions of the invention are for use in treating brain injury resulting from encephalitis. In some embodiments, the compositions of the invention are for use in treating brain injury resulting from cerebral hypoxia. In some embodiments, the compositions of the invention are for use in treating brain injury resulting from cerebral anoxia.

In preferred embodiments, the compositions of the invention are for use in treating stroke. The effects shown in the examples are particularly relevant to the treatment of stroke. Stroke occurs when blood flow to at least a part of the brain is interrupted. Without an adequate supply of blood to provide oxygen and nutrients to the brain tissue and to remove waste products from the brain tissue, brain cells rapidly begin to die. The symptoms of stroke are dependent on the region of the brain which is affected by the inadequate blood flow. Symptoms include paralysis, numbness or weakness of the muscles, loss of balance, dizziness, sudden severe headaches, speech impairment, loss of memory, loss of reasoning ability, sudden confusion, vision impairment, coma or even death. A stroke is also referred to as a brain attack or a cerebrovascular accident (CVA). The symptoms of stroke may be brief if adequate blood flow is restored within a short period of time. However, if inadequate blood flow continues for a significant period of time, the symptoms can be permanent.

In some embodiments, the stroke is cerebral ischemia. Cerebral ischemia results when there is insufficient blood flow to the tissues of the brain to meet metabolic demand. In some embodiments, the cerebral ischemia is focal cerebral ischemia, i.e. confined to a specific region of the brain. In some embodiments the cerebral ischemia is global cerebral ischemia, i.e. encompassing a wide area of the brain tissue. Focal cerebral ischemia commonly occurs when a cerebral vessel has become blocked, either partially or completely, reducing the flow of blood to a specific region of the brain. In some embodiments the focal cerebral ischemia is ischemic stroke. In some embodiments, the ischemic stroke is thrombotic, i.e. caused by a thrombus or blood clot, which develops in a cerebral vessel and restricts or blocks blood flow. In some embodiments the ischemic stroke is a thrombotic stroke. In some embodiments the ischemic stroke is embolic, i.e. caused by an embolus, or an unattached mass that travels through the bloodstream and restricts or blocks blood flow at a site distant from its point of origin. In some embodiments the ischemic stroke is an embolic stroke. Global cerebral ischemia commonly occurs when blood flow to the brain as a whole is blocked or reduced. In some embodiments the global cerebral ischemia is caused by hypoperfusion, i.e. due to shock. In some embodiments the global cerebral ischemia is a result of a cardiac arrest.

In some embodiments the subject diagnosed with brain injury has suffered cerebral ischemia. In some embodiments, the subject diagnosed with brain injury has suffered focal cerebral ischemia. In some embodiments, the subject diagnosed with brain injury has suffered an ischemic stroke. In some embodiments, the subject diagnosed with brain injury has suffered a thrombotic stroke. In some embodiments, the subject diagnosed with brain injury has suffered an embolic stroke. In some embodiments, the subject diagnosed with brain injury has suffered global cerebral ischemia. In some embodiments, the subject diagnosed with brain injury has suffered hypoperfusion. In some embodiments, the subject diagnosed with brain injury has suffered a cardiac arrest.

In some embodiments, the compositions of the invention are for use in treating cerebral ischemia. In some embodiments, the compositions of the invention are for use in treating focal cerebral ischemia. In some embodiments, the compositions of the invention are for use treating ischemic stroke. In some embodiments, the compositions of the invention are for use in treating thrombotic stroke. In some embodiments, the compositions of the invention are for use in treating embolic stroke. In some embodiments, the compositions of the invention are for use in treating global cerebral ischemia. In some embodiments, the compositions of the invention are for use in treating hypoperfusion.

In some embodiments, the stroke is hemorrhagic stroke. Hemorrhagic stroke is caused by bleeding into or around the brain resulting in swelling, pressure and damage to the cells and tissues of the brain. Hemorrhagic stroke is commonly a result of a weakened blood vessel that ruptures and bleeds into the surrounding brain. In some embodiments, the hemorrhagic stroke is an intracerebral hemorrhage, i.e. caused by bleeding within the brain tissue itself. In some embodiments the intracerebral hemorrhage is caused by an intraparenchymal hemorrhage. In some embodiments the intracerebral hemorrhage is caused by an intraventricular hemorrhage. In some embodiments the hemorrhagic stroke is a subarachnoid hemorrhage i.e. bleeding that occurs outside of the brain tissue but still within the skull. In some embodiments, the hemorrhagic stroke is a result of cerebral amyloid angiopathy. In some embodiments, the hemorrhagic stroke is a result of a brain aneurysm. In some embodiments, the hemorrhagic stroke is a result of cerebral arteriovenous malformation (AVM).

In some embodiments the subject diagnosed with brain injury has suffered hemorrhagic stroke. In some embodiments, the subject diagnosed with brain injury has suffered an intracerebral hemorrhage. In some embodiments, the subject diagnosed with brain injury has suffered an intraparenchymal hemorrhage. In some embodiments, the subject diagnosed with brain injury has suffered an intraventricular hemorrhage. In some embodiments, the subject diagnosed with brain injury has suffered a subarachnoid hemorrhage. In some embodiments, the subject diagnosed with brain injury has suffered cerebral amyloid angiopathy. In some embodiments, the subject diagnosed with brain injury has suffered a brain aneurysm. In some embodiments, the subject diagnosed with brain injury has suffered cerebral AVM

In some embodiments, the compositions of the invention are for use in treating hemorrhagic stroke. In some embodiments, the compositions of the invention are for use in treating an intracerebral hemorrhage. In some embodiments, the compositions of the invention are for use in treating an intraparenchymal hemorrhage. In some embodiments, the compositions of the invention are for use in treating an intraventricular hemorrhage. In some embodiments, the compositions of the invention are for use in treating a subarachnoid hemorrhage. In some embodiments, the compositions of the invention are for use in treating a cerebral amyloid angiopathy. In some embodiments, the compositions of the invention are for use in treating a brain aneurysm. In some embodiments, the compositions of the invention are for use in treating cerebral AVM

Restoration of adequate blood flow to the brain after a period of interruption, though effective in alleviating the symptoms associated with stroke, can paradoxically result in further damage to the brain tissue. During the period of interruption, the affected tissue suffers from a lack of oxygen and nutrients, and the sudden restoration of blood flow can result in inflammation and oxidative damage through the induction of oxidative stress. This is known as reperfusion injury, and is well documented not only following stroke, but also following a heart attack or other tissue damage when blood supply returns to the tissue after a period of ischemia or lack of oxygen. In some embodiments the subject diagnosed with brain injury has suffered from reperfusion injury as a result of stroke. In some embodiments, the compositions of the invention are for use in treating reperfusion injury as a result of stroke.

A transient ischemic attack (TIA), often referred to as a mini-stroke, is a recognised warning sign for a more serious stroke. Subjects who have suffered one or more TIAs are therefore at greater risk of stroke. In some embodiments the subject diagnosed with brain injury has suffered a TIA. In some embodiments, the compositions of the invention are for use in treating a TIA. In some embodiments, the compositions of the invention are for use in treating brain injury in a subject who has suffered a TIA.

High blood pressure, high blood cholesterol, a familial history of stroke, heart disease, diabetes, brain aneurysms, arteriovenous malformations, sickle cell disease, vasculitis, bleeding disorders, use of nonsteroidal anti-inflammatory drugs (NSAIDs), smoking tobacco, drinking large amounts of alcohol, illegal drug use, obesity, lack of physical activity and an unhealthy diet are all considered to be risk factors for stroke. In particular, lowering blood pressure has been conclusively shown to prevent both ischemic and hemorrhagic strokes [20, 21]. In some embodiments, the compositions of the invention are for use in treating brain injury in a subject who has at least one risk factor for stroke. In some embodiments the subject has two risk factors for stroke. In some embodiments the subject has three risk factors for stroke. In some embodiments the subject has four risk factors for stroke. In some embodiments the subject has more than four risk factors for stroke. In some embodiments the subject has high blood pressure. In some embodiments the subject has high blood cholesterol. In some embodiments the subject has a familial history of stroke. In some embodiments the subject has heart disease. In some embodiments the subject has diabetes. In some embodiments the subject has a brain aneurysm. In some embodiments the subject has arteriovenous malformations. In some embodiments the subject has vasculitis. In some embodiments the subject has sickle cell disease. In some embodiments the subject has a bleeding disorder. In some embodiments the subject has a history of use of nonsteroidal anti-inflammatory drugs (NSAIDs). In some embodiments the subject smokes tobacco. In some embodiments the subject drinks large amounts of alcohol. In some embodiments the subject uses illegal drugs. In some embodiments the subject is obese. In some embodiments the subject is overweight. In some embodiments the subject has a lack of physical activity. In some embodiments the subject has an unhealthy diet.

The examples indicate that the compositions of the invention may be useful for treating brain injury and aiding recovery when administered before the injury event occurs. Therefore, the compositions of the invention may be particularly useful for treating brain injury when administered to subjects at risk of brain injury, such as stroke.

In certain embodiments, the compositions of the invention are for use in reducing the damage caused by a potential brain injury, preferably a stroke. The compositions may reduce the damage caused when they are administered before the potential brain injury occurs, in particular when administered to a patient identified as at risk of a brain injury.

The examples indicate that the compositions of the invention may be useful for treating brain injury and aiding recovery when administered after the injury event occurs. Therefore, the compositions of the invention may be particularly useful for treating brain injury when administered to subjects following a brain injury, such as stroke.

In some embodiments, the compositions of the invention treat brain injury by reducing motoric damage. In some embodiments, the compositions of the invention treat brain injury by improving motor function. In some embodiments, the compositions of the invention treat brain injury by improving muscle strength. In some embodiments, the compositions of the invention treat brain injury by improving memory. In some embodiments, the compositions of the invention treat brain injury by improving social recognition. In some embodiments, the compositions of the invention treat brain injury by improving neurological function.

Treatment of brain injury may refer to, for example, an alleviation of the severity of symptoms. Treatment of brain injury may also refer to reducing the neurological impairments following stroke. Compositions of the invention for use in treating stroke may be provided to the subject in advance of the onset of stroke, for example in a patient identified as being at risk of stroke. Compositions of the invention for use in treating stroke may be provided after a stroke has occurred, for example, during recovery. Compositions of the invention for use in treating stroke may be provided during the acute phase of recovery (i.e. up to one week after stroke). Compositions of the invention for use in treating stroke may be provided during the subacute phase of recovery (i.e. from one week up to three months after stroke). Compositions of the invention for use in treating stroke may be provided during the chronic phase of recovery (from three months after stroke).

In certain embodiments, the compositions of the invention are for use in combination with a secondary active agent. In certain embodiments, the compositions of the invention are for use in combination with aspirin or tissue plasminogen activator (tPA). Other secondary agents include other antiplatelets (such as clopidogrel), anticoagulants (such as heparins, warfarin, apixaban, dabigatran, edoxaban or rivaroxaban), antihypertensives (such as diuretics, ACE inhibitors, calcium channel blockers, beta-blockers or alpha-blockers) or statins. The compositions of the invention may improve the patient's response to the secondary active agent.

In certain embodiments, the compositions of the invention reduce the effect of ischemia on tissues. In certain embodiments, the compositions of the invention reduce the amount of damage to tissues caused by ischemia. In certain embodiments, the tissues damaged by ischemia are the cerebral tissues. In certain embodiments, the compositions of the invention reduce necrosis or the number of necrotic cells. In certain embodiments, the compositions of the invention reduce apoptosis or the number of apoptotic cells. In certain embodiments, the compositions of the invention reduce the number of necrotic and apoptotic cells. In certain embodiments, the compositions of the invention prevent cell death by necrosis and/or apoptosis. In certain embodiments, the compositions of the invention prevent cell death by necrosis and/or apoptosis caused by ischemia. In certain embodiments, the compositions of the invention improve the recovery of the tissue damaged by ischemia. In certain embodiments, the compositions of the invention improve the speed of clearance of necrotic cells and/or apoptotic cells. In certain embodiments, the compositions of the invention improve the efficacy of the clearance of necrotic cells and/or apoptotic cells. In certain embodiments, the compositions of the invention improve the replacement and/or regeneration of cells within tissues. In certain embodiments, the compositions of the invention improve the replacement and/or regeneration of cells within tissues damaged by ischemia. In certain embodiments, the compositions of the invention improve the overall histology of the tissue (for example upon a biopsy).

The examples demonstrate that the compositions of the invention activate MAP2 (Microtubule - associated protein 2) activation. MAP2 is a gene associated with neuronal differentiation of MAP2 and is thought to be essential for microtubule formation in neuritogenesis, so compositions of the invention may be particularly useful for treating brain injuries. In some embodiments, the compositions of the invention are for use in treating brain injuries by activating or increasing the levels of MAP2. Moreover, as MAP2 promotes neurite outgrowth, which play a major role in re-networking of damaged neurons and synaptogenesis, MAP2 expression might go beyond being a marker of neuronal differentiation and indicate "neuronal re-wiring" associated with the therapeutic outcome of neuropathological disease [22].

### Inflammatory and autoimmune disorders

The examples demonstrate that the compositions of the invention have HDAC inhibitory activity. HDAC activity is central to the pathology of many inflammatory and autoimmune disorders, and HDAC inhibitors have shown efficacy in the treatment of many inflammatory and autoimmune disorders, as discussed below in relation to specific conditions (see also [23]). Therefore, the compositions of the invention may be useful for treating inflammatory and autoimmune disorders, in particular inflammatory and autoimmune disorders mediated by elevated histone deacetylase (HDAC) activity.

In certain embodiments, the compositions of the invention are for use in a method of treating or preventing an inflammatory or autoimmune disorder. In certain embodiments, the compositions of the invention are for use in treating or preventing an inflammatory or autoimmune disease, wherein said treatment or prevention is achieved by reducing or preventing HDAC activation. In certain embodiments, the compositions of the invention are for use in treating a patient with an inflammatory or autoimmune disease, wherein the patient has elevated HDAC levels or activity. In certain embodiments, the patient may have been diagnosed with a chronic inflammatory or autoimmune disease or condition, or the composition of the invention may be for use in preventing an inflammatory or autoimmune disease or condition developing into a chronic inflammatory or autoimmune disease or condition. In certain embodiments, the disease or condition may not be responsive to treatment with TNF-α inhibitors.

HDAC may be associated with chronic inflammatory and autoimmune diseases, so the compositions of the invention may be particularly useful for treating or preventing chronic diseases or conditions as listed above. In certain embodiments, the compositions are for use in patients with chronic disease. In certain embodiments, the compositions are for use in preventing the development of chronic disease.

The compositions of the invention may be useful for treating diseases and conditions mediated by HDAC and for addressing HDAC activation, so the compositions of the invention may be particularly useful for treating or preventing chronic disease, treating or preventing disease in patients that have not responded to other therapies (such as treatment with TNF-α inhibitors), and/or treating or preventing the tissue damage and symptoms associated with HDAC.

The examples demonstrate that the compositions of the invention reduce IL-6 production and secretion, which may be particularly useful for treating inflammatory and autoimmune disorders. In certain embodiments, the compositions of the invention are for use in reducing inflammation in the treatment of disease. In certain embodiments, the compositions of the invention decrease IL-6 production and secretion. In certain embodiments, the compositions of the invention decrease the activation of the NFκB promoter. In certain embodiments, the compositions of the invention are able to modulate the activation of IL-6 production by the potent pro-inflammatory endotoxin lipopolysaccharide (LPS).

### - Inflammatory bowel disease

The examples demonstrate that the compositions of the invention have HDAC inhibitory activity, and so they may be useful in the treatment of inflammatory bowel disease. Overexpression of different HDAC isoforms have been implicated in a variety of disease pathologies, including colitis. Additionally, valproic acid has been associated with class I HDAC inhibition and amelioration of colitis in a DSS-colitis murine model [24]. This study suggested a role for HDAC class I inhibitors in IFN-γ, IL-10, IL-1β and TNF-α suppression, assigning functionality to HDAC inhibition and efficacy in colitis. Therefore, the examples indicate that the compositions of the invention may be useful for treating inflammatory bowel diseases.

In certain embodiments, the compositions of the invention are for use in treating or preventing inflammatory bowel disease. In certain embodiments, the compositions of the invention are for use in treating or preventing inflammatory bowel disease, wherein said treatment or prevention is achieved by reducing or preventing HDAC activation. In certain embodiments, the compositions of the invention are for use in treating a patient with inflammatory bowel disease, wherein the patient has elevated HDAC levels or activity.

Inflammatory bowel disease (IBD) is a complex disease that can be caused by multiple environmental and genetic factors. Factors contributing to the onset of IBD include diet, microbiota, intestinal permeability, and genetic susceptibility to increased inflammatory response to gut infection. Symptoms of inflammatory bowel disease include abdominal pain, vomiting, diarrhoea, rectal bleeding, severe internal cramps/muscle spasms in the pelvic region, weight loss and anaemia. In certain embodiments, the compositions are for use in reducing one or more symptoms associated with IBD. In certain embodiments, the compositions of the invention are for use in preventing one or more symptoms of IBD.

IBD may accompany other diseases or conditions, such as arthritis, pyoderma gangrenosum, primary sclerosing cholangitis, non-thyroidal illness syndrome, deep vein thrombosis, bronchiolitis obliterans organizing pneumonia. In certain embodiments, the compositions of the invention are for use in the treatment or prevention of one or more diseases or conditions that accompany IBD.

Inflammatory bowel disease is generally diagnosed by biopsy or colonoscopy. Measurements of faecal calprotectin is useful for the preliminary diagnosis of IBD. Other laboratory test for the diagnosis of IBD include, complete blood count, erythrocyte sedimentation rate, comprehensive metabolic panel, faecal occult blood test or C-reactive protein test. Typically a combination of laboratory testing and biopsy/colonoscopy will be used to confirm diagnosis of IBD. In certain embodiments, the compositions of the invention are for use in a subject diagnosed with IBD.

In certain embodiments the inflammatory bowel disease is Crohn's disease. Studies have shown that several HDACs are upregulated in the inflammatory muscosa of patients with Crohn's disease. Therefore, inhibition of HDAC activity may be useful in the treatment of Crohn's disease. In certain embodiments, the compositions of the invention are for use in the treatment or prevention of Crohn's disease.

Crohn's disease is a complex disease with an array of probable causes, including genetic risk factors, diet, other lifestyle factors, such as smoking and alcohol consumption, and microbiome composition. Crohn's disease can manifest anywhere along the gastrointestinal tract.

Gastrointestinal symptoms of Crohn's disease range from mild to severe and include abdominal pain, diarrhoea, faecal blood, ileitis, increased bowel movements, increased flatulence, intestinal stenosis, vomiting, and perianal discomfort. The compositions of the invention may be for use in the treatment of prevention of one or more gastrointestinal symptoms of Crohn's disease.

Systemic symptoms of Crohn's disease include growth defects, such as the inability to maintain growth during puberty, decreased appetite, fever and weight loss. Extra-intestinal features of Crohn's disease include uveitis, photobia, episcleritis, gall stones, seronegative spondyloarthropathy, arthritis, enthesitis, erythema nodosum, pyoderma gangrenosum, deep venous thrombosis, pulmonary embolism, autoimmune haemolytic anaemia, clubbing and osteoporosis. Extra-intestinal features are additional conditions associated with Crohn's disease that manifest outside the GI tract. Subjects with Crohn's disease also exhibit increased susceptibility to neurological complications such as seizures, strokes, myopathy, peripheral neuropathy, headache and depression. In certain embodiments, the compositions of the invention are for use in the treatment or prevention of one or more systemic symptoms of Crohn' disease. In certain embodiments, the compositions of the invention are for use in the treatment or prevention of one or more extra-intestinal features of Crohn's disease.

The diagnosis of Crohn's disease usually involves carrying out multiple tests and surgical procedures, such as gastroscopy and/or colonoscopy and biopsy, typically of the ileum, radiologic tests, complete blood counts, C-reactive protein tests and erythrocyte sedimentation rates. In certain embodiments, the compositions of the invention are for use in subjects diagnosed with Crohn's disease. In some embodiments, compositions of the invention are for use in treating a subject who has been diagnosed with Crohn's disease.

Crohn's disease is classified depending on the extent of the region of the GI tract affected [25]. A disease of both the ileum and colon is classified as Ileocolic Crohn's. In some embodiments, the compositions are for use in the treatment or prevention of Ileocolic Crohn's. In some embodiments, the compositions are for use in a subject diagnosed with Ileocolic Crohn's/ Crohn's ileitis is classified if only the ileum is affected. Crohn's colitis is classified if only the colon is affected. In certain embodiments, the compositions are for use in the treatment or prevention of Crohn's ileitis. In some embodiments, the compositions are for use in a subject diagnosed with Crohn's ileitis. In certain embodiments, the compositions are for use in the treatment or prevention of Crohn's colitis. In some embodiments, the compositions are for use in a subject diagnosed with Crohn's colitis.

Crohn's disease may be treated with a number of therapeutic agents, such as corticosteroids, such as prednisone, immunosuppressive agents, such as azathioprine, or biologics, such as infliximab, adalimumab, and golimumab, vedolizumab and etrolizumab. In certain embodiments, the compositions of the invention are for use in the treatment or prevention of Crohn's disease in combination with an additional therapeutic agent. In certain embodiments, the additional therapeutic agent is for use in the treatment or prevention of Crohn's disease.

The examples demonstrate that the compositions of the invention reduce modulate expression of IDO1 in the intestine, which may be particularly useful for treating inflammatory bowel diseases. In certain embodiments, the compositions of the invention increase IDOl expression.

### - Multiple Sclerosis

Multiple sclerosis (MS) is an autoimmune inflammatory disorder of the central nervous system. MS can be modelled in animals by the induction of experimental autoimmune encephalomyelitis (EAE). HDAC inhibitors have been shown to reduce clinical symptoms and inhibit disease progress in mice with adoptive EAE (Dasgupta et al., 2003, J Immunol, 170 (7), 3874-3882). Injection of an HDAC inhibitor has also been shown to significantly reduce neurological impairment and disability in mice with an experimental model of chronic MS (Camelo et al., 2005, J Neuroimmunol, 164(1-2), 10-21). Inhibition of HDAC activity has been suggested as a promising therapy for MS (Gray et al., 2006, Epigenetics, 1:2, 67-75). Therefore, the compositions of the invention may be useful for treating or preventing multiple sclerosis in a subject.

In certain embodiments, the compositions of the invention are for use in treating or preventing multiple sclerosis, wherein said treatment or prevention is achieved by reducing or preventing HDAC activation. In certain embodiments, the compositions of the invention are for use in treating a patient with multiple sclerosis, wherein the patient has elevated HDAC levels or activity.

In preferred embodiments, the compositions of the invention are for use in treating or preventing multiple sclerosis. The compositions of the invention may achieve HDAC inhibition, and so they may be useful in the treatment or prevention of multiple sclerosis. Multiple sclerosis is an inflammatory disorder associated with damage to the myelin sheaths of neurons, particularly in the brain and spinal column. Multiple sclerosis is a chronic disease, which is progressively incapacitating and which evolves in episodes.

In certain embodiments, treatment with the compositions of the invention results in a reduction in disease incidence or disease severity. In certain embodiments, the compositions of the invention are for use in reducing disease incidence or disease severity. In certain embodiments, treatment with the compositions of the invention prevents a decline in motor function or results in improved motor function. In certain embodiments, the compositions of the invention are for use in preventing a decline in motor function or for use in improving motor function. In certain embodiments, treatment with the compositions of the invention prevents the development of paralysis. In certain embodiments, the compositions of the invention are for use in preventing paralysis in the treatment of multiple sclerosis.

The compositions of the invention may be useful for modulating a patient's immune system, so in certain embodiments the compositions of the invention are for use in preventing multiple sclerosis in a patient that has been identified as at risk of multiple sclerosis, or that has been diagnosed with early-stage multiple sclerosis or "relapsing-remitting" multiple sclerosis. The compositions of the invention may be useful for preventing the development of sclerosis.

The compositions of the invention may be useful for managing or alleviating multiple sclerosis. The compositions of the invention may be particularly useful for reducing symptoms associated with multiple sclerosis. Treatment or prevention of multiple sclerosis may refer to, for example, an alleviation of the severity of symptoms or a reduction in the frequency of exacerbations or the range of triggers that are a problem for the patient.

### - Arthritis

Arthritis is a disease characterised by chronic joint inflammation. Rheumatoid arthritis is a chronic autoimmune disorder that typically results in swollen and painful joints. HDAC inhibition has been proposed to treat rheumatoid arthritis by a variety of mechanisms, including influencing cytokine production, inhibiting T-cell differentiation, suppressing proliferation of synovial fibroblasts and reducing bone loss by influencing osteoclasts and osteoblasts (Vojinov et al., 2011, Mol Med, 17 (5-6) 397-403). HDAC inhibition has been shown to have a strong anti-inflammatory effect in several animal models of arthritis (Joosten et al., 2011, Mol Med, 17 (5-6), 391-396). Therefore, the compositions of the invention may be useful for treating or preventing arthritis in a subject.

In preferred embodiments, the compositions of the invention are for use in treating or preventing rheumatoid arthritis (RA). In certain embodiments, the compositions of the invention are for use in treating or preventing rheumatoid arthritis, wherein said treatment or prevention is achieved by reducing or preventing HDAC activation. In certain embodiments, the compositions of the invention are for use in treating a patient with rheumatoid arthritis, wherein the patient has elevated HDAC levels or activity.

In certain embodiments, treatment with the compositions of the invention results in a reduction in the swelling of joints. In certain embodiments, the compositions of the invention are for use in patients with swollen joints or patients identified as at risk of having swollen joints. In certain embodiments, the compositions of the invention are for use in a method of reducing joint swelling in RA.

In certain embodiments, treatment with the compositions of the invention results in a reduction in cartilage damage or bone damage. In certain embodiments, the compositions of the invention are for use in reducing or preventing cartilage or bone damage in the treatment of RA. In certain embodiments, the compositions are for use in treating patient with severe RA that are at risk of cartilage or bone damage.

In certain embodiments, the compositions of the invention are for use in preventing bone erosion or cartilage damage in the treatment of RA. In certain embodiments, the compositions are for use in treating patients that exhibit bone erosion or cartilage damage or patients identified as at risk of bone erosion or cartilage damage.

The compositions of the invention may be useful for modulating a patient's immune system, so in certain embodiments the compositions of the invention are for use in preventing RA in a patient that has been identified as at risk of RA, or that has been diagnosed with early-stage RA. The compositions of the invention may be useful for preventing the development of RA

The compositions of the invention may be useful for managing or alleviating RA. The compositions of the invention may be particularly useful for reducing symptoms associated with joint swelling or bone destruction. Treatment or prevention of RA may refer to, for example, an alleviation of the severity of symptoms or a reduction in the frequency of exacerbations or the range of triggers that are a problem for the patient.

### - Asthma

Asthma is a chronic inflammatory respiratory disease. HDAC inhibitors have been shown to have anti-inflammatory effects that relieve airway inflammation, airway remodelling and airway hypersensitivity in a mouse model of chronic asthma (Ren et al., 2016, Inflamm Res, 65, 995-1008). Therefore, the compositions of the invention may be useful for treating or preventing asthma in a subject.

In preferred embodiments, the compositions of the invention are for use in treating or preventing asthma. In certain embodiments, the compositions of the invention are for use in treating or preventing asthma, wherein said treatment or prevention is achieved by reducing or preventing HDAC activation. In certain embodiments, the compositions of the invention are for use in treating a patient with asthma, wherein the patient has elevated HDAC levels or activity.

In certain embodiments, the asthma is eosinophilic or allergic asthma. Eosinophilic and allergic asthma are characterised by increased numbers of eosinophils in peripheral blood and in airway secretions and is associated pathologically with thickening of the basement membrane zone and pharmacologically by corticosteroid responsiveness [26]. Compositions that reduce or inhibit eosinophil recruitment or activation may be useful for treating or preventing eosinophilic and allergic asthma. Eosinophilic and allergic asthma are also characterised by a cascade of inflammatory events mediated by T helper type 2 lymphocyte (Th2) processes. Compositions that reduce or inhibit T helper type 2 lymphocyte (Th2) processes may be useful for treating or preventing eosinophilic and allergic asthma.

In additional embodiments, the compositions of the invention are for use in treating or preventing neutrophilic asthma (or non-eosinophilic asthma). High neutrophil numbers are associated with severe asthma that may be insensitive to corticosteroid treatment. Compositions that reduce or inhibit neutrophil recruitment or activation may be useful for treating or preventing neutrophilic asthma.

Eosinophilic asthma (also referred to as Th2-high asthma) and neutrophilic asthma (also referred to as Th2-low or non-Th2 asthma) have different underlying pathophysiological mechanisms and present different clinical features. For example, Th2-high asthma generally presents early onset and exhibits seasonal variations of symptoms, whereas Th2-low asthma has a much later onset, typically around the age of 40 or later. Th2-high asthma is also characterised by increased immunoglobulin E (IgE) blood levels, whereas this feature is absent in Th2-low asthma. Th2 high asthma is also characterised by high sputum levels of eosinophils. By contrast, Th2-low asthma may be characterised by elevated levels of sputum neutrophils. In certain embodiments, the compositions of the invention are for use in treating Th2-low or non-Th2 asthma. In certain embodiments, the compositions of the invention are for use in treating Th2-high asthma.

Eosinophilic and neutrophilic asthma are not mutually exclusive conditions and treatments that help address either the eosinophil and neutrophil responses may be useful for treating asthma in general.

In certain embodiments, the compositions of the invention are for use in methods reducing an eosinophilic inflammatory response in the treatment or prevention of asthma, or for use in methods of reducing a neutrophilic inflammatory response in the treatment or prevention of asthma. As noted above, high levels of eosinophils in asthma is associated pathologically with thickening of the basement membrane zone, so reducing eosinophilic inflammatory response in the treatment or prevention of asthma may be able to specifically address this feature of the disease. Also, elevated neutrophils, either in combination with elevated eosinophils or in their absence, is associated with severe asthma and chronic airway narrowing. Therefore, reducing the neutrophilic inflammatory response may be particularly useful for addressing severe asthma.

In certain embodiments, the compositions reduce peribronchiolar infiltration in allergic asthma, or are for use in reducing peribronchiolar infiltration in the treatment of allergic asthma. In certain embodiments, the compositions reduce peribronchiolar and/or perivascular infiltration in neutrophilic asthma, or are for use in reducing peribronchiolar and/or perivascular infiltration in the treatment of allergic neutrophilic asthma.

In certain embodiments, treatment with compositions of the invention provides a reduction or prevents an elevation in TNFα levels.

In certain embodiments, the compositions of the invention are for use in a method of treating asthma that results in a reduction of the eosinophilic and/or neutrophilic inflammatory response. In certain embodiments, the patient to be treated has, or has previously been identified as having, elevated neutrophil or eosinophil levels, for example as identified through blood sampling or sputum analysis. The compositions of the invention may be useful for preventing the development of asthma in a new-born when administered to the new-born, or to a pregnant woman. The compositions may be useful for preventing the development of asthma in children. The compositions of the invention may be useful for treating or preventing adult-onset asthma. The compositions of the invention may be useful for managing or alleviating asthma. The compositions of the invention may be particularly useful for reducing symptoms associated with asthma that is aggravated by allergens, such as house dust mites.

Treatment or prevention of asthma may refer to, for example, an alleviation of the severity of symptoms or a reduction in the frequency of exacerbations or the range of triggers that are a problem for the patient.

### - Psoriasis

Psoriasis is a chronic inflammatory skin disease. Overexpression of HDAC1 has been reported for in skin biopsies from psoriatic pateints (Tovar-Castillo et al., 2007, Int J Dermatol, 46, 239-46) and a HDAC inhibitor has been shown to block the conversion of Foxp3+ Tregs into Foxp3-RORγt+ IL-17/Tregs (a shift associated with psoriasis disease progression) (Bovenschen et al., 2011, J Invest Dermatol, 131, 1853-60). Therefore, the compositions of the invention may be useful for treating or preventing psoriasis in a subject.

In preferred embodiments, the compositions of the invention are for use in treating or preventing psoriasis. In certain embodiments, the compositions of the invention are for use in treating or preventing psoriasis, wherein said treatment or prevention is achieved by reducing or preventing HDAC activation. In certain embodiments, the compositions of the invention are for use in treating a patient with psoriasis, wherein the patient has elevated HDAC levels or activity.

### - Systemic lupus erythematosus

Systemic lupus erythematosus (SLE) is an autoimmune disease. HDAC inhibition is believed to be a promising therapeutic approach for treating SLE based on studies on cell cultures and mouse models of SLE (Reilly et al., 2011, Mol Med, 17 (5-6), 417-425). Therefore, the compositions of the invention may be useful for treating or preventing systemic lupus erythematosus in a subject.

In preferred embodiments, the compositions of the invention are for use in treating or preventing SLE. In certain embodiments, the compositions of the invention are for use in treating or preventing SLE, wherein said treatment or prevention is achieved by reducing or preventing HDAC activation. In certain embodiments, the compositions of the invention are for use in treating a patient with SLE, wherein the patient has elevated HDAC levels or activity.

### - Allograft rejection

Allograft rejection occurs when transplanted tissues are rejected by the recipient's immune system. Studies on murine cardiac transplants have shown that HDAC inhibition increases intra-graft histone 3 acetylation and is associated with increased intra-graft levels of Foxp3 protein (a forkhead transcription family member involved in controlling immune responses), maintenance of tissue architecture and a lack of the stigmata of chronic rejection relative to controls (Wang et al., Immunol Cell Biol, 1-8). Therefore, the compositions of the invention may be useful for treating or preventing allograft rejection in a subject.

In preferred embodiments, the compositions of the invention are for use in treating or preventing allograft rejection. In certain embodiments, the compositions of the invention are for use in treating or preventing allograft rejection, wherein said treatment or prevention is achieved by reducing or preventing HDAC activation. In certain embodiments, the compositions of the invention are for use in treating a patient with allograft rejection, wherein the patient has elevated HDAC levels or activity.

### - Diabetes

Diabetes mellitus is a group of diseases in which low levels of insulin and/or peripheral insulin resistance lead to hyperglycermia. HDAC inhibition has been proposed to treat diabetes by a variety of mechanisms, including de-repression of *Pdx1* (Park et al., 2008, J Clin Invest, 118, 2316-24), enhancing expression of transcription factor *Ngn3* to increase the pool of endocrine progenitor cells (Haumaitre et al., 2008, Mol Cell Biol, 28, 6373-83) and enhancing insulin expression (Molsey et al., 2003, J Biol Chem, 278, 19660-6) amongst others. HDAC inhibition is also a promising treatment for late diabetic complications such as diabetic nephropathy and retinal ischemia (Christensen et al., 2011, Mol Med, 17 (5-6), 370-390). Therefore, the compositions of the invention may be useful for treating or preventing diabetes in a subject.

In preferred embodiments, the compositions of the invention are for use in treating or preventing diabetes. In preferred embodiments, the compositions of the invention are for use in treating or preventing type I diabetes. In preferred embodiments, the compositions of the invention are for use in treating or preventing type II diabetes. In certain embodiments, the compositions of the invention are for use in treating or preventing diabetes, wherein said treatment or prevention is achieved by reducing or preventing HDAC activation. In certain embodiments, the compositions of the invention are for use in treating a patient with diabetes, wherein the patient has elevated HDAC levels or activity.

### - Graft-versus-host Disease (GVHD)

The compositions of the invention may be for use in the treatment or prevention of Graft-versus-host disease (GVHD). GVHD is a medical complication following transplantation of allogeneic tissue into a subject. GVHD commonly occurs following stem cell or bone marrow transplantation or solid organ transplantation, particularly where the genetic background of the graft (i.e. the donor) and the host (i.e. the recipient) are distinct.

The pathophysiology of GVHD comprises three distinct phases. Firstly, host antigen presenting cells (APCs), such as dendritic cells (DCs) are activated following recognition of the transplanted tissue as a foreign substance. APC activation precedes the recruitment and activation of effector immune cells, such as conventional cytotoxic T cells, which leads to destruction or rejection of the foreign tissue.

HDAC inhibition has been shown to mediate potent pleiotropic anti-inflammatory effects useful in the treatment or prevention of GVHD. HDAC inhibition may inhibit at multiple points of the GVHD pathophysiological cascade. For example, HDAC inhibition prevents antigen presenting cell and dendritic cell activation against allogeneic tissues *in vivo* by enhancing the expression of indoleamine 2,3-dioxygenase in a STAT-3 dependent manner [27]. HDAC inhibition of STAT-1 activity has also been shown to be beneficial in the treatment or prevention of GVHD [28]. In certain embodiments, the composition of the invention may be for use in the treatment or prevention of GVHD by inhibiting APC activation.

HDAC inhibition has also been shown to expand Treg cell populations and activity in vivo [29]. HDAC inhibition-mediated upregulation of Treg cell activity has been shown to supress conventional cytotoxic T cell activity, which may be useful in the treatment or prevention of GVHD by supressing the 2nd phase of the GVHD pathophysiological cascade. In certain embodiments, the compositions of the invention are for use in the treatment or prevention of GVHD by reducing conventional cytotoxic T cell activity. In certain embodiments, the compositions of the invention may be for use in reducing conventional cytotoxic T cell activity. In certain embodiments, the composition of the invention may be for use in the treatment or prevention of GVHD by upregulating Treg cell activity.

Donor NK cells have been shown to reduce GVHD by eliminating host APCs. HDAC inhibition has been shown to increase NK cell activity. Therefore, the compositions of the invention may be for use to increase NK cell activity, which may be useful in the treatment or prevention of GVHD by increasing the elimination of APCs. In certain embodiments, the compositions of the invention may be for use in the treatment or prevention of GVHD by enhancing the elimination of host APCs. In certain embodiments, the compositions of the invention may be for use in the treatment or prevention of GVHD by enhancing NK cell activity. In certain embodiments, the compositions of the invention may be for use in the treatment or prevention of GVHD by enhancing NK cell activity-mediated elimination of host APCs.

In certain embodiments, the compositions of the invention may be administered after the host has received the transplant. In certain embodiments, the compositions of the invention may be administered to the host before the subject has received the transplant. Administration of the compositions of the invention before the transplant has been received may be useful in priming the immune system of the subject to not elicit an inflammatory or autoimmune response against the transplanted tissue. In certain embodiments, the compositions of the invention may be used for preventing or preventing the onset of GVHD. In certain embodiments, the composition of the invention may be for use in the treatment or prevention of GVHD prophylactically. In certain embodiments, the compositions of the invention may be used in the prophylaxis of GVHD. In certain embodiments, the compositions of the invention may be for use in a method of preventing transplant tissue rejection in a subject.

In certain embodiments, the compositions of the invention may be useful for treating, delaying, preventing, or preventing the onset of acute GVHD. Symptoms of acute GVHD typically manifest within the first 100 days of transplantation. Delaying, treatment or prevention of acute GVHD may be particularly beneficial to aid the recovery of subjects in the immediate aftermath of transplant surgery. In certain embodiments, the compositions may treat, delay the onset of, prevent or prevent the onset of acute GVHD by inhibiting HDAC activity. In certain embodiments, the compositions may treat, delay the onset of, prevent, or prevent the onset of acute GVHD by upregulating Treg cell activity. The compositions may treat, delay the onset of, prevent or prevent the onset of acute GVHD by inhibiting conventional cytotoxic T cell activity. The compositions of the invention may treat, delay the onset of, prevent or prevent the onset of acute GVHD by enhancing NK cell activity. The compositions of the invention may treat, delay the onset of, prevent or prevent the onset of acute GVHD by inhibiting APC activation.

In certain embodiments, the compositions of the invention may treat, delay the onset of, prevent, or prevent the onset of acute GVHD when administered to a subject within 100 days following transplantation. In certain embodiments, the compositions of the invention may treat, delay the onset of, prevent, or prevent the onset of acute GVHD when administered to a subject prophylactically, for example, when the composition is administered to the subject before the transplant. In certain embodiments, the compositions of the invention may treat, delay the onset of, prevent, or prevent the onset of persistent, late-onset or recurrent acute GVHD, such as acute GVHD that occurs or recurs more than 100 days after transplantation.

In certain embodiments, the composition of the invention may treat, delay the onset of, prevent, or prevent the onset one or more symptoms of acute GVHD selected from the list consisting of macropaular skin rash, nausea, anorexia, diarrhea, severe abdominal pain, ileus and cholestatic hyperbilirubinemia.

In certain embodiments, the compositions of the invention may be useful for treating, delaying the onset of, preventing, or preventing the onset of chronic GVHD. Chronic GVHD is a complex, multisystem disorder that can involve any organ and is typically characterised by fibrosis. Chronic GVHD may evolve from acute GVHD, or may emerge after a period of quiescence following acute GVHD, or may emerge de novo. Symptoms of chronic GVHD may emerge at any time following transplantation. In certain embodiments, the compositions may be useful for treating, preventing, preventing the onset of, or delaying the onset of chronic GVHD by inhibiting HDAC activity. The compositions may treat, delay the onset of, prevent, or prevent the onset of chronic GVHD by upregulating Treg cell activity. The compositions may treat, delay the onset of, prevent, or prevent the onset of chronic GVHD by inhibiting conventional cytotoxic T cell activity. The compositions of the invention may treat, delay the onset of, prevent, or prevent the onset of chronic GVHD by enhancing NK cell activity. The compositions of the invention may treat, delay the onset of, prevent, or prevent the onset of chronic GVHD by inhibiting APC DC activation.

In certain embodiments, the compositions of the invention are for administration to a patient that has recently undergone a stem cell, bone marrow or solid organ transplant. In certain embodiments, the compositions of the invention are for administration to a patient is in need of a stem cell, bone marrow or solid organ transplant.

In certain embodiments, the composition of the invention may treat, delay the onset of, prevent, or prevent the onset of one or more symptoms of chronic GVHD selected from the list consisting of: dyspigmentation, new-onset alopecia, poikiloderma,lichen planuslike eruptions or sclerotic features, nail dystrophy or loss, xerostomia, mouth ulcers (such as aphthous stomatitis), lichen-type features in the mouth (such as lichen sclerosis), keratoconjunctivitis sicca, sicca syndrome, cicatricial conjunctivitis, fascititis, myostitis, joint stiffness, vaginal sclerosis, ulcerations, anorexia, weight loss, oesophageal web, jaundice, transaminitis, pleural effusions, bronchiolitis obliterans, nephrotic syndrome, pericarditis, thrombocytopenia, anemia, and neutropenia.

The inventors have also shown that the compositions of the invention can reduce colitis associated with GVHD. Colitis is an inflammatory side effect observed in patients with GVHD. The compositions of the invention may also be useful for treating colonic inflammation in a subject with GVHD. Therefore, in some embodiments, the compositions of the invention are for use in treating colitis in a subject with GVHD. In some embodiments, the compositions of the invention are for use in reducing the severity of colitis in a subject with GVHD. In some embodiments, the compositions of the invention are for use in reducing the severity of colitis in the treatment of GVHD. In some embodiments, the compositions of the invention are for use in treating colonic inflammation in a subject with GVHD. In some embodiments, the compositions of the invention are for use in reducing the severity of colonic inflammation in a subject with GVHD. In some embodiments, the compositions of the invention are for use in reducing colonic inflammation in the treatment of GVHD.

The inventors have also found that the compositions of the invention are useful for maintaining gut-barrier function in subjects with GVHD. Maintaining gut-barrier function reduces the translocation of inflammatory cytokines through the gut-barrier, which aggravates toxicity in GVHD [30]. In certain embodiments, the compositions of the invention are for use in maintaining gut-barrier function in the treatment of GVHD. In some embodiments, the compositions of the invention are for use in reducing translocation of inflammatory cytokines across the gut-barrier in the treatment of GVHD.

In certain embodiments, the compositions of the invention may be for use in combination with one or more pharmacological agents for the treatment or prevention of GVHD. In certain embodiments, the one or more pharmacological agents are for the pharmacological prevention or treatment of GVHD. In certain embodiments, the compositions of the invention are for use in the treatment or prevention of GVHD in a subject who is receiving, has received, or is about to receive, one or more of said pharmacological agents. In certain embodiments, the one or more pharmacological agents are selected from the list consisting of: suberoylanilide, vorisnostat, ITF2357 cyclosporine, ciclosporin, sirolimus, pentostatin, rituximab, imatinib, mycophenolate mofetil, tacrolimus, prednisone, methotrexate, remestemcel-L and Prochymal, wherein the pharmacological agent is administered in a therapeutically effective amount for the treatment or prevention of GVHD. In some embodiments, the compositions of the invention are for use in the treatment of GVHD in a subject who has received, is receiving, or is about to receive extracorporeal photophoreses.

### Behavioural and psychiatric disorders

The Examples show that compositions of the invention reduce hyperactivity in mice models of neurological disease. Therefore, the compositions of the invention may be useful in reducing hyperactivity in a subject. Hyperactivity is a symptom of behavioural and psychiatric disorders, such as attention deficit hyperactive disorder (ADHD), post-traumatic stress disorder, anxiety disorders, bipolar affective disorder and obsessive compulsive disorder. Hyperactivity may be a symptom of hormonal disorders, such as hyperthyroidism, hyperkinetic and resistance to thyroid hormone. Hyperactivity may also be a symptom of neuronal disorders, such as adrenoleukodystrophy. Hyperactivity may also be a symptom of hyperkinetic disorder, catatonic schizophrenia, anorexia nervosa, Fragile X Syndrome (FXS), phenylketonuria (PKU), foetal alcohol syndrome (FAS), anxiety, depression and Tourette's syndrome. In certain embodiments, the compositions of the invention are for use in the treatment or prevention of behavioural disorders. In certain embodiments, the compositions of the invention are for use in the treatment or prevention of psychiatric disorders. In certain embodiments, the compositions of the invention are for use in the treatment of emotional and behavioural disorders.

In certain embodiments, the compositions are for use in reducing hyperactivity in the treatment of hyperthyroidism or resistance to thyroid hormone. In certain embodiments, the compositions of the invention are for use in treating hyperactivity in a patient diagnosed with hyperthyroidism or resistance to thyroid hormone.

In certain embodiments, the compositions are for use in reducing hyperactivity in the treatment of adrenoleukodystrophy. In certain embodiments, the compositions of the invention are for use in treating hyperactivity in a patient diagnosed with adrenoleukodystrophy.

In certain embodiments, the compositions are for use in reducing hyperactivity in the treatment of catatonic schizophrenia. In certain embodiments, the compositions of the invention are for use in treating hyperactivity in a patient diagnosed with catatonic schizophrenia.

In certain embodiments, the compositions are for use in reducing hyperactivity in the treatment of anorexia nervosa. In certain embodiments, the compositions of the invention are for use in treating hyperactivity in a patient diagnosed with anorexia nervosa.

In certain embodiments, the compositions are for use in reducing hyperactivity in the treatment of Fragile X Syndrome. In certain embodiments, the compositions of the invention are for use in treating hyperactivity in a patient diagnosed with Fragile X Syndrome (FXS).

In certain embodiments, the compositions are for use in reducing hyperactivity in the treatment of phenylketonuria. In certain embodiments, the compositions of the invention are for use in treating hyperactivity in a patient diagnosed with phenylketonuria.

In certain embodiments, the compositions are for use in reducing hyperactivity in the treatment of foetal alcohol syndrome. In certain embodiments, the compositions of the invention are for use in treating hyperactivity in a patient diagnosed with foetal alcohol syndrome.

In certain embodiments, the compositions are for use in reducing hyperactivity in the treatment of anxiety. In certain embodiments, the compositions of the invention are for use in treating hyperactivity in a patient diagnosed with anxiety.

In certain embodiments, the compositions are for use in reducing hyperactivity in the treatment of depression. In certain embodiments, the compositions of the invention are for use in treating hyperactivity in a patient diagnosed with depression.

In certain embodiments, the compositions are for use in reducing hyperactivity in the treatment of Tourette 's syndrome. In certain embodiments, the compositions of the invention are for use in treating hyperactivity in a patient diagnosed with Tourette's syndrome.

### - ADHD

In certain embodiments, composition of the invention are for use in treating or preventing ADHD. In certain embodiments, the compositions of the invention are for use in the treatment or prevention of hyperactivity in subjects with behavioural disorders. In certain embodiments, the compositions of the invention are for use in the treatment or prevention of hyperactivity in subjects with ADHD. ADHD can manifest in both children and in adults. In some embodiments, the compositions of the invention are for use in the treatment or prevention of ADHD in adults. In some embodiments, the compositions are for use in the treatment or prevention of ADHD in children.

In some embodiments, the compositions are for use in subjects diagnosed with ADHD. Diagnosis of ADHD is complex procedure often involving psychological evaluation of a subject displaying symptoms of ADHD, coupled with physical examination and possibly the detection of biological markers associated with ADHD, such as platelet monoamine oxidase expression, urinary norepinephrine, urinary MHPG, and urinary phenethylamine levels.

Formal diagnosis is typically made by a psychiatric health care professional. Different countries use different metrics for the diagnosis and classification of ADHD. In some countries, diagnosis and classification is made according to the criteria defined by the American Psychiatric Association in the Diagnostic and Statistical Manual of Mental Disorders (DSM). The DSM classifies ADHD in different sub-types depending on the array of symptoms exhibited by the subject. ADHD may be diagnosed as ADHD predominantly inattentive type (ADHD-pi). In certain embodiments, the compositions of the invention are for use in the treatment or prevention of ADHD-pi. In some embodiments, the compositions of the invention are for use in a subject diagnosed with ADHD-pi. In some embodiments, the compositions of the invention are for use in a method of treating a subject diagnosed with ADHD-pi. ADHD may also be diagnosed as ADHD predominantly hyperactive-impulsive type. In some embodiments, the compositions are for use in the treatment or prevention of ADHD predominantly hyperactive-impulsive type. In some embodiments, the compositions of the invention are for use in a subject diagnosed with ADHD predominantly hyperactive-impulsive type. In some embodiments, the compositions of the invention are for use in a method of treatment of a subject diagnosed with ADHD predominantly hyperactive-impulsive type.

Symptoms of ADHD include being easily distracted, forgetful, daydreaming, disorganization, poor concentration, and difficulty completing tasks, with excessive fidgetiness and restlessness, hyperactivity, difficulty waiting and remaining seated, immature behavior. Destructive behaviors may also be present. For symptoms to be associated with ADHD, they must be present for more than six months, and must appear in more than one environment (such as at home and at school or work). In certain embodiments, the compositions are for use in treating or preventing one or more symptoms of ADHD. In certain embodiments, the compositions are for use in the treatment or prevention of a subj ect displaying one or more symptoms of ADHD. In some embodiments, the compositions of the invention are for use in the treatment or prevention of hyperactivity. In some embodiments, the compositions are for use in a method of reducing hyperactivity in a subject. In some embodiments, the compositions of the invention are for use as anti-hyperactivity medicaments.

Other methods of treatment of ADHD include psychological therapy, behavioral therapy, cognitive behavioral therapy, interpersonal psychotherapy, stimulant medications, such as methtylphenidate, non-stimulant medications, such as atomoxetine, bupropion, guanfacine and clonidine. In certain embodiments, the compositions of the invention are for use in combination with an additional method of treatment for ADHD.

### - Obsessive compulsive disorder (OCD)

In certain embodiments, the compositions of the invention are for use in treating or preventing OCD. In certain embodiments, the compositions are for use in reducing hyperactivity in the treatment of OCD. In certain embodiments, the compositions of the invention are for use in treating hyperactivity in a patient diagnosed with OCD.

OCD is a heterogeneous, chronic and disabling disorder belonging to the anxiety disorders. According to the DSM-IV definition, the essential features of OCD are recurrent obsessions and/or compulsions (criterion A) that are severe and time consuming (more than one hour a day) or cause marked distress or significantly interfere with the subject's normal routine, occupational functioning, usual social activities or relationships (criterion C). As some point during the course of the disorder, the person has recognised that the obsessions or compulsions are excessive or unreasonable (criterion B).

Obsessions are defined as recurrent and persistent thoughts, impulses or images that are experienced as intrusive and inappropriate and cause marked anxiety or distress. The thoughts, impulses or images are not simply excessive worries about real-life problems, they are recognised by the patient as a product of his own mind (e.g. fear for contamination, symmetry obsession). The person attempts to ignore, suppress or neutralise the obsessions with some other thoughts or actions.

Compulsions are defined as repetitive behaviours (e.g. hand washing, ordering, hoarding, checking) or mental acts (e.g. praying, counting, repeating words silently) that the person feels driven to perform in response to an obsession or according to rules that must be applied rigidly.

OCD is often associated with co-morbidity rates of other psychiatric diseases including major depressive disorder, other anxiety disorders (generalised anxiety disorder, social anxiety disorder, panic disorder), substance abuse and eating disorders (anorexia and bulimia).

OCD is a psychiatric disorder that may develop or persist due to dysfunction of the microbiota-gut-brain axis. Accordingly, in preferred embodiments, the compositions of the invention are for use in treating or preventing OCD in a subject.

In certain embodiments, the compositions of the invention prevent, reduce or alleviate the essential symptomatic features of OCD. In certain embodiments, the compositions of the invention prevent, reduce or alleviate recurrent obsessions and/or compulsions in a subject. In certain embodiments, the obsessions are recurrent or persistent thoughts, impulses or images that are experiences as intrusive and inappropriate and cause marked anxiety or distress. In certain embodiments, the compulsions are repetitive behaviours that the subject feels driven to perform in response to an obsession or according to rules that must be applied rigidly.

In certain embodiments, the compositions of the invention improve symptoms of OCD in a subject accordingly to the Y-BOCS and/or the NIMH-OC diagnostic and/or symptomatic scales. In some embodiments, the Y-BOCS scale is used to monitor improvement of primary endpoints. In some embodiments, the NIMH-OC scale is used to monitor improvement of secondary parameters.

In some embodiments, the compositions of the invention improve the Clinical Global Impression - Global Improvement (CGI-I) scale for assessing psychiatric and neurological disorders. In some embodiments, the compositions of the invention display a positive effect on global social functioning (relationships, work, *etc*.) of the subject with ASDs. In some embodiments, the global scale is the Sheehan disability scale.

In preferred embodiments, the compositions of the invention prevent, reduce or alleviate at least one comorbidity of OCD. The comorbidities of OCD include major depressive disorder, other anxiety disorders (generalised anxiety disorder, social anxiety disorder, panic disorder), substance abuse and eating disorders (anorexia and bulimia) Gilles de la Tourette syndrome, ADHD (Attention-Deficit/Hyperactivity Disorder) and developmental disorders.

In some embodiments, the compositions of the invention are particularly effective at preventing, reducing or alleviating OCD when used in combination with another therapy for treating OCD. Such therapies include serotonin and dopamine reuptake inhibitors; clomipramine and anti-psychotics.

### - Anxiety disorders

In certain embodiments, the compositions of the invention are for use in treating or preventing anxiety disorders. In certain embodiments, the compositions are for use in reducing hyperactivity in the treatment of an anxiety disorder. In certain embodiments, the compositions of the invention are for use in treating hyperactivity in a patient diagnosed with an anxiety disorder.

Anxiety disorders are a group of mental disorders characterised by feelings of anxiety and fear. There are a number of anxiety disorders including generalised anxiety disorder (GAD); specific phobia; social anxiety disorder; separation anxiety disorder; agroraphobia; panic disorder and selective mutism.

GAD is diagnosed according to DMS-5 in six criterion. The first criterion is too much anxiety or worry over more than six months wherein the anxiety or worry is present most of the time in regards to many activities. The second criterion is that the subject is unable to manage the symptoms of the first criterion. The third criterion is that at least three (one in children) of the following occurs: restlessness; tires easily; problems concentrating; irritability; muscle tension and problems with sleep. The final three criterion are that the symptoms results in significant social, occupational and functional impairment; the symptoms are not due to medications, drugs, or other physical health problems; and the symptoms do not fit better with another psychiatric problem such as panic disorder. All other anxiety disorders may be considered as differential diagnoses of GAD

GAD is frequently associated with a wide spectrum of other mental disorders as comorbidities including depression; substance use disorders; stress; IBS; insomnia; headaches; pain; cardiac events; interpersonal problems and ADHD

Anxiety disorders are psychiatric disorders that may develop or persist due to dysfunction of the microbiota-gut-brain axis. Accordingly, in preferred embodiments, the compositions of the invention are for use in treating or preventing anxiety disorders in a subject. In certain embodiments, the anxiety disorder is generalised anxiety disorder (GAD); specific phobia; social anxiety disorder; separation anxiety disorder; agoraphobia; panic disorder and selective mutism.

In certain embodiments, the compositions of the invention prevent, reduce or alleviate one or more of the symptoms of GAD in a subject as classified by the DMS-5 criteria listed herein. According to DMS-5, the same symptoms are associated with other anxiety disorders. Therefore, in certain embodiments, the compositions of the invention prevent, reduce or alleviate one or more of the symptoms of anxiety disorders in a subject. In preferred embodiments, the compositions of the invention prevent, reduce or alleviate the anxiety or worry of the subject. In certain embodiments, the compositions of the invention reduce the occurrence of symptoms within a six month period. In certain embodiments, the composition of the invention prevents, reduces or alleviates restlessness; fatigue; loss of concentration; irritability; muscle tension; and/or problems with sleep. In some embodiments, the compositions of the invention prevent, reduce or alleviate social, occupational and functional impairment associated with anxiety disorders.

In some embodiments, the compositions of the invention improve the symptoms of anxiety disorders according to a symptomatic or diagnostic scale. In certain embodiments, the scale for assessing symptomatic improvement includes the Hamilton Anxiety Rating Scale (HAM-A). In some embodiments, the HAM-A total scale is used to assess primary endpoint. In other embodiments, the HAM-A psychic anxiety factor may be useful as a secondary endpoint.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairment of the subject with anxiety disorder. In some embodiments, the global scale is the Sheehan disability scale.

In preferred embodiments, the compositions of the invention prevent, reduce or alleviate at least one comorbidity of GAD and anxiety disorders. The comorbidities of GAD include depression; substance use disorders; stress; IBS; insomnia; headaches; pain; cardiac events; interpersonal problems and ADHD

In some embodiments, the compositions of the invention are particularly effective at preventing, reducing or alleviating anxiety disorders when used in combination with another therapy for treating anxiety disorders. Such therapies include selective serotonin reuptake inhibitors (venlafaxine, duloxetine, escitalopram and paroxetine); benzodiazepines (alprazolam, lorazepam and clonazepam); pregabalin (Lyrica®) and gabapentin (Neurontin ®); serotonin receptor partial agonists (buspirone and tandospirone); atypical serotonergic antidepressants (such as imipramine and clomipramine); monoamine oxidase inhibitors (MAOIs) (such as moclobemide and phenelzine); hydroxyzine; propranolol; clonidine; guanfacine and prazosin.

### - Post-traumatic stress disorder (PTSD)

In certain embodiments, the compositions of the invention are for use in treating or preventing PTSD. In certain embodiments, the compositions are for use in reducing hyperactivity in the treatment of PTSD. In certain embodiments, the compositions of the invention are for use in treating hyperactivity in a patient diagnosed with PTSD.

PTSD is a severe and disabling disorder, an essential feature of which is the inclusion of a traumatic event as a precipitating factor of this disorder.

The symptoms of PTSD are grouped into four main clusters according to the DMS-V criteria: (i) intrusion: examples include nightmares, unwanted thoughts of the traumatic events, flashbacks, and reacting to traumatic reminders with emotional distress or physiological reactivity; (ii) avoidance: examples include avoiding triggers for traumatic memories including places, conversations, or other reminders; (iii) negative alterations in cognitions and mood: examples include distorted blame of self or others for the traumatic event, negative beliefs about oneself or the world, persistent negative emotions (e.g., fear, guilt, shame), feeling alienated, and constricted affect (e.g., inability to experience positive emotions); (iv) alterations in arousal and reactivity: examples include angry, reckless, or self-destructive behaviour, sleep problems, concentration problems, increased startle response, and hypervigilance.

Symptoms that resolve within 4 weeks of the traumatic event meet the criteria for an Acute Stress Disorder. The DSM distinguishes between acute (duration of symptoms for less than three months) and chronic PTSD (duration of symptoms longer than 3 months). If the symptoms begin more than 6 months after the stressor, the disorder is defined as delayed onset PTSD.

PTSD carries high comorbidities with major depressive disorder and substance use disorders.

PTSD is a psychiatric disorder that may develop or persist due to dysfunction of the microbiota-gut-brain axis. Accordingly, in preferred embodiments, the compositions of the invention are for use in treating or preventing PTSD in a subject. According to a similar pathogenesis, in certain embodiments, the compositions of the invention are for use in treating or preventing stress disorders. In certain embodiments, the compositions of the invention treat acute stress disorder. In some embodiments, the compositions of the invention treat acute and/or chronic PTSD. In some embodiments, the compositions of the invention treat delayed onset PTSD.

In certain embodiments, the compositions of the invention prevent, reduce or alleviate one or more of the symptoms of PTSD (or stress disorder) in a subject as classified by the DMS-5 criteria listed herein. In preferred embodiments, the compositions of the invention prevent, reduce or alleviate intrusive thoughts in a subject with PTSD. In preferred embodiments, the compositions of the invention prevent, reduce or alleviate avoidance behaviour in a subject with PTSD. In preferred embodiments, the compositions of the invention prevent, reduce or alleviate negative alterations in cognitions and mood in a subject with PTSD. In preferred embodiments, the compositions of the invention prevent alterations in arousal and reactivity in a subject with PTSD.

In some embodiments, the compositions of the invention improve the symptoms of PTSD and stress disorders according to a symptomatic or diagnostic scale. In certain embodiments, the scale for assessing symptomatic improvement is the Clinical-Administered PTSD (CAPS) scale.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairment of the subject with PTSD and stress disorders. In some embodiments, the global scale is the Sheehan disability scale.

In preferred embodiments, the compositions of the invention prevent, reduce or alleviate at least one comorbidity of PTSD and stress disorders. The comorbidities of PTSD and stress disorders include MDD, substance use disorders; stress and anxiety.

In some embodiments, the compositions of the invention are particularly effective at preventing, reducing or alleviating PTSD and stress disorders when used in combination with another therapy for treating PTSD and stress disorders. Such therapies include serotoninergic agents, tricyclic antidepressants, mood stabilisers, adrenergic inhibiting agents, antipsychotics, benzodiazepines, sertraline (Zoloft®), fluoxetine (Prozac®) and/or paroxetine (Paxil®).

### - Bipolar disorder

In certain embodiments, the compositions of the invention are for use in treating or preventing bipolar disorder. In certain embodiments, the compositions are for use in reducing hyperactivity in the treatment of bipolar disorder. In certain embodiments, the compositions of the invention are for use in treating hyperactivity in a patient diagnosed with bipolar disorder.

Bipolar disorder in general is a chronic disease. Mania is the cardinal symptom of bipolar disorder. There are several types of bipolar disorder based upon the specific duration and pattern of manic and depressive episodes. In DMS-5, a distinction is made between bipolar I disorder, bipolar II disorder, cyclothymic disorder, rapid-cycling bipolar disorder and bipolar disorder NOS.

According to the DSM, mania is a distinct period of abnormally and persistently elevated, expansive, or irritable mood. The episode must last a week, and the mood must have at least three of the following symptoms: high self-esteem; reduced need for sleep; increase rate of speech; rapid jumping of ideas; easily distracted; an increased interest in goals or activities; psychomotor agitation; increased pursuit of activities with a high risk of danger.

Bipolar I disorder involves one or more manic or mixed (mania and depression) episodes and at least one major depressive episode (see above for symptoms of MDD episodes). Bipolar II disorder has one or more major depressive episodes accompanied by at least one hypomanic episode. There are no manic or mixed episodes. Hypomania is a lesser form of mania. The symptoms are responsible for significant social, occupational and functional impairments. Cyclothymia is characterized by changing low-level depression along with periods of hypomania. The symptoms must be present for at least two years in adults or one year in children before a diagnosis can be made. Symptom free periods in adults and children last no longer than two months or one month, respectively. Rapid cycling bipolar disorder is a severe form of bipolar disorder. It occurs when a person has at least four episodes of major depression, mania, hypomania, or mixed states within a year. Not-otherwise specified (NOS) bipolar disorder classified bipolar symptoms that do not clearly fit into other types. NOS is diagnosed when multiple bipolar symptoms are present but not enough to meet the label for any of the other subtypes.

Bipolar disorder is associated with the following comorbidities: ADHD; anxiety disorders; substance disorders; obesity and metabolic syndrome.

Bipolar disorder is a psychiatric disorder that may develop or persist due to dysfunction of the microbiota-gut-brain axis. Therefore, in preferred embodiments, the compositions of the invention are for use in treating or preventing bipolar disorder in a subject. In certain embodiments, the bipolar disorder is bipolar I disorder. In certain embodiments, the bipolar disorder is bipolar II disorder. In certain embodiments, the bipolar disorder is cyclothymic disorder. In certain embodiments, the bipolar disorder is rapid-cycling bipolar disorder. In certain embodiments, the bipolar disorder is bipolar disorder NOS.

In preferred embodiments, the compositions of the invention prevent, reduce or alleviate one or more of the symptoms of bipolar disorder in a subject. In certain embodiments, the compositions of the invention prevent, reduce or alleviate the occurrence of manic episodes in a subject. In certain embodiments, the compositions of the invention prevent, reduce or alleviate the occurrence of an abnormally and persistently elevated, expansive, or irritable mood. In certain embodiments, the compositions of the invention prevent, reduce or alleviate one or more of the following symptoms: high self-esteem; reduced need for sleep; increase rate of speech; rapid jumping of ideas; easily distracted; an increased interest in goals or activities; psychomotor agitation; increased pursuit of activities with a high risk of danger. In certain embodiments, the compositions of the invention prevent, reduce or alleviate the occurrence of one or more manic or mixed episodes in a subject. In certain embodiments, the compositions of the invention reduce the occurrence of at least one major depressive episode in a subject. In certain embodiments, the compositions of the invention prevent, reduce or alleviate the occurrence of at least one major depressive episode accompanied by at least one hypomanic episode.

In preferred embodiments, the compositions of the invention treat the acute phase of bipolar disorder and/or prevent the occurrence of further episodes. In certain embodiments, the compositions of the invention treat the acute phase of manic/depressive episodes in a subject with bipolar disorder and prevent occurrence of further manic/depressive episodes.

In some embodiments, the compositions of the invention improve the symptoms of bipolar disorder according to a symptomatic or diagnostic scale. In certain embodiments, the scale for assessing symptomatic improvement of manic episodes is the Manic State Rating Scale and the Young Mania Rating Scale. In certain embodiments, the scale is the Bech-Rafaelsen Mania Scale (BRMAS). In certain embodiments, scales for assessing symptomatic improvement of the switch from manic to depressive episodes include the Hamilton Depression Rating Scale, the Montgomery-Asberg Rating Scale, and the Bech-Rafaelsen Depression Scale.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairments of the subject with bipolar disorder.

In preferred embodiments, the compositions of the invention prevent, reduce or alleviate at least one comorbidity of bipolar disorder. In certain embodiments, the comorbidity is selected from ADHD, anxiety disorders, substance disorder, obesity and metabolic syndrome.

In certain embodiments, the compositions of the invention are for use in treating or preventing manic-depressive illness and bipolar disorder unresponsive to lithium and divalproex.

In some embodiments, the compositions of the invention are particularly effective at preventing, reducing or alleviating bipolar disorder when used in combination with another therapy for treating bipolar disorder. In certain embodiments, such therapies include lithium carbonate, anticonvulsant drugs (including valproate, divalproex, carbamazepine and lamotrigine) and antipsychotic drugs (including aripiprazole, olanzapine, quetiapine and risperidone).

### Cancer

HDAC function and expression is perturbed in a variety of cancers and often leads to poor prognosis. HDAC function in cancer is associated with the aberrant expression or function of genes that promote cellular proliferation and tumorigenic phenotypes. In certain cancers HDACs primarily regulate the onset of cancer and are described as oncogenes. In other cancers onco-fusion proteins recruit Class I HDACs to repress the expression of genes that regulate cellular differentiation or cell cycle control, leading to cellular transformation. The knockdown or inhibition of HDAC expression has been shown to have multiple anti-cancer effects, such as cell cycle arrest and inhibition of proliferation, apoptosis, differentiation and senescence and disruption of angiogenesis. Therefore, the compositions of the invention may be useful in the treatment of cancers mediated by HDAC activity, by inhibiting HDAC activity.

In certain embodiments, the compositions of the invention are for use in treating or preventing cancer. In certain embodiments, the composition of the invention are for use in treating or preventing cancers mediated by elevated HDAC activity. In certain embodiments, the compositions of the invention are for use in treating or preventing colorectal cancer.

In certain embodiments, treatment with the compositions of the invention results in a reduction in tumour size or a reduction in tumour growth. In certain embodiments, the compositions of the invention are for use in reducing tumour size or reducing tumour growth. The compositions of the invention may be effective for reducing tumour size or growth. In certain embodiments, the compositions of the invention are for use in patients with solid tumours. In certain embodiments, the compositions of the invention are for use in reducing or preventing angiogenesis in the treatment of cancer. Genes regulated by HDACs have central roles in angiogenesis. In certain embodiments, the compositions of the invention are for use in preventing metastasis.

In certain embodiments, the compositions of the invention are for use in treating or preventing gastric cancer. HDAC2 has been shown to play a functional role in the development of gastric cancers and colorectal tumorigenesis [31,32]. In mice models of colorectal cancer, inhibition of HDAC2 resulted in a reduced rates of tumour development. In certain embodiments, the compositions of the invention that selectively inhibit HDAC2 are for use in treating or preventing colorectal cancer, in particular colorectal cancer mediated by elevated HDAC2 activity.

In certain embodiments, the compositions of the invention are for use in treating or preventing breast cancer. The compositions of the invention may be effective for treating breast cancer, and HDACs have been shown to be upregulated in breast cancer [33]. In certain embodiments, the compositions of the invention are for use in reducing tumour size, reducing tumour growth, or reducing angiogenesis in the treatment of breast cancer.

In certain embodiments, the compositions of the invention are for use in treating or preventing prostate cancer. The compositions of the invention may be effective for treating prostate cancer, as HDAC activity play a major role in the development of prostate cancer [34]. In certain embodiments, the compositions of the invention are for use in reducing tumour size, reducing tumour growth, or reducing angiogenesis in the treatment of prostate cancer. In certain embodiments, the cancer is hormone refractory prostate cancer.

In certain embodiments, the compositions of the invention are for use in treating or preventing lung cancer. The compositions of the invention may be effective for treating lung cancer, and HDACs have been shown to be upregulated in lung cancer [35]. In certain embodiments, the compositions of the invention are for use in reducing tumour size, reducing tumour growth, or reducing angiogenesis in the treatment of lung cancer. In preferred embodiments the cancer is lung carcinoma. In preferred embodiments, the compositions are for use in the treatment of lung cancer with high levels of expression of HDAC2. Certain lung cancer tissues have be shown to abundantly express HDAC2. Inactivation of HDAC2 represses lung cancer cell growth. High levels of HDAC2 activity has been shown to repress p53 activity [36]. Active p53 arrests cell division and ultimately leads to the onset of apoptosis. In certain embodiments, compositions of the invention that inhibit HDAC2 are for use in the treatment of lung cancers with high levels of HDAC2 activity.

In certain embodiments, the compositions of the invention are for use in treating or preventing liver cancer. The compositions of the invention may be effective for treating liver cancer, and HDACs have been shown to be upregulated in liver cancer [37]. In certain embodiments, the compositions of the invention are for use in reducing tumour size, reducing tumour growth, or reducing angiogenesis in the treatment of liver cancer. In preferred embodiments the cancer is hepatoma (hepatocellular carcinoma). In certain embodiments, the cancer is a low-grade or early-stage tumour

In certain embodiments, the compositions of the invention are for use in treating or preventing carcinoma. The compositions of the invention may be particularly effective for treating carcinoma. In certain embodiments, the compositions of the invention are for use in treating or preventing non-immunogenic cancer. The compositions of the invention may be effective for treating non-immunogenic cancers.

In further embodiments, the compositions of the invention are for use in treating or preventing acute lymphoblastic leukemia (ALL), acute myeloid leukemia, adrenocortical carcinoma, basal-cell carcinoma, bile duct cancer, bladder cancer, bone tumor, osteosarcoma/malignant fibrous histiocytoma, brainstem glioma, brain tumor, cerebellar astrocytoma, cerebral astrocytoma/malignant glioma, ependymoma, medulloblastoma, supratentorial primitive neuroectodermal tumors, breast cancer, bronchial adenomas/carcinoids, Burkitt's lymphoma, carcinoid tumor, cervical cancer, chronic lymphocytic leukemia, chronic myelogenous leukemia, chronic myeloproliferative disorders, colon cancer, cutaneous T-cell lymphoma, endometrial cancer, ependymoma, esophageal cancer, Ewing's sarcoma, intraocular melanoma, retinoblastoma, gallbladder cancer, gastric cancer, gastrointestinal carcinoid tumor, gastrointestinal stromal tumor (GIST), germ cell tumor, glioma, childhood visual pathway and hypothalamic, Hodgkin lymphoma, melanoma, islet cell carcinoma, Kaposi sarcoma, renal cell cancer, laryngeal cancer, leukaemias, lymphomas, mesothelioma, neuroblastoma, non-Hodgkin lymphoma, oropharyngeal cancer, osteosarcoma, ovarian cancer, pancreatic cancer, parathyroid cancer, pharyngeal cancer, pituitary adenoma, plasma cell neoplasia, prostate cancer, renal cell carcinoma, retinoblastoma, sarcoma, testicular cancer, thyroid cancer, or uterine cancer.

The compositions of the invention may be particularly effective when used in combination with further therapeutic agents. The HDAC inhibitory effects of the compositions of the invention may be effective when combined with more direct anti-cancer agents. Therefore, in certain embodiments, the invention provides a composition comprising a bacterial strain of the genus *Bariatricus* and an anticancer agent. In preferred embodiments the anticancer agent is an immune checkpoint inhibitor, a targeted antibody immunotherapy, a CAR-T cell therapy, an oncolytic virus, or a cytostatic drug. In preferred embodiments, the composition comprises an anti-cancer agent selected from the group consisting of: Yervoy (ipilimumab, BMS); Keytruda (pembrolizumab, Merck); Opdivo (nivolumab, BMS); MEDI4736 (AZ/MedImmune); MPDL3280A (Roche/Genentech); Tremelimumab (AZ/MedImmune); CT-011 (pidilizumab, CureTech); BMS-986015 (lirilumab, BMS); MEDI0680 (AZ/MedImmune); MSB-0010718C (Merck); PF-05082566 (Pfizer); MEDI6469 (AZ/MedImmune); BMS-986016 (BMS); BMS-663513 (urelumab, BMS); IMP321 (Prima Biomed); LAG525 (Novartis); ARGX-110 (arGEN-X); PF-05082466 (Pfizer); CDX-1127 (varlilumab; CellDex Therapeutics); TRX-518 (GITR Inc.); MK-4166 (Merck); JTX-2011 (Jounce Therapeutics); ARGX-115 (arGEN-X); NLG-9189 (indoximod, NewLink Genetics); INCB024360 (Incyte); IPH2201 (Innate Immotherapeutics/AZ); NLG-919 (NewLink Genetics); anti-VISTA (JnJ); Epacadostat (INCB24360, Incyte); F001287 (Flexus/BMS); CP 870893 (University of Pennsylvania); MGA271 (Macrogenix); Emactuzumab (Roche/Genentech); Galunisertib (Eli Lilly); Ulocuplumab (BMS); BKT140/BL8040 (Biokine Therapeutics); Bavituximab (Peregrine Pharmaceuticals); CC 90002 (Celgene); 852A (Pfizer); VTX-2337 (VentiRx Pharmaceuticals); IMO-2055 (Hybridon, Idera Pharmaceuticals); LY2157299 (Eli Lilly); EW-7197 (Ewha Women's University, Korea); Vemurafenib (Plexxikon); Dabrafenib (Genentech/GSK); BMS-777607 (BMS); BLZ945 (Memorial Sloan-Kettering Cancer Centre); Unituxin (dinutuximab, United Therapeutics Corporation); Blincyto (blinatumomab, Amgen); Cyramza (ramucirumab, Eli Lilly); Gazyva (obinutuzumab, Roche/Biogen); Kadcyla (adotrastuzumab emtansine, Roche/Genentech); Perjeta (pertuzumab, Roche/Genentech); Adcetris (brentuximab vedotin, TakedalMillennium); Arzerra (ofatumumab, GSK); Vectibix (panitumumab, Amgen); Avastin (bevacizumab, Roche/Genentech); Erbitux (cetuximab, BMS/Merck); Bexxar (tositumomab-I131, GSK); Zevalin (ibritumomab tiuxetan, Biogen); Campath (alemtuzumab, Bayer); Mylotarg (gemtuzumab ozogamicin, Pfizer); Herceptin (trastuzumab, Roche/Genentech); Rituxan (rituximab, Genentech/Biogen); volociximab (Abbvie); Enavatuzumab (Abbvie); ABT-414 (Abbvie); Elotuzumab (Abbvie/BMS); ALX-0141 (Ablynx); Ozaralizumab (Ablynx); Actimab-C (Actinium); Actimab-P (Actinium); Milatuzumab-dox (Actinium); Emab-SN-38 (Actinium); Naptumonmab estafenatox (Active Biotech); AFM13 (Affimed); AFM11 (Affimed); AGS-16C3F (Agensys); AGS-16M8F (Agensys); AGS-22ME (Agensys); AGS-15ME (Agensys); GS-67E (Agensys); ALXN6000 (samalizumab, Alexion); ALT-836 (Altor Bioscience); ALT-801 (Altor Bioscience); ALT-803 (Altor Bioscience); AMG780 (Amgen); AMG 228 (Amgen); AMG820 (Amgen); AMG172 (Amgen); AMG595 (Amgen); AMG110 (Amgen); AMG232 (adecatumumab, Amgen); AMG211 (Amgen/MedImmune); BAY20-10112 (Amgen/Bayer); Rilotumumab (Amgen); Denosumab (Amgen); AMP-514 (Amgen); MEDI575 (AZ/MedImmune); MEDI3617 (AZ/MedImmune); MEDI6383 (AZ/MedImmune); MEDI551 (AZ/MedImmune); Moxetumomab pasudotox (AZ/MedImmune); MEDI565 (AZ/MedImmune); MEDI0639 (AZ/MedImmune); MEDI0680 (AZ/MedImmune); MEDI562 (AZ/MedImmune); AV-380 (AVEO); AV203 (AVEO); AV299 (AVEO); BAY79-4620 (Bayer); Anetumab ravtansine (Bayer); vantictumab (Bayer); BAY94-9343 (Bayer); Sibrotuzumab (Boehringer Ingleheim); BI-836845 (Boehringer Ingleheim); B-701 (BioClin); BIIB015 (Biogen); Obinutuzumab (Biogen/Genentech); BI-505 (Bioinvent); BI-1206 (Bioinvent); TB-403 (Bioinvent); BT-062 (Biotest) BIL-010t (Biosceptre); MDX-1203 (BMS); MDX-1204 (BMS); Necitumumab (BMS); CAN-4 (Cantargia AB); CDX-011 (Celldex); CDX1401 (Celldex); CDX301 (Celldex); U3-1565 (Daiichi Sankyo); patritumab (Daiichi Sankyo); tigatuzumab (Daiichi Sankyo); nimotuzumab (Daiichi Sankyo); DS-8895 (Daiichi Sankyo); DS-8873 (Daiichi Sankyo); DS-5573 (Daiichi Sankyo); MORab-004 (Eisai); MORab-009 (Eisai); MORab-003 (Eisai); MORab-066 (Eisai); LY3012207 (Eli Lilly); LY2875358 (Eli Lilly); LY2812176 (Eli Lilly); LY3012217(Eli Lilly); LY2495655 (Eli Lilly); LY3012212 (Eli Lilly); LY3012211 (Eli Lilly); LY3009806 (Eli Lilly); cixutumumab (Eli Lilly); Flanvotumab (Eli Lilly); IMC-TR1 (Eli Lilly); Ramucirumab (Eli Lilly); Tabalumab (Eli Lilly); Zanolimumab (Emergent Biosolution); FG-3019 (FibroGen); FPA008 (Five Prime Therapeutics); FP-1039 (Five Prime Therapeutics); FPA144 (Five Prime Therapeutics); catumaxomab (Fresenius Biotech); IMAB362 (Ganymed); IMAB027 (Ganymed); HuMax-CD74 (Genmab); HuMax-TFADC (Genmab); GS-5745 (Gilead); GS-6624 (Gilead); OMP-21M18 (demcizumab, GSK); mapatumumab (GSK); IMGN289 (ImmunoGen); IMGN901 (ImmunoGen); IMGN853 (ImmunoGen); IMGN529 (ImmunoGen); IMMU-130 (Immunomedics); milatuzumab-dox (Immunomedics); IMMU-115 (Immunomedics); IMMU-132 (Immunomedics); IMMU-106 (Immunomedics); IMMU-102 (Immunomedics); Epratuzumab (Immunomedics); Clivatuzumab (Immunomedics); IPH41 (Innate Immunotherapeutics); Daratumumab (Janssen/Genmab); CNTO-95 (Intetumumab, Janssen); CNTO-328 (siltuximab, Janssen); KB004 (KaloBios); mogamulizumab (Kyowa Hakko Kirrin); KW-2871 (ecromeximab, Life Science); Sonepcizumab (Lpath); Margetuximab (Macrogenics); Enoblituzumab (Macrogenics); MGD006 (Macrogenics); MGF007 (Macrogenics); MK-0646 (dalotuzumab, Merck); MK-3475 (Merck); Sym004 (Symphogen/Merck Serono); DI17E6 (Merck Serono); MOR208 (Morphosys); MOR202 (Morphosys); Xmab5574 (Morphosys); BPC-1C (ensituximab, Precision Biologics); TAS266 (Novartis); LFA102 (Novartis); BHQ880 (Novartis/Morphosys); QGE031 (Novartis); HCD122 (lucatumumab, Novartis); LJM716 (Novartis); AT355 (Novartis); OMP-21M18 (Demcizumab, OncoMed); OMP52M51 (Oncomed/GSK); OMP-59R5 (Oncomed/GSK); vantictumab (Oncomed/Bayer); CMC-544 (inotuzumab ozogamicin, Pfizer); PF-03446962 (Pfizer); PF-04856884 (Pfizer); PSMA-ADC (Progenics); REGN1400 (Regeneron); REGN910 (nesvacumab, Regeneron/Sanofi); REGN421 (enoticumab, Regeneron/Sanofi); RG7221, RG7356, RG7155, RG7444, RG7116, RG7458, RG7598, RG7599, RG7600, RG7636, RG7450, RG7593, RG7596, DCDS3410A, RG7414 (parsatuzumab), RG7160 (imgatuzumab), RG7159 (obintuzumab), RG7686, RG3638 (onartuzumab), RG7597 (Roche/Genentech); SAR307746 (Sanofi); SAR566658 (Sanofi); SAR650984 (Sanofi); SAR153192 (Sanofi); SAR3419 (Sanofi); SAR256212 (Sanofi), SGN-LIV1A (lintuzumab, Seattle Genetics); SGN-CD33A (Seattle Genetics); SGN-75 (vorsetuzumab mafodotin, Seattle Genetics); SGN-19A (Seattle Genetics) SGN-CD70A (Seattle Genetics); SEA-CD40 (Seattle Genetics); ibritumomab tiuxetan (Spectrum); MLN0264 (Takeda); ganitumab (Takeda/Amgen); CEP-37250 (Teva); TB-403 (Thrombogenic); VB4-845 (Viventia); Xmab2512 (Xencor); Xmab5574 (Xencor); nimotuzumab (YM Biosciences); Carlumab (Janssen); NY-ESO TCR (Adaptimmune); MAGE-A-10 TCR (Adaptimmune); CTL019 (Novartis); JCAR015 (Juno Therapeutics); KTE-C19 CAR (Kite Pharma); UCART19 (Cellectis); BPX-401 (Bellicum Pharmaceuticals); BPX-601 (Bellicum Pharmaceuticals); ATTCK20 (Unum Therapeutics); CAR-NKG2D (Celyad); Onyx-015 (Onyx Pharmaceuticals); H101 (Shanghai Sunwaybio); DNX-2401 (DNAtrix); VCN-01 (VCN Biosciences); Colo-Adl (PsiOxus Therapeutics); ProstAtak (Advantagene); Oncos-102 (Oncos Therapeutics); CG0070 (Cold Genesys); Pexa-vac (JX-594, Jennerex Biotherapeutics); GL-ONC1 (Genelux); T-VEC (Amgen); G207 (Medigene); HF10 (Takara Bio); SEPREHVIR (HSV1716, Virttu Biologics); OrienX010 (OrienGene Biotechnology); Reolysin (Oncolytics Biotech); SW-001 (Neotropix); Cacatak (CVA21, Viralytics); Alimta (Eli Lilly), cisplatin, oxaliplatin, irinotecan, folinic acid, methotrexate, cyclophosphamide, 5-fluorouracil, Zykadia (Novartis), Tafinlar (GSK), Xalkori (Pfizer), Iressa (AZ), Gilotrif (Boehringer Ingelheim), Tarceva (Astellas Pharma), Halaven (Eisai Pharma), Veliparib (Abbvie), AZD9291 (AZ), Alectinib (Chugai), LDK378 (Novartis), Genetespib (Synta Pharma), Tergenpumatucel-L (NewLink Genetics), GV1001 (Kael-GemVax), Tivantinib (ArQule); Cytoxan (BMS); Oncovin (Eli Lilly); Adriamycin (Pfizer); Gemzar (Eli Lilly); Xeloda (Roche); Ixempra (BMS); Abraxane (Celgene); Trelstar (Debiopharm); Taxotere (Sanofi); Nexavar (Bayer); IMMU-132 (Immunomedics); E7449 (Eisai); Thermodox (Celsion); Cometriq (Exellxis); Lonsurf (Taiho Pharmaceuticals); Camptosar (Pfizer); UFT (Taiho Pharmaceuticals); and TS-1 (Taiho Pharmaceuticals).

### Neurodegenerative diseases

### - Alzheimer's disease and dementia

Aberrant accumulation of hyperphosphorylated tau is a hallmark of neurodegenerative tauopathies such Alzheimer's disease. Reduction in HDAC activity can reduce levels of hyperphosphorylated tau and alleviate symptoms of tau-driven neurological disorders [38]. Therefore, in certain embodiments, the compositions of the invention are for use in the treatment or prevention of neurodegenerative tauopathies. In certain embodiments, the compositions of the invention are for use in the treatment of Alzheimer's disease.

In DSM-5, the term dementia was replaced with the terms major neurocognitive disorder and mild neurocognitive disorder. Neurocognitive disorder is a heterogeneous class of psychiatric diseases. The most common neurocognitive disorder is Alzheimer's disease, followed by vascular dementias or mixed forms of the two. Other forms of neurodegenerative disorders (e.g. Lewy body disease, frontotemporal dementia, Parkinson's dementia, Creutzfeldt-Jakob disease, Huntington's disease, and Wernicke-Korsakoff syndrome) are accompanied by dementia.

Alzheimer's disease and dementia are also characterised by neuronal loss, so the neuroprotective and neuroproliferative effects shown in the examples for the compositions of the invention indicate that they may be useful for treating or preventing these conditions.

The symptomatic criteria for dementia under DSM-5 are evidence of significant cognitive decline from a previous level of performance in one or more cognitive domains selected from: learning and memory; language; executive function; complex attention; perceptual-motor and social cognition. The cognitive deficits must interfere with independence in everyday activities. In addition, the cognitive deficits do not occur exclusively in the context of a delirium and are not better explained by another mental disorder (for example MDD or schizophrenia).

In addition to the primary symptom, subjects with neurodegenerative disorders display behavioural and psychiatric symptoms including agitation, aggression, depression, anxiety, apathy, psychosis and sleep-wake cycle disturbances.

Neurodegenerative disorders may develop or persist due to dysfunction of the microbiota-gut-brain axis. Therefore, in preferred embodiments, the compositions of the invention are for use in treating or preventing neurodegenerative disorders in a subject. In preferred embodiments, the neurodegenerative disorder is Alzheimer's disease. In other embodiments, the neurodegenerative disorder is selected from vascular dementias; mixed form Alzheimer's disease and vascular dementia; Lewy body disease; frontotemporal dementia; Parkinson's dementia; Creutzfeldt-Jakob disease; Huntington's disease; and Wernicke-Korsakoff syndrome.

In preferred embodiments, the compositions of the invention prevent, reduce or alleviate one or more of the symptoms of neurodegenerative disorders in a subject. In certain embodiments, the compositions of the invention prevent, reduce or alleviate the occurrence of cognitive decline in a subject. In certain embodiments, the compositions of the invention improve the level of performance of a subject with neurodegenerative disorders in one or more cognitive domains selected from: learning and memory; language; executive function; complex attention; perceptual-motor and social cognition. In some embodiments, the compositions of the invention prevent, reduce or alleviate the occurrence of one or more behavioural and psychiatric symptoms associated with neurodegenerative disorders selected from agitation, aggression, depression, anxiety, apathy, psychosis and sleep-wake cycle disturbances.

In certain embodiments, the compositions of the invention prevent, reduce or alleviate symptomatic disease by intervention in suspected pathogenic mechanisms at a preclinical stage. In certain embodiments, the compositions of the invention improve disease modification, with slowing or arrest of symptom progression. In some embodiments, the slowing or arrest of symptom progression correlates with evidence in delaying the underlying neuropathological process. In preferred embodiments, the compositions of the invention improve symptoms of neurodegenerative disorders comprising enhanced cognitive and functional improvement. In preferred embodiments, the compositions of the invention improve the behavioural and psychiatric symptoms of dementia (BPSD). In preferred embodiments, the compositions of the invention improve the ability of a subject with neurodegenerative disorder to undertake everyday activities.

In preferred embodiments, the compositions of the invention improve both cognition and functioning in a subject with Alzheimer's disease. In some embodiments, the composition of the invention improves the cognitive endpoint in a subject with Alzheimer's disease. In some embodiments, the compositions of the invention improve the functional endpoint in a subject with Alzheimer's disease. In preferred embodiments, the compositions of the invention improve the cognitive and functional endpoint in a subject with Alzheimer's disease. In yet further preferred embodiments, the compositions of the invention improve the overall clinical response (the global endpoint) in a subject with Alzheimer's disease.

In some embodiments, the compositions of the invention improve the symptoms of neurodegenerative disorders according to a symptomatic or diagnostic test. In certain embodiments, the tests for assessing symptomatic improvement of Alzheimer's disease (and other neurodegenerative disorders) are selected from objective cognitive, activities of daily living, global assessment of change, health related quality of life tests and tests assessing behavioural and psychiatric symptoms of neurodegenerative disorders.

In certain embodiments, the objective cognitive tests for assessment of symptomatic improvement use the Alzheimer's disease Assessment Scale cognitive subscale (ADAS-cog) and the classic ADAS scale. In certain embodiments, symptomatic improvement of cognition is assessed using the Neurophysiological Test Battery for Use in Alzheimer's Disease (NTB).

In some embodiments, the global assessment of change test uses the Clinical Global Impression - Global Improvement (CGI-I) scale for assessing psychiatric and neurological disorders. In some embodiments, the global scale is the Clinician's Interview Based Impression of Change plus (CIBIC-plus). In some embodiments, the global scale is the Alzheimer's Disease Cooperative Study Unit Clinician's Global Impression of Change (ADCS-CGIC).

In certain embodiments, the health related quality of life measures are the Alzheimer's Disease-Related QOL (ADRQL) and the QOL-Alzheimer's Disease (QOL-AD).

In certain embodiments, the tests assessing behavioural and psychiatric symptoms of neurodegenerative disorders are selected from the Behavioural pathology in Alzheimer's Disease Rating Scale (BEHAVE-AD); the Behavioural Rating Scale for Dementia (BRSD); the Neuropsychiatric Inventory (NPI); and the Cohen-Mansfield Agitation Inventory (CMAI).

In some embodiments, the compositions of the invention are particularly effective at preventing, reducing or alleviating neurodegenerative disorders when used in combination with another therapy for treating neurodegenerative disorders. In certain embodiments, such therapies include acetylcholinesterase inhibitors including donepezil (Aricept®), galantamine (Razadyne®) and rivastigmine (Exelon ®), and memantine.

### - Parkinson's disease

Parkinson's disease is a common neurodegenerative disease neuropathologically characterised by degeneration of heterogeneous populations of neural cells (dopamine-producing cells). The clinical diagnosis of Parkinson's disease requires bradykinesia and at least one of the following core symptoms: resting tremor; muscle rigidity and postural reflex impairment. Other signs and symptoms that may be present or develop during the progression of the disease are autonomic disturbances (sialorrhoea, seborrhoea, constipation, micturition disturbances, sexual functioning, orthostatic hypotension, hyperhydrosis), sleep disturbances and disturbances in the sense of smell or sense of temperature. Parkinson's disease is a neurodegenerative diseases that may develop or persist due to HDAC activity. For example, HDAC activity has been shown to regulate aggregation and deposition toxic intracellular proteinaceous filaments that are a hallmark of neurodegenerative diseases such as Parkinson's disease [39]. Inhibition of HDAC activity has been shown to reduce toxic protein misfolding events in Parkinson's disease models. Therefore, in preferred embodiments, the compositions of the invention are for use in treating or preventing Parkinson's disease in a subject.

In further preferred embodiments, compositions of the invention are for use in a method of treating or preventing Parkinson's disease. Compositions of the invention may improve motor and cognitive functions in models of Parkinson's disease. Treatment with the compositions may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of Parkinson's disease. In preferred embodiments, the invention provides a composition comprising a bacterial strain of the species *Bariatricus massiliensis* for use in a method of treating or preventing Parkinson's disease. Compositions using *Bariatricus* may be particularly effective for treating Parkinson's disease. The composition may further comprise an organic acid.

In preferred embodiments, the compositions of the invention prevent, reduce or alleviate one or more of the symptoms of Parkinson's disease in a subject. In preferred embodiments, the compositions of the invention prevent, reduce or alleviate one or more core symptoms of Parkinson's disease in a subject. In certain embodiments, the compositions of the invention prevent, reduce or alleviate bradykinesia in a subject. In certain embodiments, the compositions of the invention prevent, reduce or alleviate resting tremor; muscle rigidity and/or postural reflex impairment in a subject. In certain embodiments, the compositions of the invention prevent, reduce or alleviate one or more symptoms associated with Parkinson's disease progression selected from autonomic disturbances (sialorrhoea, seborrhoea, constipation, micturition disturbances, sexual functioning, orthostatic hypotension, hyperhydrosis), sleep disturbances and disturbances in the sense of smell or sense of temperature.

In preferred embodiments, the compositions of the invention prevent, reduce or alleviate depressive symptoms comorbid with Parkinson's disease. In certain embodiments, the compositions of the invention improve verbal memory and/or executive functions. In certain embodiments, the compositions of the invention improve attention, working memory, verbal fluency and/or anxiety.

In other preferred embodiments, the compositions of the invention prevent, reduce or alleviate cognitive dysfunctions comorbid with Parkinson's disease.

In certain embodiments, the compositions of the invention prevent, reduce or alleviate hyperactivity or anxiety-like behaviour comorbid with Parkinson's disease. Mice models of Parkinson's disease have been shown to exhibit hyperactivity. Certain models have indicated that hyperactivity may be a consequence of imbalanced neurotransmitter levels in the brain or functional changes in other structures within the brain that precede degeneration of dopaminergic neurons. Thus, behavioural disturbances, such as hyperactivity, may be symptoms of Parkinson's disease that precede the onset of motor disturbances. The compositions of the invention have been shown to reduce hyperactivity in mice models of Parkinson's disease. Therefore, in certain embodiments, the compositions of the invention may be for use in the prevention of motor disturbances in Parkinson's disease. In certain embodiments, the compositions of the invention are for use in the treatment or prevention of behavioural disturbances associated with Parkinson's disease.

In certain embodiments, the compositions of the invention prevent, reduce or alleviate Parkinson's disease progression. In certain embodiments, the compositions of the invention prevent, reduce or alleviate later motor complications. In certain embodiments, the compositions of the invention prevent, reduce or alleviate late motor fluctuations. In certain embodiments, the compositions of the invention prevent, reduce or alleviate neuronal loss. In certain embodiments, the compositions of the invention improve symptoms of Parkinson's disease dementia (PDD). In certain embodiments, the compositions of the invention prevent, reduce or alleviate impairment of executive function, attention and/or working memory. In certain embodiments, the compositions of the invention improve dopaminergic neurotransmission. In certain embodiments, the compositions of the invention prevent, reduce or alleviate impaired dopaminergic neurotransmission.

In some embodiments, the compositions of the invention improve the symptoms of Parkinson's disease according to a symptomatic or diagnostic scale. In certain embodiments, the tests for assessing symptomatic improvement of motor function in Parkinson's disease is the Unified Parkinson's Disease Rating Scale. In particular, UPDRS II considers the activity of daily life and UPDRS III considers motor-examination.

In some embodiments, the compositions of the invention improve the symptoms associated with PDD according to a symptomatic or diagnostic test and/or scale. In certain embodiments, the test or scale is selected from the Hopkins Verbal Learning Test - Revised (HVLT-R); the Delis-Kaplan Executive Function System (D-KEFS) Color-Word Interference Test; the Hamilton Depression Rating Scale (HAM-D 17; depression); the Hamilton Anxiety Rating Scale (HAM-A; anxiety) and the Unified Parkinson's Disease Rating Scale (UPDRS; PD symptom severity).

In some embodiments, the compositions of the invention improve the Clinical Global Impression - Global Improvement (CGI-I) scale for assessing psychiatric and neurological disorders. In some embodiments, the compositions of the invention display a positive effect on global social and occupational impairment of the subject with Parkinson's disease.

In certain embodiments, the compositions of the invention are for use in treating or preventing neurological disorders such as Parkinson's disease in a subject wherein said use involves reducing or preventing loss of dopaminergic cells in the substantia nigra. In certain embodiments, the compositions of the invention are for use in treating or preventing neurological disorders such as Parkinson's disease in a subject wherein said use involves reducing or preventing the degeneration of dopaminergic neurons in the substantia nigra pars compacta. In certain embodiments, the compositions of the invention are for use in treating or preventing neurological disorders such as Parkinson's disease in a subject wherein said use involves reducing or preventing the degeneration of dopaminergic neurons in the substantia nigra pars compacta and the consequent loss of their projecting nerve fibers in the striatum. In certain embodiments, the compositions of the invention are for use in treating or preventing neurological disorders such as Parkinson's disease in a subject wherein said use involves reducing or preventing loss of nigrostriatal dopaminergic neurons.

In certain embodiments, the compositions of the invention are for use in treating or preventing neurological disorders such as Parkinson's disease in a subject wherein said use involves increasing dopamine levels. In certain embodiments, the compositions of the invention are for use in treating or preventing neurological disorders such as Parkinson's disease in a subject wherein said use involves increasing DOPAC levels. In certain embodiments, the compositions of the invention are for use in treating or preventing neurological disorders such as Parkinson's disease in a subject wherein said use involves increasing dopamine and DOPAC levels. In certain embodiments, the dopamine and/or DOPAC levels are increased in the striatum.

The examples demonstrate that the compositions of the invention activate MAP2 (Microtubule - associated protein 2) activation. MAP2 is a gene associated with neuronal differentiation of MAP2 and is thought to be essential for microtubule formation in neuritogenesis, so compositions of the invention may be particularly useful for treating neurodegenerative diseases. In some embodiments, the compositions of the invention are for use in treating a neurodegenerative disease, such as Alzheimer's disease or Parkinson's disease, by activating or increasing the levels of MAP2. Moreover, as MAP2 promotes neurite outgrowth, which play a major role in re-networking of damaged neurons and synaptogenesis, MAP2 expression might go beyond being a marker of neuronal differentiation and indicate "neuronal re-wiring" associated with the therapeutic outcome of neuropathological disease [22].

The examples demonstrate that the compositions of the invention modulate the expression of a number of proteins in the brain. In particular, compositions of the invention increase the expression of BDNF in the hippocampus and the prefrontal cortex. BDNF is essential for adult synaptic plasticity and the formation of memories and a decrease in the levels of BDNF is observed in Alzheimer's and Huntington's patients. The compositions of the invention are therefore particularly useful for the treatment of Alzheimer's and Huntington's disease. In certain embodiments, compositions of the invention increase expression of BDNF in the brain.

### Modes of administration

Preferably, the compositions of the invention are to be administered to the gastrointestinal tract in order to enable delivery to and / or partial or total colonisation of the intestine with the bacterial strain of the invention. Generally, the compositions of the invention are administered orally, but they may be administered rectally, intranasally, or via buccal or sublingual routes.

In certain embodiments, the compositions of the invention may be administered as a foam, as a spray or a gel.

In certain embodiments, the compositions of the invention may be administered as a suppository, such as a rectal suppository, for example in the form of a theobroma oil (cocoa butter), synthetic hard fat (e.g. suppocire, witepsol), glycero-gelatin, polyethylene glycol, or soap glycerin composition.

In certain embodiments, the composition of the invention is administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to the stomach, jejunum and other suitable access ports.

The compositions of the invention may be administered once, or they may be administered sequentially as part of a treatment regimen. In certain embodiments, the compositions of the invention are to be administered daily.

In certain embodiments of the invention, treatment according to the invention is accompanied by assessment of the patient's gut microbiota. Treatment may be repeated if delivery of and / or partial or total colonisation with the strain of the invention is not achieved such that efficacy is not observed, or treatment may be ceased if delivery and / or partial or total colonisation is successful and efficacy is observed.

In certain embodiments, the composition of the invention may be administered to a pregnant animal, for example a mammal such as a human in order to prevent an inflammatory or autoimmune disease developing in her child *in utero* and / or after it is born.

The compositions of the invention may be administered to a patient that has been diagnosed with a disease or condition mediated by elevated histone deacetylase activity, or that has been identified as being at risk of a disease or condition mediated by elevated histone deacetylase activity. The compositions may also be administered as a prophylactic measure to prevent the development of diseases or conditions mediated by elevated histone deacetylase activity in a healthy patient.

The compositions of the invention may be administered to a patient that has been identified as having an abnormal gut microbiota. For example, the patient may have reduced or absent colonisation by *Bariatricus,* and in particular *Bariatricus massiliensis.*

The compositions of the invention may be administered as a food product, such as a nutritional supplement.

Generally, the compositions of the invention are for the treatment of humans, although they may be used to treat animals including monogastric mammals such as poultry, pigs, cats, dogs, horses or rabbits. The compositions of the invention may be useful for enhancing the growth and performance of animals. If administered to animals, oral gavage may be used.

### Compositions

The composition of the invention comprises bacteria. In preferred embodiments of the invention, the composition is formulated in freeze-dried form. For example, the composition of the invention may comprise granules or gelatin capsules, for example hard gelatin capsules, comprising a bacterial strain of the invention.

Preferably, the composition of the invention comprises lyophilised bacteria. Lyophilisation of bacteria is a well-established procedure and relevant guidance is available in, for example, references [40,,42].

Alternatively, the composition of the invention may comprise a live, active bacterial culture.

In preferred embodiments, the composition of the invention is encapsulated to enable delivery of the bacterial strain to the intestine. Encapsulation protects the composition from degradation until delivery at the target location through, for example, rupturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule. Guidance on encapsulation that may be useful for preparing compositions of the invention is available in, for example, references [43] and [44].

The composition may be administered orally and may be in the form of a tablet, capsule or powder. Encapsulated products are preferred because *Bariatricus* are anaerobes. Other ingredients (such as vitamin C, for example), may be included as oxygen scavengers and prebiotic substrates to improve the delivery and / or partial or total colonisation and survival *in vivo.* Alternatively, the probiotic composition of the invention may be administered orally as a food or nutritional product, such as milk or whey based fermented dairy product, or as a pharmaceutical product.

The composition may be formulated as a probiotic.

A composition of the invention includes a therapeutically effective amount of a bacterial strain of the invention. A therapeutically effective amount of a bacterial strain is sufficient to exert a beneficial effect upon a patient. A therapeutically effective amount of a bacterial strain may be sufficient to result in delivery to and / or partial or total colonisation of the patient's intestine.

A suitable daily dose of the bacteria, for example for an adult human, may be from about 1 x 10³ to about 1 x 10¹¹ colony forming units (CFU); for example, from about 1 x 10⁷ to about 1 x 10¹⁰ CFU; in another example from about 1 x 10⁶ to about 1 x 10¹⁰ CFU; in another example from about 1 x 10⁷ to about 1 x 10¹¹ CFU; in another example from about 1 x 10⁸ to about 1 x 10¹⁰ CFU; in another example from about 1 x 10⁸ to about 1 x 10¹¹ CFU.

In certain embodiments, the dose of the bacteria is at least 10⁹ cells per day, such as at least 10¹⁰, at least 10¹¹, or at least 101² cells per day.

In certain embodiments, the composition contains the bacterial strain in an amount of from about 1 x 10⁶ to about 1 x 10¹¹ CFU/g, respect to the weight of the composition; for example, from about 1 x 10⁸ to about 1 x 10¹⁰ CFU/g. The dose may be, for example, 1 g, 3g, 5g, and 10g.

In certain embodiments, the invention provides the above pharmaceutical composition, wherein the amount of the bacterial strain is from about 1 × 10³ to about 1 × 10¹¹ colony forming units per gram with respect to a weight of the composition.

In certain embodiments, the invention provides the above pharmaceutical composition, wherein the composition is administered at a dose of between 500mg and 1000mg, between 600mg and 900mg, between 700mg and 800mg, between 500mg and 750mg or between 750mg and 1000mg. In certain embodiments, the invention provides the above pharmaceutical composition, wherein the lyophilised bacteria in the pharmaceutical composition is administered at a dose of between 500mg and 1000mg, between 600mg and 900mg, between 700mg and 800mg, between 500mg and 750mg or between 750mg and 1000mg.

Typically, a probiotic, such as the composition of the invention, is optionally combined with at least one suitable prebiotic compound. A prebiotic compound is usually a non-digestible carbohydrate such as an oligo- or polysaccharide, or a sugar alcohol, which is not degraded or absorbed in the upper digestive tract. Known prebiotics include commercial products such as inulin and transgalacto-oligosaccharides.

In certain embodiments, the probiotic composition of the present invention includes a prebiotic compound in an amount of from about 1 to about 30% by weight, respect to the total weight composition, (e.g. from 5 to 20% by weight). Carbohydrates may be selected from the group consisting of: fructo- oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

The compositions of the invention may comprise pharmaceutically acceptable excipients or carriers. Examples of such suitable excipients may be found in the reference [45]. Acceptable carriers or diluents for therapeutic use are well known in the pharmaceutical art and are described, for example, in reference [46]. Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

The compositions of the invention may be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the composition of the invention is formulated as a milk-based product. The term "milk-based product" means any liquid or semi-solid milk- or whey- based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks; flavoured milks, ice cream; milk-containing food such as sweets.

In certain embodiments, the compositions of the invention contain a single bacterial strain or species and do not contain any other bacterial strains or species. Such compositions may comprise only *de minimis* or biologically irrelevant amounts of other bacterial strains or species. Such compositions may be a culture that is substantially free from other species of organism. In certain embodiments, the compositions of the invention consist of 1, 2, 3, 4, 5, 6, 7, 8, 9, 10, 11, 12, 13, 14, 15 or 16 bacterial strains or species. In certain embodiments, the compositions consist of from 1 to 10, preferably from 1 to 5 bacterial strains or species.

The compositions for use in accordance with the invention may or may not require marketing approval.

In some cases, the lyophilised bacterial strain is reconstituted prior to administration. In some cases, the reconstitution is by use of a diluent described herein.

The compositions of the invention can comprise pharmaceutically acceptable excipients, diluents or carriers.

In certain embodiments, the invention provides a pharmaceutical composition comprising: a bacterial strain of the invention; and a pharmaceutically acceptable excipient, carrier or diluent; wherein the bacterial strain is in an amount sufficient to treat a disorder when administered to a subject in need thereof; and wherein the disorder is selected from the group consisting of neurodegenerative diseases, such as Alzheimer's disease, Huntington's disease or Parkinson's disease, brain injury, such as stroke, behavioural disorders, such as attention deficit hyperactivity disorder, inflammatory bowel diseases, such as Crohn's disease, cancer, such as prostate cancer, colorectal cancer, breast cancer, lung cancer, liver cancer or gastric cancer.

In certain embodiments, the invention provides pharmaceutical composition comprising: a bacterial strain of the invention; and a pharmaceutically acceptable excipient, carrier or diluent; wherein the bacterial strain is in an amount sufficient to treat or prevent a disease or condition mediated by elevated HDAC activity. In preferred embodiments, said disease or condition is selected from the group consisting of neurodegenerative diseases, such as Alzheimer's disease, Huntington's disease or Parkinson's disease, brain injury, such as stroke, behavioural disorders, such as attention deficit hyperactivity disorder, inflammatory bowel diseases, such as Crohn's disease, cancer, such as prostate cancer, colorectal cancer, breast cancer, lung cancer, liver cancer or gastric cancer.

In certain embodiments, the invention provides the above pharmaceutical composition, wherein the amount of the bacterial strain is from about 1 × 10³ to about 1 × 10¹¹ colony forming units per gram with respect to a weight of the composition.

In certain embodiments, the invention provides the above pharmaceutical composition, wherein the composition is administered at a dose of 1 g, 3 g, 5 g or 10 g.

In certain embodiments, the invention provides the above pharmaceutical composition, wherein the composition is administered by a method selected from the group consisting of oral, rectal, subcutaneous, nasal, buccal, and sublingual.

In certain embodiments, the invention provides the above pharmaceutical composition, comprising a carrier selected from the group consisting of lactose, starch, glucose, methyl cellulose, magnesium stearate, mannitol and sorbitol.

In certain embodiments, the invention provides the above pharmaceutical composition, comprising a diluent selected from the group consisting of ethanol, glycerol and water.

In certain embodiments, the invention provides the above pharmaceutical composition, comprising an excipient selected from the group consisting of starch, gelatin, glucose, anhydrous lactose, free-flow lactose, beta-lactose, corn sweetener, acacia, tragacanth, sodium alginate, carboxymethyl cellulose, polyethylene glycol, sodium oleate, sodium stearate, magnesium stearate, sodium benzoate, sodium acetate and sodium chloride.

In certain embodiments, the invention provides the above pharmaceutical composition, further comprising at least one of a preservative, an antioxidant and a stabilizer.

In certain embodiments, the invention provides the above pharmaceutical composition, comprising a preservative selected from the group consisting of sodium benzoate, sorbic acid and esters of p-hydroxybenzoic acid.

In certain embodiments, the invention provides the above pharmaceutical composition, wherein said bacterial strain is lyophilised.

In certain embodiments, the invention provides the above pharmaceutical composition, wherein when the composition is stored in a sealed container at about 4°C or about 25°C and the container is placed in an atmosphere having 50% relative humidity, at least 80% of the bacterial strain as measured in colony forming units, remains after a period of at least about: 1 month, 3 months, 6 months, 1 year, 1.5 years, 2 years, 2.5 years or 3 years.

### Culturing methods

The bacterial strains for use in the present invention can be cultured using standard microbiology techniques as detailed in, for example, references [47,,49].

The solid or liquid medium used for culture may be YCFA agar or YCFA medium. YCFA medium may include (per 100ml, approximate values): Casitone (1.0 g), yeast extract (0.25 g), NaHCO₃ (0.4 g), cysteine (0.1 g), K₂HPO₄ (0.045 g), KH₂PO₄ (0.045 g), NaCl (0.09 g), (NH₄)₂SO₄ (0.09 g), MgSO₄ · 7H₂O (0.009 g), CaCl₂ (0.009 g), resazurin (0.1 mg), hemin (1 mg), biotin (1 µg), cobalamin (1 µg), *p*-aminobenzoic acid (3 µg), folic acid (5 µg), and pyridoxamine (15 µg).

### Bacterial strains for use in vaccine compositions

The inventors have identified that the bacterial strains of the invention are useful for treating or preventing diseases or conditions mediated by HDAC. This is likely to be a result of the effect that the bacterial strains of the invention have on the host immune system. Therefore, the compositions of the invention may also be useful for preventing diseases or conditions mediated by HDAC, when administered as vaccine compositions. In certain such embodiments, the bacterial strains of the invention may be killed, inactivated or attenuated. In certain such embodiments, the compositions may comprise a vaccine adjuvant. In certain embodiments, the compositions are for administration via injection, such as via subcutaneous injection.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references [50] and [51,57], *etc.*

The term "comprising" encompasses "including" as well as "consisting" e.g. a composition "comprising" X may consist exclusively of X or may include something additional e.g. X + Y.

The term "about" in relation to a numerical value *x* is optional and means, for example, x+10%.

The word "substantially" does not exclude "completely" e.g. a composition which is "substantially free" from Y may be completely free from Y. Where necessary, the word "substantially" may be omitted from the definition of the invention.

References to a percentage sequence identity between two nucleotide sequences means that, when aligned, that percentage of nucleotides are the same in comparing the two sequences. This alignment and the percent homology or sequence identity can be determined using software programs known in the art, for example those described in section 7.7.18 of ref. [58]. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. [59].

Unless specifically stated, a process or method comprising numerous steps may comprise additional steps at the beginning or end of the method, or may comprise additional intervening steps. Also, steps may be combined, omitted or performed in an alternative order, if appropriate.

Various embodiments of the invention are described herein. It will be appreciated that the features specified in each embodiment may be combined with other specified features, to provide further embodiments. In particular, embodiments highlighted herein as being suitable, typical or preferred may be combined with each other (except when they are mutually exclusive).

### MODES FOR CARRYING OUT THE INVENTION

### Example 1 - Efficacy of bacteria on histone deacetylase activity

### Introduction

The inventors sought to investigate the effectiveness of the *Bariatricus massiliensis* strain 43042 and its metabolites on HDAC inhibition.

### Material and Methods

### Bacterial culture and cell-free supernatant collection

Pure cultures of 43042 bacteria were grown anaerobically in YCFA broth until they reached their stationary growth phase. Cultures were centrifuged at 5,000 x g for 5 minutes and the cell-free supernatant (CFS) was filtered using a 0.2 µM filter (Millipore, UK). 1 mL aliquots of the CFS were stored at -80 °C until use. Sodium butyrate, hexanoic and valeric acid were obtained from Sigma Aldrich (UK) and suspensions were prepared in YCFA broth.

### SCFA and MCFA quantification of bacterial supernatants

Short chain fatty acids (SCFAs) and medium chain fatty acids (MCFAs) from bacterial supernatants were analysed and quantified by MS Omics APS as follows. Samples were acidified using hydrochloride acid, and deuterium labelled internal standards where added. All samples were analyzed in a randomized order. Analysis was performed using a high polarity column (Zebron™ ZB-FFAP, GC Cap. Column 30 m x 0.25 mm x 0.25 µm) installed in a GC (7890B, Agilent) coupled with a quadropole detector (59977B, Agilent). The system was controlled by ChemStation (Agilent). Raw data was converted to netCDF format using Chemstation (Agilent), before the data was imported and processed in Matlab R2014b (Mathworks, Inc.) using the PARADISe software described by Johnsen, 2017, J Chromatogr A, 1503, 57-64.

### Global HDAC activity analysis

Whole cells and cell free supernatants of stationary phase 43042 cultures were isolated by centrifugation and filtering in a 0.22 uM filter. HT-29 cells were used 3 days' post confluence and stepped down in 1 mL DTS 24 hours prior to commencement of the experiment. The HT-29 cells were challenged with 10 % cell free supernatant diluted in DTS and this was left to incubate for 48 hours. Nuclease proteins were then extracted using the Sigma Aldrich Nuclease extraction kit and samples were snap frozen prior to HDAC activity measurement. HDAC activity kit was assessed fluorometrically using the Sigma Aldrich (UK) kit.

### Specific HDAC activity analysis

Specific HDAC inhibition activity was analysed for HDAC1, 2, 3, 4, 5, 6, 9 using fluorogenic assay kits for each type of HDAC (BPS Bioscience, CA). Assays were conducted according to manufacturer's instructions and each sample were performed in replicates. Cell free supernatants were diluted 1 in 10 and exposed to specific HDAC proteins provided in the kit to maintain consistency between methods.

### Results

### 43042 whole cells and cell free supernatants reduce global HDAC activity

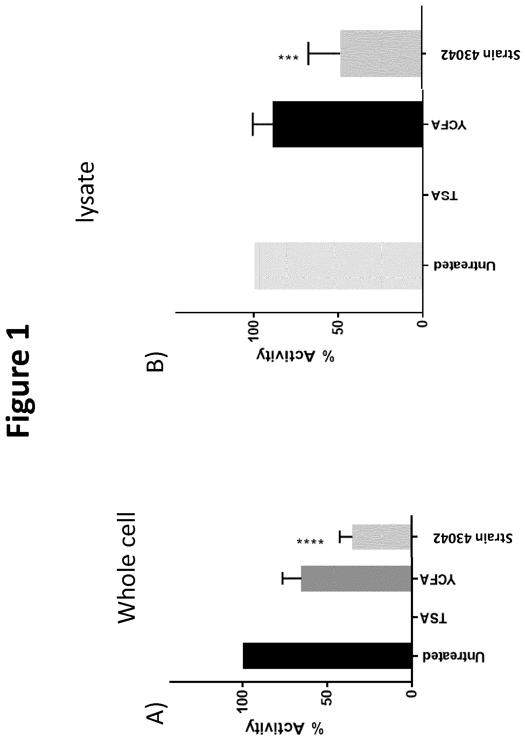

The results displayed in Figure 1 show that 43042 whole cells and CFS reduce global HDAC activity by a statistically significant amount.

### 43042 produces the HDAC inhibiting metabolite butyrate

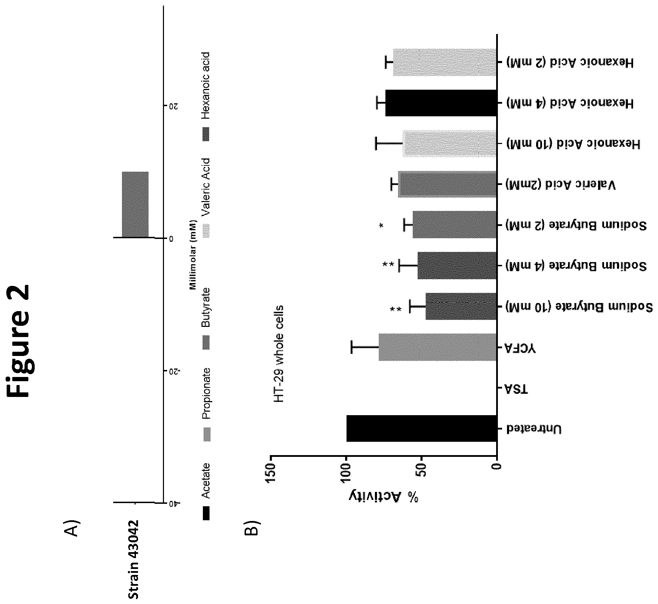

43042 supernatant showed strong HDAC inhibition and was found to produce significant amounts of butyrate (Figure 2A).

To investigate which metabolites were responsible for the strain-induced HDAC inhibition, different concentrations of hexanoic acid, valeric acid and sodium butyrate were measured for their HDAC inhibition on whole HT-29 cells and on HT-29 cell lysate. The results in Fig. 2B show significant (P<0.05) inhibition of HDAC activity by sodium butyrate on whole cells as well as on the cell lysate, while hexanoic acid did show significant inhibitory activity. Valeric acid inhibited total HDAC activity (* (p<0.05), ** (p<0.005), *** (P<0.001), ****(p<0.0001)).

### Potent total HDAC inhibitors investigated target class IHDACs.

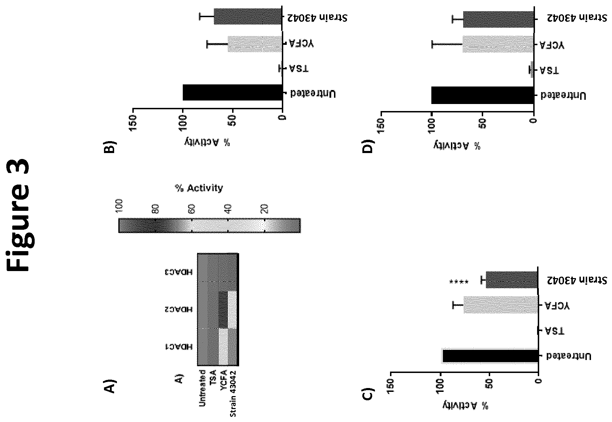

The specific HDAC inhibition profile of the test bacteria strain was investigated. Specific HDAC inhibition assays (BPS Bioscience, CA) were carried out for Class I HDACs. The ability of the bacterial strain to inhibit HDAC enzymes was analysed. The results (Figure 3) demonstrate that 43042 is a potent inhibitor of Class 1 HDAC enzymes (HDAC1, 2 and 3), in particular HDAC2.

### Discussion

Interestingly, the results for specific HDAC activity show that the tested strain is a potent inhibitor of Class I HDACs, and particularly HDAC2 (Figure 3). Class I HDACs (HDAC1, 2, 3 and 8) reside in the nucleus and are ubiquitously expressed in several human cell types. HDACs 1-3 share more than 50% homology, but have distinct structures and cellular functions [60]. They are primarily involved in cell survival, proliferation and differentiation, and thus there inhibition may be useful is wide array of diseases [61,62,63,64,65] . These data show that the compositions of the invention may be useful for treating diseases mediated by HDAC.

### Example 2 - Efficacy of bacteria on hyperactivity

### Introduction

The aim of this study was to evaluate the effects of anaerobic bacteria using MPTP lesioned mice. A number of 72 mice were lesioned with MPTP (6 groups with n=12 animals each) were treated daily for 18 days via oral gavage with 43042, vehicle or reference compound, starting 14 days before disease induction. A group of n=12 animals served as sham lesioned control and was treated with vehicle.

### Material and Methods

### Animals

All animals were housed in individual ventilated cages (IVCs) and were pathogen free but not kept under SPF. In addition the cages were opened under laminar air flow.

Mice were housed in individual ventilated cages from two different suppliers and in three different sizes: Polycarbonate cages from the company Ehret either Type-1-long: 426 cm² or Type-2-long: 530 cm² or Polysulfone cages from Tecniplast "greenline": 500 cm². Both air ventilation systems included HEPA filter systems (class HEPA 14), which meet the current standard.

For evaluating the health status of animals sentinel animals are used.

Adult (at least 8 weeks), female (to avoid aggression between males) CD1 mice were used are sentinels. Sentinels were housed in pairs per cage.

The Following parameters were maintained for the animal housing:
- 12 hours light-dark cycle (summer time: lights on from 6 a.m. to 6 p.m., lights off from 6 p.m.to 6 a.m.; winter time: lights on from 5 a.m. to 5 p.m., lights off from 5 p.m. to 5 a.m.)
- Light intensity: critical value: Mouse [<400 Lux]
- Volume: critical value: [<60 dB]
- Continuous background noise at low volume (e.g. Radio on during the light phase)
- Room temperature: 20-24°C
- Humidity: 30-70%
- The humidity and the room temperature were monitored centrally by an alarm system.
- As bedding material wooden granulate material was used which owns a high suction property and showed a low fine material portion (e.g. Lignocel)
- As enrichment nesting material "Nestlets" were used
- Standard maintenance food for mice and rats or breeding food for breeding animals were used (e.g. Altromin)
- Normal tap water was used as water supply

The least number of animals was used in compliance with current regulations and scientific integrity. The welfare of the animals was taken into account in terms of number and extent of procedures to be performed.

Safety precautions operating within the test facility were applied to the study.

### Details of animals used

- Mouse line:: C57BL/6J (JAX™ Mice Strain) JAX™ Mice Stock Number 000664
- Provider:: Charles River Laboratories
- Age at start:: ^{∼}10 weeks

### Accommodation of Animals

As soon as the animals arrived at QPS Austria they were brought to the assigned animal room, were unpacked and checked for their health status. Information on the transportation system and the data provided by Charles River Laboratories (CRL) beforehand were crosschecked. An animal list was generated including IRN, age at delivery and sex. Animals were habituated for at least one week prior to study start.

### Housing

Animals were housed in groups of two. The room temperature was maintained at approximately 20-24 °C and the relative humidity between 30 to 70 %. Animals were housed under a constant light-cycle (12 hours light/dark; (summer time: lights on from 6 a.m. to 6 p.m., lights off from 6 p.m. to 6 a.m.; winter time: lights on from 5 a.m. to 5 p.m., lights off from 5 p.m. to 5 a.m.). Dried, pelleted standard rodent chow (Altromin) as well as normal tap water was available to the animals ad libitum.

### Identification

Animals were numbered consecutively by classical earmarking, if not already present at arrival.

Each cage was identified by a colored card indicating the study number, sex, the individual registration numbers (IRN) of the animals, age at delivery and the treatment group allocation.

### Group Allocation

Only animals in apparently good health condition were included to the study. Animals were allocated to the treatment groups regarding body weight, results of baseline IRWIN test and respectively to the composition in the cages.

Details of the methods that were not specified in the subsequent sections of this study plan are contained in the appropriate standard operating procedures.

### Specific handling of Animals and randomization

Gloves had to be changed between each treatment group and sprayed with 70% ethanol solution between each cage of the same group to minimize the risk of contamination whenever animals were handled (e.g.: treatment, behavioural testing, cleaning and tissue sampling).

The treatment had to be at random and alternated daily so as to prevent the same groups being treated at the same time each day. Also behavioural testing had to be carried out at pseudo-random order, alternating each day, so as to prevent the same animals being handled at the same-time points. Animals were also randomized per cage at the tissue sampling.

### Treatment group allocation, treatment

72 male mice were allocated to 5 different treatment groups. Groups were treated daily for 18 days via oral gavage with 43042 (Group C), or vehicle (PBS) (Groups A, B and E) or vehicle (anaerobic PBS - Group D). Oral treatment started 14 days before MPTP lesion. Group E animals received a daily vehicle (PBS) p.o. treatment and were injected i.p. (intraperitoneal) with the reference drug 30 min before and 90 min after first MPTP on day 0. The application volume for p.o. and vehicle treatment was 200 µl per mouse. 43042 were from glycerol stocks (gly). For oral treatment, gavages for applications were stored in vial containing 70% Ethanol and were flushed before and after each use with distilled water. Every treatment group had its own gavage and ethanol vial and distilled water vial. Directly before treatment each syringe was flushed with N2.

On day 0 MPTP (20 mg/kg b.w. 4 times, 2h inter-treatment interval) was injected i.p. in animals of groups B, C, D and E. One group of animals (A) was sham lesioned by i.p. administration of the MPTP vehicle (0.9% saline). The application volume was 10 µl per g body weight. Weighing of the animals was performed before the MPTP treatment to dose the animals according to their actual body weight. Afterwards animals received the daily p.o. treatment.

### Open Field Test

The open field test was performed on day 2.

Spontaneous activity and anxiety were assessed in the Open field by evaluating the following parameters: hyperactivity[s], activity[s]. For that purpose, a Plexiglas Open Field (48x48 cm; TSE-System®) was used. The infrared photo beams were placed in a 1.4 cm distance around the box. To detect rearing (standing on the hind paws) another row of photo beams were mounted 4 cm above the first one. Each test session lasted for 5 minutes to check the mice's behaviour in the new surroundings, as the first minutes of the Open Field test were the most suitable to display the exploration behaviour of the animals. Thereafter the number of faecal boluses was counted, as a measure of emotionality. The Open Field was cleaned with 70% ethanol after each mouse to get rid of odor traces. Testing was performed under standard room lighting conditions during the light phase of the circadian cycle.

### Statistics

Basic statistical analysis was performed therefore raw data was tested for normality by using the Kolmogorow-Smirnow-Test. If normal distribution was confirmed differences between two groups were tested with the T-test or between more than two groups with the One-way analysis of variance. If data was not normally distributed differences between two groups were tested with the Mann-Whitney test or between more than two groups with the Kruskal-Wallis-Test. The Bonferroni posttest was used as post hoc test for One-way analysis of variance and the Dunn's test was used as post hoc test for the Kruskal-Wallis-Test. Group differences over time were tested by using the Two-way analysis of variance followed by Bonferroni posttest. If appropriate, data were presented as mean ± or + standard error of mean (SEM).

Statistical analyses were performed by comparing all groups against A (positive control), B (negative control), E (reference item) and C against D (vehicle for group C).

### Results

### General Observations and Health Status

Administration of bacteria strains were well tolerated by the animals. On the MPTP lesion day and if necessary on the day afterwards a red light was used to warm the animals. If animals were in sub-optimal conditions (felt cold, dehydrated, abnormal behavior), they were supplied with wet food and subcutaneous saline treatment if necessary. During the study the following 6 animals died due to unknown reasons.

### Open Field Testing

On day 2 of the study course (2 days after MPTP lesion) animals were tested in the Open Field test. The testing showed that hyperactivity and activity are significantly reduced in animals of group C (43042) and group D (Vehicle (anaerobic PBS)) compared to sham lesion and vehicle treated animals of group A (Figure 84). Also compared to reference item group E (7-nitroindazole) animals of group G showed a significantly reduced hyperactivity in the Open Field testing.

### Discussion

The marked reduction in hyperactivity in mice administered 43042 7compared with vehicle-only controls highlights that 43042 may be useful in the treatment or prevention of conditions or diseases associated with hyperactivity, such as behavioural disorders, including ADHD, and Parkinson's disease. Hyperactivity has been associated with increased anxiety in subjects with Parkinson's disease.

### Example 3 - Efficacy of Bariatricus massiliensis strain 43171 in enhancing survival from GVHD

### Objective

The inventors sought to determine the effect of *Bariatricus massiliensis* strain 43171 on graft versus host disease (GVHD) induced in Balb/C mice.

### Material and methods

### - Animals

Male Balb/C mice (BALB/cAnNCrl; 6-8 weeks old; n=125) with an average starting body weight (± SEM) of 20.67±0.11 g were obtained from Charles River Laboratories (Wilmington, MA). An additional n=75 male C57B1/6 (C57B1/6NCrl; 6-8 weeks old) were obtained from the same vendor. Animals were acclimatized prior to study commencement. During this period, the animals were observed daily in order to reject any that presented in poor condition.

### - Housing

The study was performed in animal rooms provided with HEPA filtered air at a temperature of 70 ± 5oF and 50% ± 20% relative humidity. Animals were housed in groups of 4-6 per cage. Specifically, groups with 8 animals/group were housed at n=4/cage; groups with 10 animals/group were housed at n=5/cage; and groups with 12 animals per group were housed at n=6/cage. Animals were housed in HEPA-filtered, individually ventilated cages. Cages were geographically separated on the racks to minimize cross-contamination between groups. Animal rooms were set to maintain a minimum of 12 to 15 air changes per hour. The room was on an automatic timer for a light/dark cycle of 12 hours on and 12 hours off with no twilight. Alpha-dri® bedding (irradiated) was used. In addition to bedding, each cage was provided with enviro-dri and a shepherd shack (enrichment). Floors were swept daily and mopped a minimum of twice weekly with a commercial detergent. Walls and cage racks were sponged a minimum of once per month with a dilute bleach solution. A cage card or label with the appropriate information necessary to identify the study, dose, animal number, and treatment group was used to mark all cages. The temperature and relative humidity was recorded during the study, and the records retained. All technicians donned PPE (lab coat, gloves, safety goggles) prior to entering the lab/vivarium and working with animals.

### - Diet

Animals were fed with LabDiet 5053 sterile (irradiated) rodent chow and water (reverse osmosis) was provided ad libitum. No food-based enrichment was provided.

### - Animal Randomization and Allocations

Animals were randomized into 5 groups at the start of the study. Each group comprised between 8 and 12 mice. Each group was further sub-divided into cohorts A and B (n=4-6 mice per group per cohort); cohorts had staggered disease timelines.

### - Analysis of growth kinetics of NCIMB 43171

Prior to administration of NCIMB 43171 growth curve/maximum OD were determined and virtual colony count (VCC) at maximum OD600 and after wash were determined. Growth curve/maximum OD analysis took place as follows. At 6 AM, one tube each of frozen bacterial stocks were brought into the Coy chamber. Tubes were allowed to thaw, were mixed carefully by pipetting up and down, and two tubes (duplicates) containing 9.5 mL of pre-reduced, pre-warmed (37°C) YCFA broth were inoculated with 500 µL bacterial stock. These were the pre-cultures. Pre-cultures were incubated at 37°C in the Coy chamber for 24 hours. At 6 AM the next day (ie after 24 hours of incubation), a small aliquot of each culture was removed from the Coy chamber, and the OD600 was determined by nanodrop. Tubes were mixed by inversion prior to removing the aliquot for OD600 measurement. The remainder of the 24 hour cultures (using the tube with the higher OD600 as determined above) were cultured in duplicate as follows: 250 µL of NCIMB 43171 24 hour culture was used to inoculate two tubes containing 24.75 mL pre-warmed YCFA broth. These cultures were incubated at 37°C in the Coy chamber for 24 hours, and a small aliquot was removed from the Coy chamber for measurement of OD600 every two hours for 16 hours (i.e. at 8 AM, 10 AM, 12 PM, 2 PM, 4 PM, 6 PM, 8 PM, and 10 PM), and at 24 hours (6 AM the next day). Tubes were mixed by inversion prior to removing the aliquot for OD600 measurement.

VCC at maximum OD analysis occurred as follows: one tube of NCIMB 43171 stock was brought into the Coy chamber. Tubes were allowed to thaw, were mixed carefully by pipetting up and down, and two tubes (duplicates) containing 9.5 mL of pre-reduced, pre-warmed YCFA broth was inoculated with 500 µL bacterial stock. These were the pre-cultures. Pre-cultures were incubated at 37°C in the Coy chamber for 24 hours. The next day (after 24 hours of incubation), a small aliquot the pre-culture was removed from the Coy chamber, and the OD600 was determined by nanodrop. Tubes were mixed by inversion prior to removing the aliquot for OD600 measurement. The remainder of the 24 hour, cultures (using the tubes with the higher OD) were cultured in duplicate as follows: 250 µL was used to inoculate two tubes containing 24.75 mL pre-warmed YCFA broth. These were the main cultures. A small aliquot of main culture was removed from the Coy chamber at the indicated time, and the OD600 was determined by nanodrop. Tubes were mixed by inversion prior to removing the aliquot for OD600 measurement. The VCC of the remaining stock was determined as follows: an individual dilution series (undiluted, 1:103, 1:104, 1:105, and 1:106) was prepared in PBS. The remainder of each culture was then transferred to a 50 mL conical tube, and the tubes were removed from the Coy chamber and centrifuged (3500xg; 15 minutes). Once centrifugation was complete, the tubes were returned to the Coy chamber, and the supernatants were removed (with care taken to avoid disturbing the pellets), and measured. The pellets were resuspended in volumes of PBS equivalent to that of the removed supernatants, and were mixed carefully with a pipette (no vortexing). An individual dilution series (undiluted, 1:103, 1:104, 1:105, and 1:106) was prepared in PBS. Both dilution series (broth and PBS suspended) were spot plated (20 µL) in triplicate in one quadrant of a pre-reduced YCFA agar plate. Plates were incubated at 37°C in the Coy chamber for 48 hours, and the VCC of whichever dilution yielded spots with 5-20 CFU/spot was counted. The three spot VCC/spot values were averaged to determine the VCC/mL of overnight cultures in broth and centrifuged/resuspended in PBS.

### - NCIMB 43171 Dosage preparation

Two days prior to each dosing timepoint, one tube (1 mL/tube) per strain of frozen stocks of NCIMB 43171 were entered into the Coy chamber. The tubes were allowed to thaw, and two 15 mL tubes, each containing 9.5 mL of pre-reduced and pre-warmed YCFA broth were inoculated with 0.5 mL of each bacteria stock. These were the pre-cultures (tubes 1 and 2). Pre-cultures were incubated at 37°C in the Coy chamber for 24 hours.

After incubation for 24 hours, one day before each dosing timepoint, cultures were mixed by inversion, and a small aliquot (20 µL) of the cultures were removed from the Coy chamber for OD600 determination by nanodrop. Whichever tube (1 or 2) per strain had the higher OD600 value was used for the main culture, in duplicate, as follows: 250 µL of the pre-culture with the higher OD600 was used to inoculate 24.75 mL of pre-warmed YCFA broth in a 50 mL conical (in duplicate; tubes A and B). These main cultures were incubated in the Coy chamber (at 37°) for 14 hours.

On each dosing day (after the above main culture incubations), cultures were mixed by inversion, and a small aliquot (20 µL) of the cultures were removed from the Coy chamber for OD600 determination by nanodrop. Tubes were then removed from the Coy chamber and centrifuged (3500xg; 15 minutes). Once centrifugation was complete, the tubes were returned to the Coy chamber, and the supernatant was removed (with care taken to avoid disturbing the pellet) by pipette. Pellets were resuspended in 2.04 ml PBS. Pellets were mixed carefully by pipetting (no vortexing). An aliquot (0.5 mL) of each strain was pipetted into Eppendorf tubes and retained in the Coy chamber. The remainder of each strain was removed from the Coy chamber and used for dosing (0.1 mL per animal), with care taken to dose the animals as quickly as possible following the resuspension.

The 0.5 mL aliquot of each strain retained in the Coy chamber was used for preparation of an individual dilution series in pre-reduced MRD; the 1:107, 1:108, 1:109, and 1:1010 dilutions were spot plated (20 µL) in triplicate in one quadrant of a pre-reduced YCFA agar plate. Plates were incubated at 37°C in the Coy chamber for 48 hours, and the VCC of whichever dilution yielded spots with 5-20 CFU/spot were counted. The three spot VCC/spot values were averaged to determine the VCC/mL of the experimental dosing material.

### - Pre-treatment phase

All animals were weighed and randomized by weight into treatment groups prior to study start. Prior to GVHD induction on Days -1 and 0, all animals were pre-treated (PO) with PBS (Groups 1-4), NCIMB 43171 (Group 5), or Butyrate salt control (Group 6) daily starting on Day - 14. Butyrate was used as a positive control as butyrate deficit has been identified in the gut of GVHD patients. Treatments were administered to groups at random, and group treatments were alternated daily to prevent the same groups from being treated at the same time each day. Once test article dosing had begun, care was taken to minimize group cross-contamination: gloves were changed by the technician between treatment groups, and were sprayed with 70% isopropyl alcohol between each cage of the same group.

### - GVHD modelling

GVHD was induced in mice using a single acute dose of 8 Gy of total body irradiation (TBI) on Day -1. On Day 0, these recipient mice were given an intravenous injection of a combination of T cell depleted bone marrow cells and splenic cells from donor C57B1/6 mice in PBS. Bone marrow cells were isolated using standard flushing practices, and were T cell depleted using the cell surface T cell antigen CD3, with a CD3-biotin kit (Miltenyi Biotec catalog 130-094-973). Splenocytes were isolated using Miltenyi GentleMACS Dissociators. Animals in Group 1 served as naive controls and received neither TBI nor cell transfer. Animals in Group 2 served as irradiation controls and received the 8 Gy of TBI on Day -1, but did not receive a cell transfer on Day 0. Animals in Group 3 served as syngeneic adoptive transfer controls; these animals received 8 Gy of TBI on Day -1, and an intravenous injection of a combination of T cell depleted bone marrow cells and splenic cells from donor Balb/C mice in sterile PBS.

Daily test article dosing continued for the duration of the study (Days -14 to 30). Animal survival was recorded daily, as an indication of GVHD severity. Animals were also weighed, observed, and given a clinical GVHD score daily for the duration of the study following GVHD induction. The GVHD score was assessed by a standard scoring system based on five criteria (Table 21): percentage of weight change, posture (hunching), activity, fur texture, and skin integrity (maximum score = 10).

**Table 1: Assessment of GVHD in Transplanted Mice (Daily Scoring)**

| **Criteria** | **Grade 0** | **Grade 1** | **Grade 2** |
|---|---|---|---|
| **Weight loss** | <10% | >10% <25% | >25% |
| **Posture** | Normal | Hunching noted only at rest | Severe gait, impaired movement |
| **Activity** | Normal | Mild to moderately decreased | Stationary until stimulated |
| **Fur texture** | Normal | Mild to moderate ruffling | Severe ruffling and/or poor grooming |
| **Skin integrity** | Normal | Scaling of paws and/or tail | Obvious areas of denuded skin |

Animals that lost 20% of their body weight were administered sub-cutaneous fluids (SID; saline) and provided with softened food. If any individual study animal required softened food, all study animals were provided with softened food until that individual animal's weight loss had been rescued. Treatment continued until either scheduled euthanasia or body weight loss greater than 30%. Animals that were unable to right themselves, were cold to the touch, or were moribund were euthanized.

On Day 29, all surviving animals underwent endoscopy to monitor colonic inflammation. Images were taken and colitis severity and stool consistency were scored using the scoring scale shown in Table 2.

**Table 2: Endoscopy Colitis Scoring Scale**

| **Score** | **Description:** |
|---|---|
| 0 | Normal |
| 1 | Loss of vascularity |
| 2 | Loss of vascularity and friability |
| 3 | Friability and erosions |
| 4 | Ulcerations and bleeding |

On Day 30, blood was collected by RO bleed; blood (approximately 150 - 300 µL) was collected into two tubes - approximately two third of the blood was collected into K2EDTA tubes, and the remaining one third was collected into lithium-heparin tubes. Both samples were centrifuged and processed for plasma, and plasma tubes were clearly labeled to indicate the anti-coagulant used. For the K2EDTA sample, plasma was aliquoted as follows: 25 µL (for use in downstream citrulline assay), and remainder. All plasma was frozen at -80°C. All K2EDTA samples were assessed for citrulline by ELISA upon study completion
Euthanasia was performed by CO2 inhalation and cervical dislocation, without organ collections for animals euthanized off-schedule during the TBI phase of the study. Euthanasia was performed by cervical dislocation only, with organ collections, for animals euthanized off-schedule during the GVHD phase of the study. Terminal collections occurred on the benchtop. The benchtop was cleaned with 70% isopropyl alcohol and a commercial disinfectant before beginning. Instruments were cleaned with 70% isopropyl alcohol between animals, and with a commercial disinfectant between groups.

### - Statistical Analyses

Parametric data was analyzed by one-way ANOVA with Tukey's multiple comparisons test to compare all groups to one another. Non-parametric data was analyzed by Kruskal-Wallis test with Dunn's multiple comparisons test to compare all groups to one another. All statistical analyses were performed using GraphPad Prism 7 (La Jolla, CA).

### Results and discussion

### - Body weight

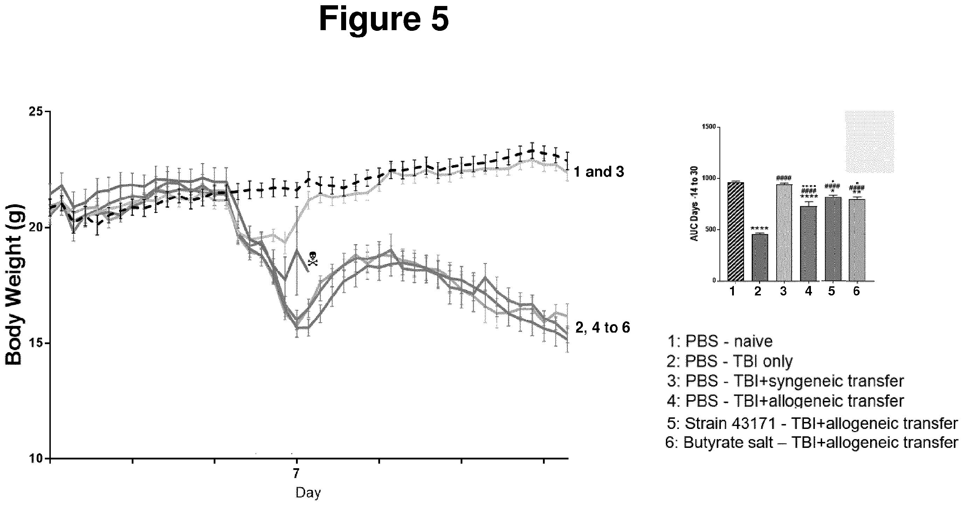

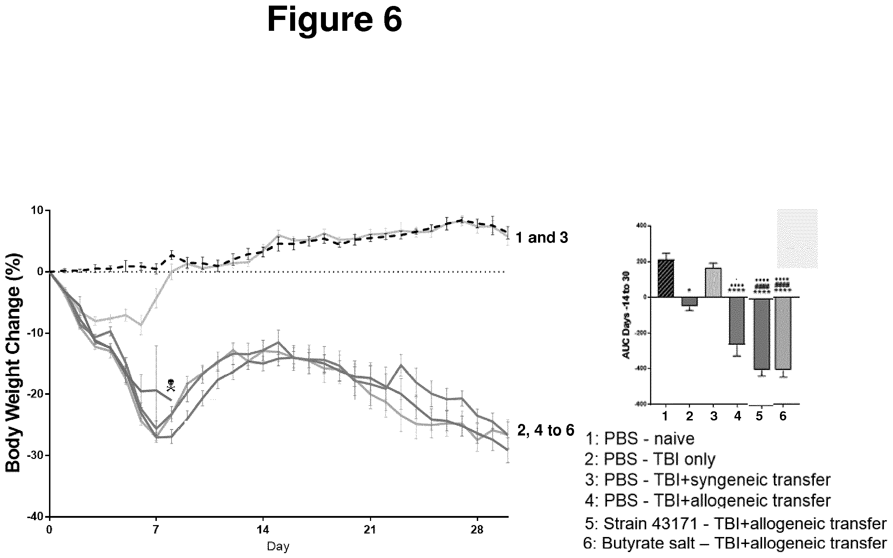

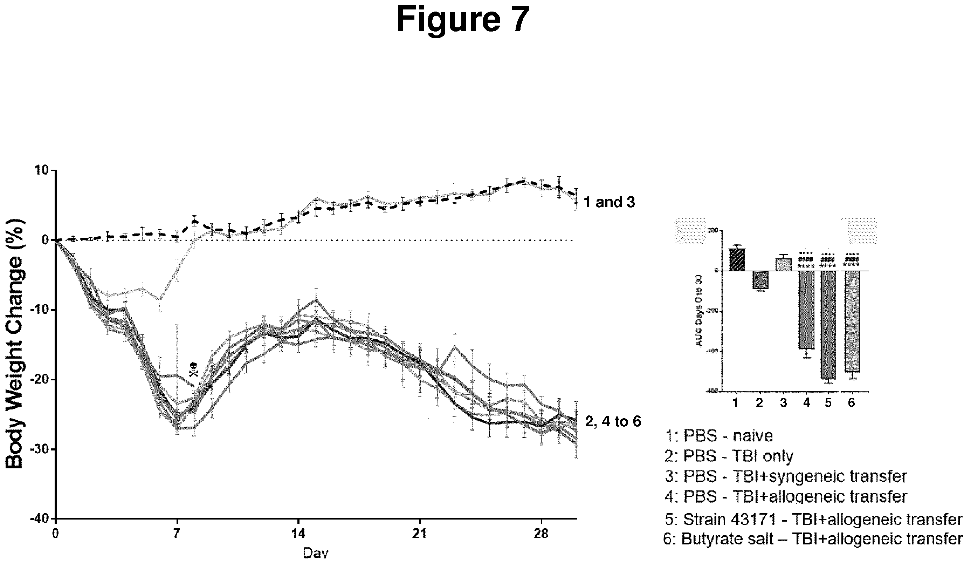

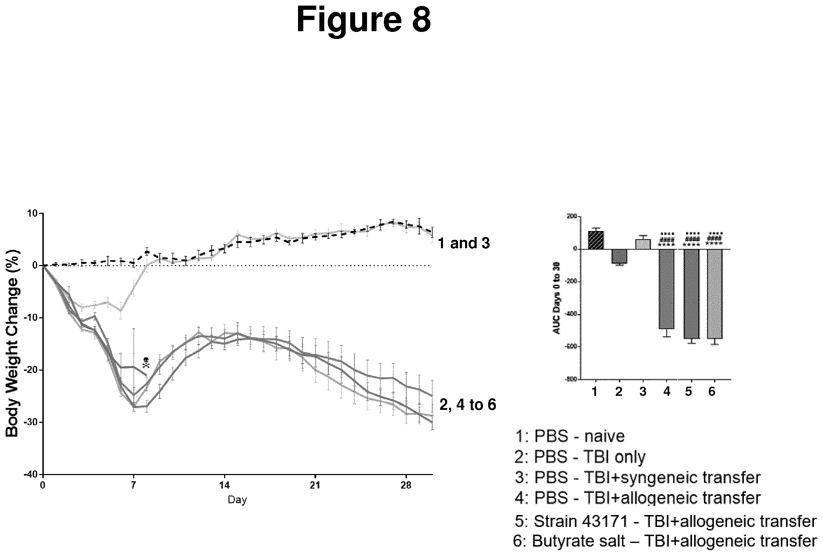

Animals were weighed on a daily basis for the duration of the study, and the mean body weight for all groups over the course of the study is shown in Figure 5. Body weight change relative to either Day - 14 (Figure 6) or Day 0 (Figure 7) was calculated. In order to determine statistically significant differences between groups in either mean body weight or mean body weight change relative to either Day -14 or Day 0, the area under the curve (AUC) was calculated using the trapezoidal transformation rule and is shown in the insets of Figures 5, 6 and 7. To account for group attrition, the body weight change relative to Day 0 shown with the body weight with which an animal died carried forward for the duration of the study for animals found dead or euthanized (for all groups with the exception of Group 2) is shown in Figure 8, with the AUC inset.

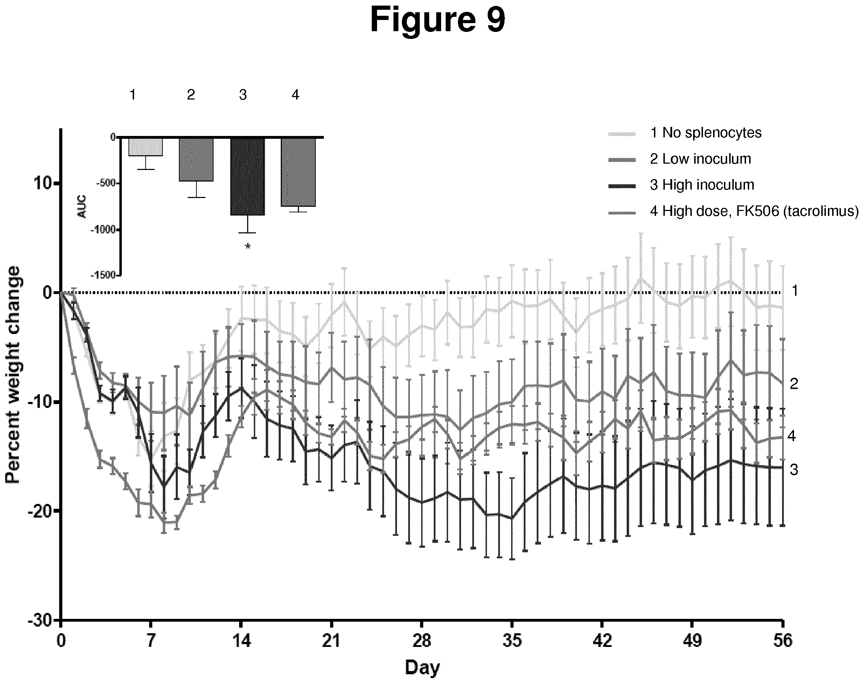

No major differences in mean body weight (Figure 5) were observed for any groups during the pre-treatment period. All groups exposed to TBI demonstrated body weight loss from Days 0 to 3. Mean body weight for animals in Group 3 (PBS - TBI + syngeneic transfer) recovered from this point forward and ultimately returned to baseline. Mean body weight for animals in Group 2 (PBS - TBI only) failed to recover prior to the death of all animals within the group. For all other study groups, mean body weight continued to decrease through Day 7, increased through Day 14, and subsequently decreased through the duration of the study. The mean body weight in Groups 2, 5 and 6 was significantly decreased over the course of the study as compared to Group 1 (PBS - naive). In contrast, the mean body weight in Groups 3-6 were significantly increased over the course of the study as compared to Group 2. Finally, the mean body weight in Groups 5 and 6 was significantly decreased over the course of the study as compared to Group 3. No significant differences in mean body weight over the course of the study were observed when comparing treatment groups (Groups 5 and 6) with Group 4 (PBS - TBI+allogeneic transfer). This same trend was observed when mice were administered the immunosuppressant tacrolimus, a known therapy of GVHD (Figure 9).

The mean body weight change relative to Day -14 (Figure 6) increased for all groups during the pre-treatment period, and the kinetics of body weight change relative to Day -14 from Day 0 onward to similar to that observed for mean body weight. Animals in Groups 2 and 4-6 demonstrated significantly increased body weight loss over the course of the study as compared to both Group 1 and Group 3.

The mean body weight change relative to Day 0 (Figures 7 and 8) decreased for all groups exposed to TBI from Days 0 to 3, at which point body weight gain began for animals in Group 3; body weight loss continued for all other groups through Day 4. Body weight change relative to Day 0 increased from Day 7 to Day 14, and mean body weight loss was observed for Groups 4-6 from Day 14 through the end of the study on Day 30. While the overall pattern in body weight change relative to Day 0 was similar regardless of whether body weight was carried forward for deceased animals, statistically significant differences between groups differed. Significantly increased body weight loss as compared to Groups 1, 2, and 3 were observed for Groups 4-6 both with and without body weight for deceased animals carried forward, again, which is similar to the trend observed in mice administered the known GVHD therapy tacrolimus (Figure 9).

### - Survival

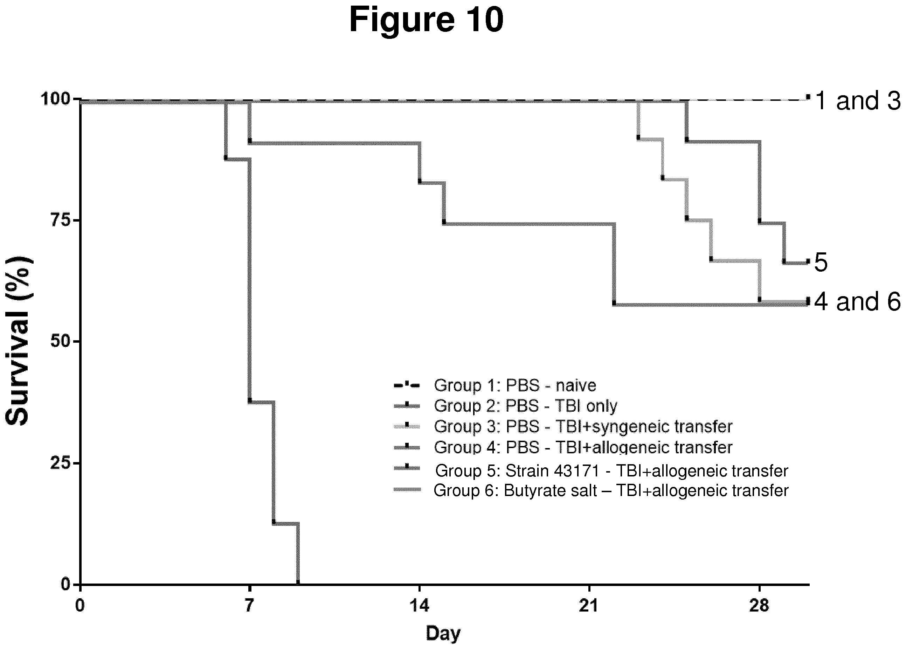

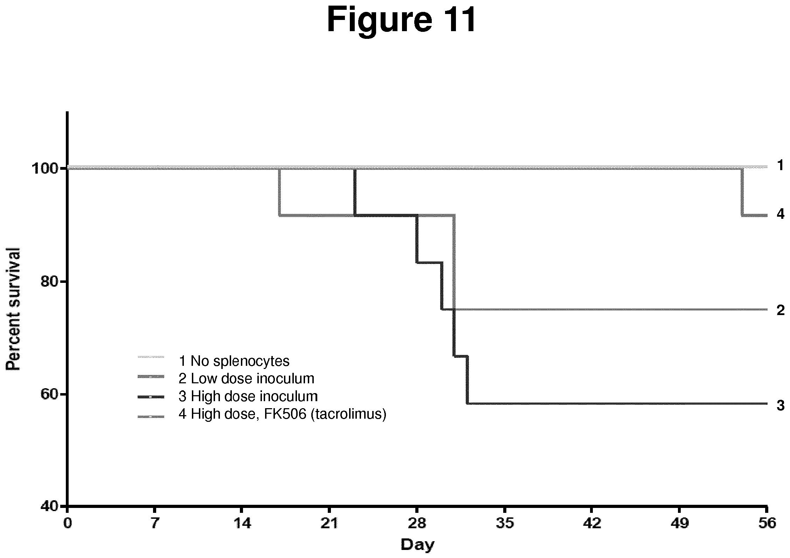

Animals were assessed daily for survival or moribundity, and a Kaplan-Meier curve showing survival over the duration of the study is shown in Figure 10. Survival was 100% in Groups 1 and 3, 0% in Group 2, and 75% in Group 5. The survival observed in Group 5 was notably improved as compared to both Groups 4 and 6. This is significant, as butyrate has been proposed as a treatment for GVHD [66]. Survival rates were comparable to mice controls administered the known GVHD treatment tacrolimus (FK506 - Figure 11)

### - GVHD Scores

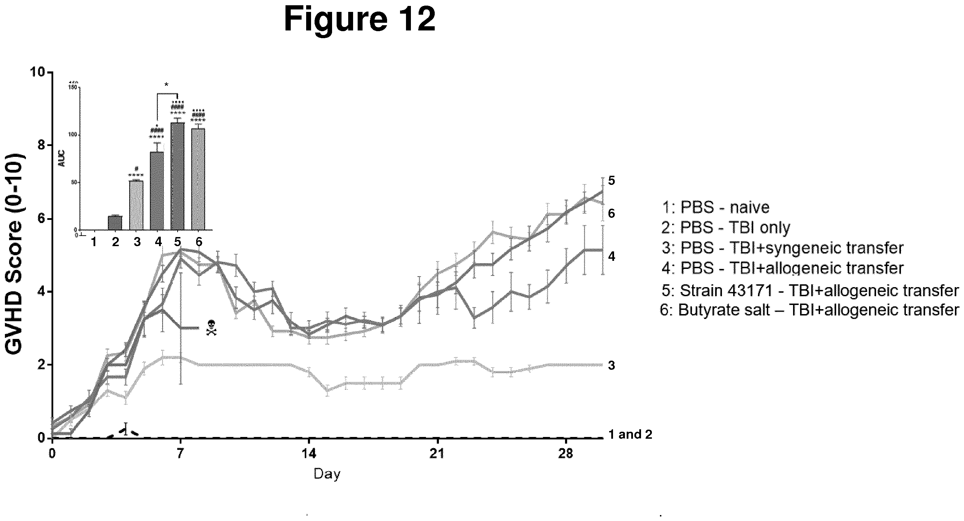

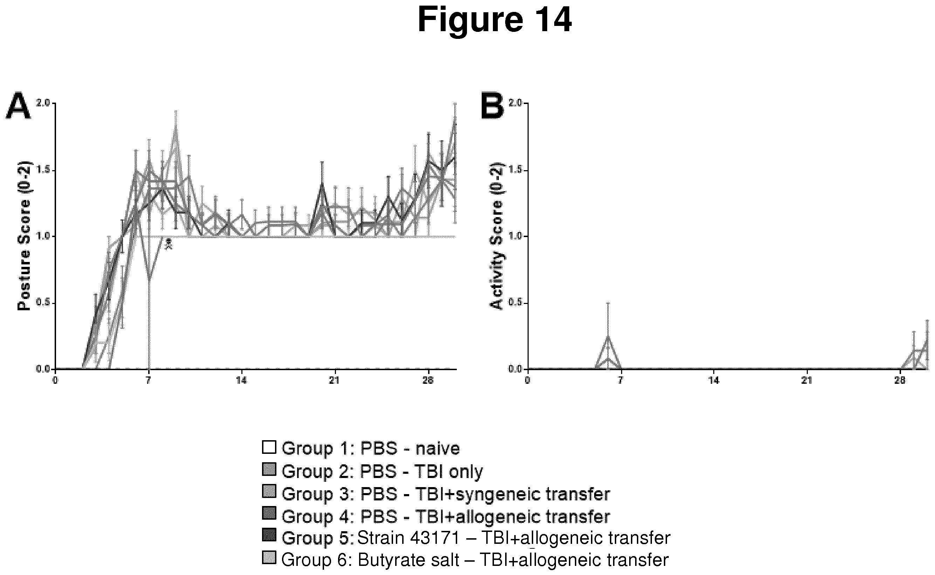

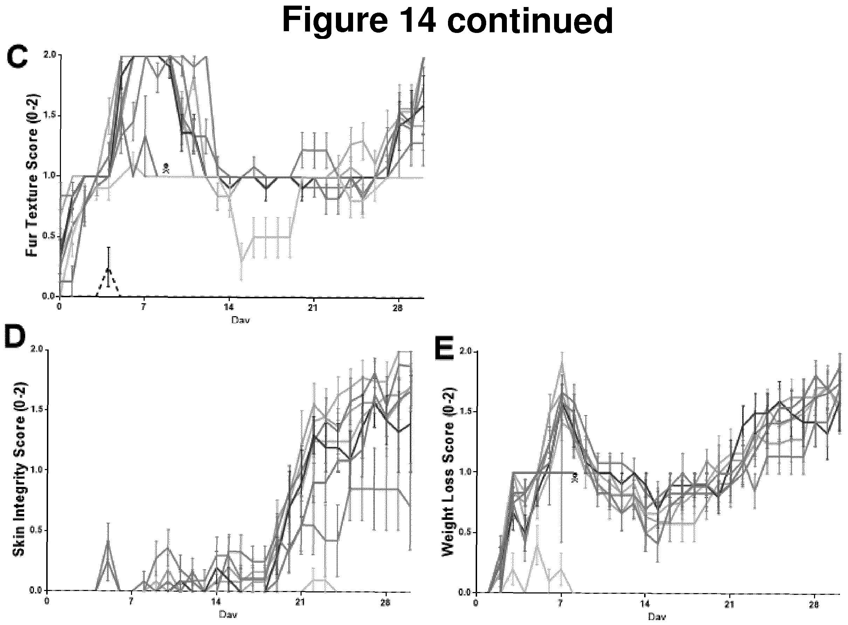

GVHD scores were assessed (as per the multi-parameter scoring shown in Table 2) in all animals from Day 0 through the end of the study on Day 30. Mean GVHD scores for all groups are shown in Figure 12, and this same data presented with the GVHD score with which an animal died carried forward is shown in Figure 13. The AUC was calculated using the trapezoidal transformation rule in order to determine statistically significant differences in overall GVHD scores between groups, and this is shown in the insets of Figures 12 and 13. The clinical GVHD score assigned to each animal is a composite consisting of posture (Fig. 14A), activity (Fig. 14B), fur texture (Fig, 14C), skin integrity (Fig. 14D) and weight loss (Fig. 14E).

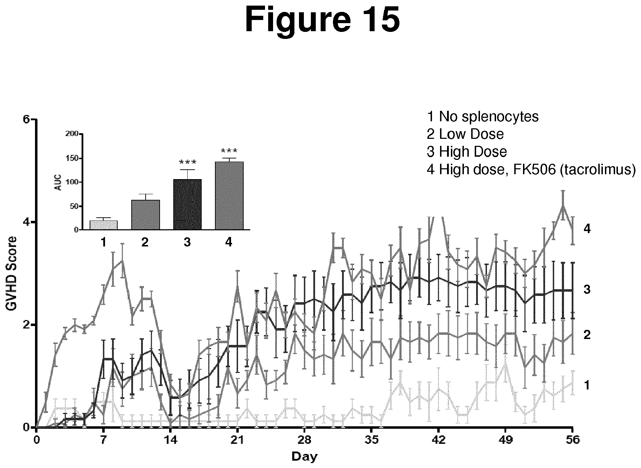

Intravenous injection of allogeneic splenocytes and bone marrow cells induced GVHD in all groups that began around Day 19 and progressively increased in severity until the conclusion of the study. There was an initial GVHD score increased between Days 0-7, presumably due to TBI and engraftment; survival of animals past this point verifies successful engraftment of the transplanted cells. While the GVHD score kinetics were similar regardless of whether GVHD score was carried forward for deceased animals, statistically significant differences between groups differed. Animals in Groups 3-6 demonstrated significantly increased mean GVHD scores as compared to both Groups 1 and 2 both with and without GVHD scores for deceased animals carried forward; likewise, animals in Groups 4-6 demonstrated significantly increased mean GVHD scores as compared to Group 3 in both instances. This trend was also observed in mice models of GVHD administered the immunosuppressant tacrolimus, which is a known therapy of GVHD (Figure 15).

### - Endoscopy

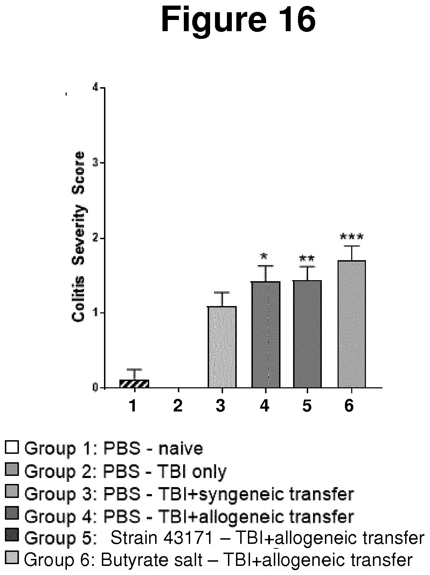

Animals underwent endoscopy on Day 29, in order to assess colonic inflammation. Colitis was scored visually on a five-point scale that ranges from 0 for normal, to 4 for severe ulceration (Table 3). The mean colitis severity is shown in Figure 16.

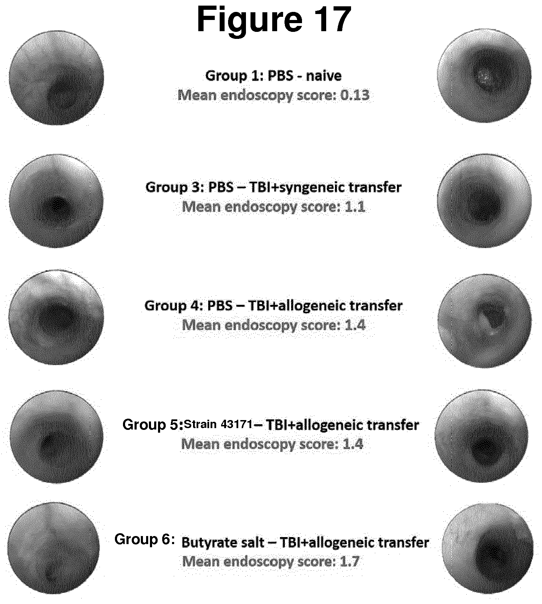

Mean colitis severity scores were increased for Strain 43171-treated and butyrate-treated animals as compared to naive mice (Group 1). However, colitis was significantly less in Strain 43171-treated mice as compared with butyrate-treated mice. This is significant, as correction of butyrate deficit has been suggested as a treatment for colitis [67]. Representative endoscopy images are shown in Figure 17.

### - Plasma Citrulline

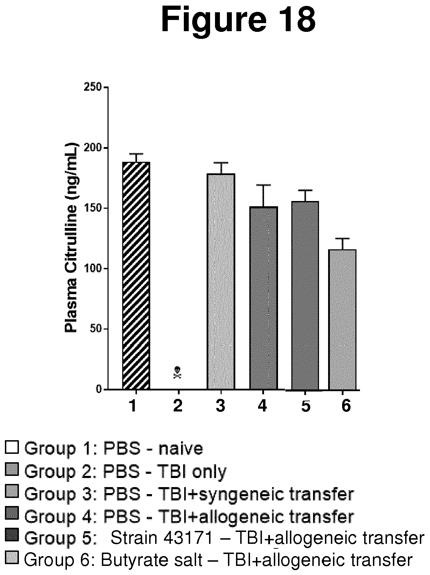

Blood was collected prior to euthanasia from all surviving animals and was processed for plasma. Plasma citrulline was assessed as a marker of intestinal permeability in duplicate by ELISA. A reduction in plasma citrulline levels corresponds to a loss in epithelial cell mass indicating an increase in gut barrier permeability. The maintenance of gut barrier function (i.e. a maintenance of gut impermeability) is important for the treatment of GVHD [68]. The results are shown in Figure 18. Mice administered Strain 43171 maintained greater levels of plasma citrulline in comparison to mice administered butyrate salts (Group 6), which is significant considering the role of butyrate in maintaining correct barrier function.

### Example 3 - Stability testing

A composition described herein containing at least one bacterial strain described herein is stored in a sealed container at 25°C or 4°C and the container is placed in an atmosphere having 30%, 40%, 50%, 60%, 70%, 75%, 80%, 90% or 95% relative humidity. After 1 month, 2 months, 3 months, 6 months, 1 year, 1.5 years, 2 years, 2.5 years or 3 years, at least 50%, 60%, 70%, 80% or 90% of the bacterial strain shall remain as measured in colony forming units determined by standard protocols.

### Example 4 -Efficacy of bacterial inocula to reduce IL-6 secretion

### Introduction

Activation of proinflammatory cytokines has been associated with damage in inflammatory disease. Lipopolysaccharide (LPS) is a known stimulator of the proinflammatory cytokine IL-6. U373 cells were treated with compositions comprising the 43042 strain of the invention in combination with LPS to observe its ability to modulate the levels of IL-6.

### Methods

U373 is a human glioblastoma astrocytoma cell line. Cells (used between passage 20th and passage 37th) were maintained in 25ml MEME 4.5 g/L D-glucose supplemented with 10% heat-inactivated FBS, 4mM L-Glutamine, 100 U/ml penicillin, 100 µg/ml streptomycin and 5 µg/ml plasmocin, 1% Non-Essential Amino Acids, 1% Sodium Pyruvate (referred to throughout as full growth media).

Cells were plated in 24-well plates at a density of 100,000 cells/well in 1ml of full growth media and left to rest at 37°C/5% CO2 for 72h. On the day of the treatment, the media was removed from each well, cells were rinsed with 0.5 ml wash media (serum free MEME), 0.9ml stimulation media (MEME media containing 2% FBS) containing 1 µg/ml LPS was added to the appropriate wells and incubated at 37°C and 5% CO2. After 1h pre-incubation, cells were removed from CO2 incubator and treated with 100 µl bacterial supernatant derived from strain 43042. Media control was used as control. Cells were then incubated for a further 24h at 37°C/5% CO2, after which cell-free supernatants were collected and spun down at 10,000g at 4°C for 3 min. Samples were aliquoted in 1.5 ml microtubes and stored in -80°C for hIL-6 and hIL-8 ELISA.

### Results

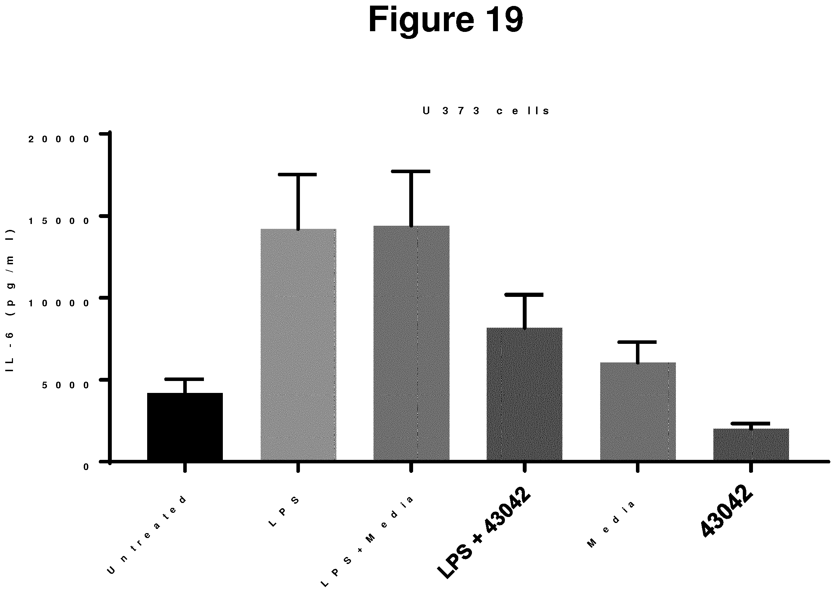

The results of these experiments are shown in Figure 19. Treatment of cells with LPS and the bacteria strain led to a decrease in the level of IL-6 secreted.

### Example 5 - activation of MAP2

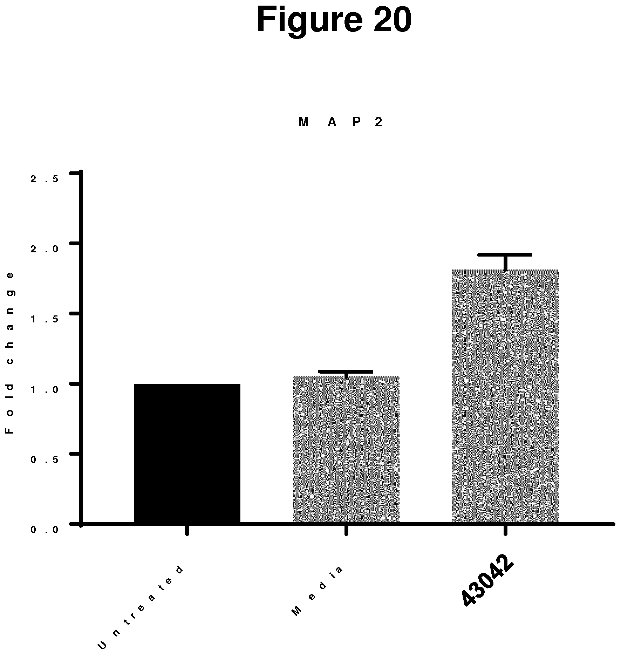

The effect of strain 43042 on expression of a gene associated with neuronal differentiation was investigated - Microtubule-associated protein 2 (MAP2). qPCR analysis revealed that MAP2 transcript was upregulated by 43042 above the media control samples in SH-SY5Y neuroblastoma cells (see Figure 20).

SH-SY5Y cells are a neuroblastoma cell line. The cells were grown in 50% MEM and 50% Nutrient Mixture F-12 Ham media supplemented with 2 mM L-Glutamine, 10% heat-inactivated FBS, 100 U/ml penicillin and 100 µg/ml streptomycin. SH-SY5Y cells were plated in 6-well plates at a density of 0.5 x 106 cells. After 24h, cells were treated in differentiation medium (growth medium containing 1% FBS without RA) with 10% bacterial supernatants or YCFA+ or 10uM RA for 24h. Cells were collected, and total RNA was isolated according to the RNeasy mini kit protocol (Qiagen). cDNA was made using the High Capacity cDNA reverse transcription kit (Applied Biosystems). Gene expression was measured by qPCR. GAPDH was used as internal control. Fold change was calculated according to the 2^(-ΔΔCₜ) method.

**List of primers used for in vitro gene expression analysis by qPCR.**

| **Gene ID** | **Forward sequence** | **Reverse sequence** |
|---|---|---|
| **ACTB** | GATCAAGATCATTGCTCCTC | TTGTCAAGAAAGGGTGTAAC |
| **GAPDH** | GGTATCGTGGAAGGACTCATG | ATGCCAGTGAGCTTCCCGTTC |
| **MAP2** | CTCAGCACCGCTAACAGAGG | CATTGGCGCTTCTCTCCTC |

### Example 6 - Effect on expression of functional markers in the intestine

### Summary

This study investigated the effect of *Bariatricus massiliensis* strain 43042 on the expression of tight junction proteins and functional markers in the intestine. Indoleamine 2,3 dioxygenase-1 (IDO1) is an enzyme that initiates tryptophan catabolism along a pathway that generates several bioactive kynurenine-based metabolites. Tryptophan hydroxylase-1 (TPH-1) catalyzes the formation of 5-hydroxy-L-tryptophan (5-HTP) from L-tryptophan, the first and rate-limiting step in the biosynthesis of 5-HT.

### Method

Male BALB/c mice received oral gavage (200µL volume) of 1 X 10⁹ CFU of the bacterial strain for 6 consecutive days. On day 7, the animals were euthanized. Intestinal tissue (1 cm segments of ileum and colon) were excised. Total RNA was extracted using the mirVana™ miRNA Isolation kit (Ambion/Llife technologies, Paisley, UK) and DNase treated (Turbo DNA-free, Ambion/life technologies) according to the manufacturer's recommendations. RNA was quantified using NanoDrop™ spectrophotometer (Thermo Fisher Scientific Inc., Wilmington, Delaware, USA) according to the manufacturer's instructions. RNA quality was assessed using the Agilent Bioanalyzer (Agilent, Stockport, UK) according to the manufacturer's procedure and an RNA integrity number (RIN) was calculated. RNA with RIN value >7 was used for subsequent experiments. RNA was reverse transcribed to cDNA using the Applied Biosystems High Capacity cDNA kit (Applied Biosystems, Warrington, UK) according to manufacturer's instructions. Briefly, Multiscribe Reverse Transcriptase (50 U/µL) was added as part of RT master mix, incubated for 25°C for 10 min, 37°C for 2 h, 85°C for 5 min and stored at 4°C. Quantitative PCR was carried out using probes (6 carboxy fluorescein - FAM) designed by Applied Biosystems to mouse specific targeted genes, while using β-actin as an endogenous control. Amplification reactions contained 1 µl cDNA, 5 µl of the 2X PCR Master mix (Roche), and 900 nM of each primer and were brought to a total of 10 µl by the addition of RNase-free water. All reactions were performed in triplicate using 96-well plates on the LightCycler®480 System. Thermal cycling conditions were as recommended by the manufacturer (Roche) for 55 cycles. To check for amplicon contamination, each run contained no template controls in triplicate for each probe used. Cycle threshold (Ct) values were recorded. Data was normalized using β-actin and transformed using the 2-ΔΔCT method and presented as a fold change vs. control group.

### Results

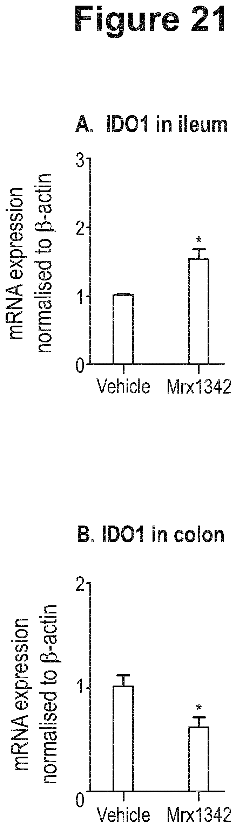

Of the genes tested, IDOl expression was different in mice who had received the bacterial strain compared with animals who received vehicle. There was a significant increase in the ileum (Fig. 21A) and a significant decrease in the colon (Fig. 21B). Expression of IDO1 in the colon in a mouse model of colitis is associated with disease amelioration [69]. Therefore, bacterial strains of the genus *Bariatricus* may be useful in the treatment or prevention of inflammatory bowel diseases.

### Example 7 - Effect on gene expression in the brain

### Summary

This study investigated the effect of *Bariatricus massiliensis* strain 43042 on the expression of certain genes of interest in the brain. mRNA levels for markers for the oxytocinergic system (oxytocin receptor and vasopressin receptor), endocrine system (mineralocorticoid (Nr3cl); glucocorticoid receptor (Nr3c2); corticosterone releasing factor (CRF) and receptors; Brain derived neurotrophic factor (BDNF)), immune system (11-6, TNF-α, TLR-4); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala, hippocampus and prefrontal cortex (PFC), which are key brain regions of the limbic system involved in emotional response.

### Materials and Methods

### Method

Male BALB/c mice received oral gavage (200µL volume) of 1 X 10⁹ CFU of the bacterial strain for 6 consecutive days. On day 7, the animals were euthanized. The brain was quickly excised, dissected and each brain region was snap-frozen on dry ice and stored at -80 °C for further analysis. mRNA expression was quantified as described in Example 6.

### Results

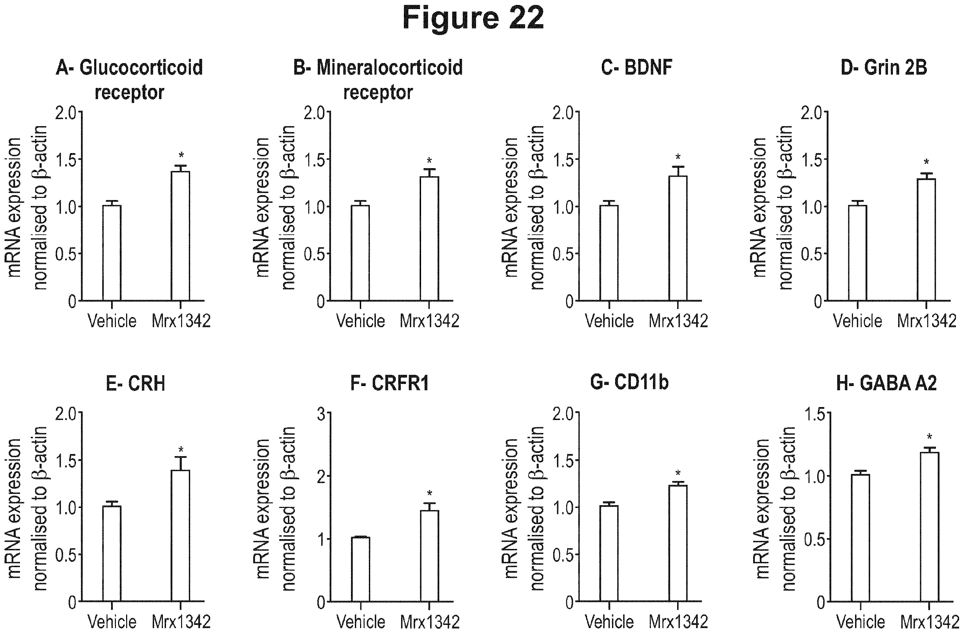

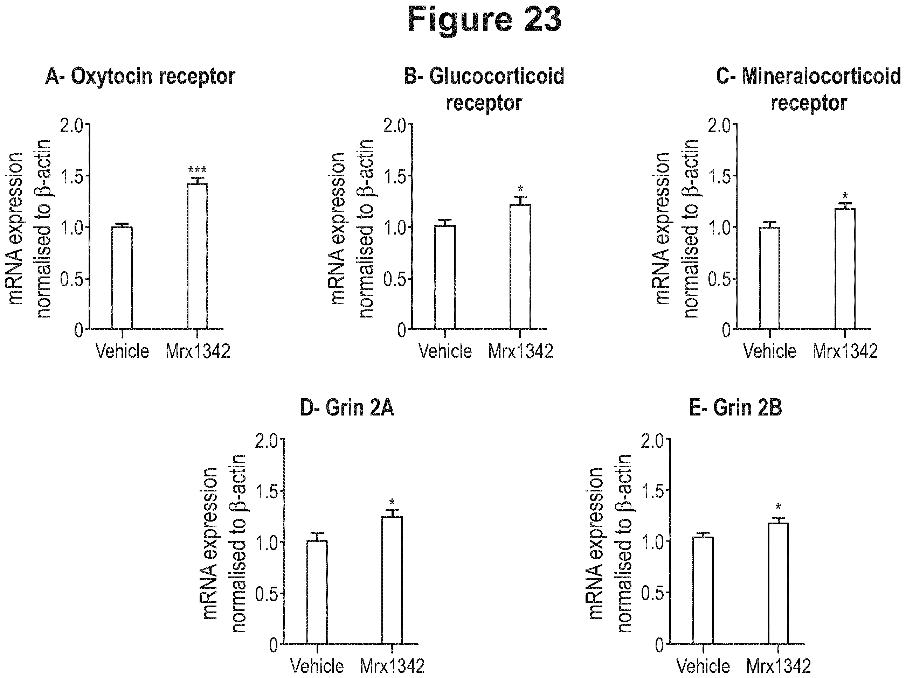

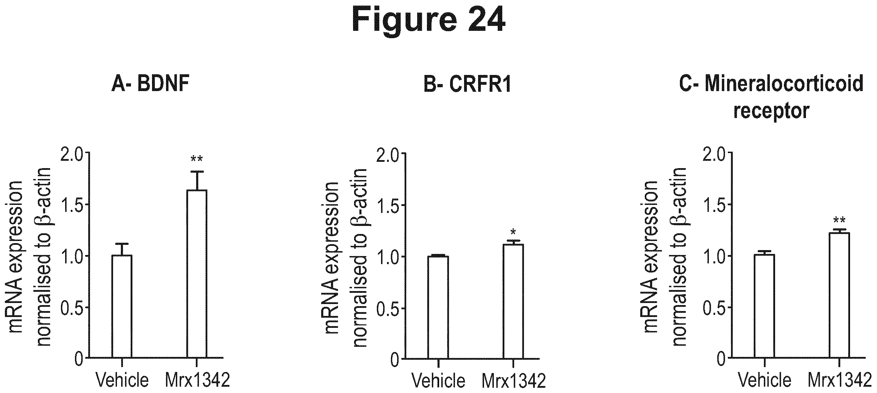

As shown in Figure 22, from the genes tested, a significant increase in the expression of glucocorticoid receptor (A), mineralocorticoid receptor (B), BDNF (C), Grin 2B (D), CRH (E), CFR1 (F), CD11b (G) and GABA A2 (H) in the hippocampus was observed. As shown in Figure 23, oxytocin receptor (A), glucocorticoid receptor (B), mineralocorticoid receptor (C), Grin 2A (D) and Grin 2B (E) exhibited significantly higher expression in the amygdala. In the prefrontal cortex, BDNF (A), CRFR1 (B) and mineralocorticoid receptor (C) were significantly upregulated as a result of treatment with the bacterium (Fig. 24).

This indicates that *Bariatricus* may be useful in the treatment or prevention of disorders or conditions that may benefit from the modulation in the levels of expression of these proteins in the brain, e.g. CNS diseases and disorders. In particular, BDNF and its receptor are essential for adult synaptic plasticity and the formation of memories [70]. A decrease in BDNF mRNA was observed in the hippocampus of individuals with Alzheimer's disease [71]. Meta-analysis studies also show that Alzheimer's disease patients have a reduced level of serum BDNF [72]. A decrease in BDNF protein was also observed in the caudate and putamen brain regions of patients suffering from Huntington's disease [73]. The bacterial strains of the invention may therefore be useful for treatment of neurodegenerative diseases, e.g. Alzheimer's and Huntington's disease.

### Sequences

### REFERENCES

[1] Spor et al. (2011) Nat Rev Microbiol. 9(4):279-90.
[2] Eckburg et al. (2005) Science. 10;308(5728):1635-8.
[3] Macpherson et al. (2001) Microbes Infect. 3(12):1021-35
[4] Macpherson et al. (2002) Cell Mol Life Sci. 59(12):2088-96.
[5] Mazmanian et al. (2005) Cell 15;122(1):107-18.
[6] Frank et al. (2007) PNAS 104(34):13780-5.
[7] Scanlan et al. (2006) J Clin Microbiol. 44(11):3980-8.
[8] Kang et al. (2010) Inflamm Bowel Dis. 16(12):2034-42.
[9] Machiels et al. (2013) Gut. 63(8):1275-83.
[10] WO 2013/050792
[11] WO 03/046580
[12] WO 2013/008039
[13] WO 2014/167338
[14] Goldin and Gorbach (2008) Clin Infect Dis. 46 Suppl 2:S96-100.
[15] Azad et al. (2013) BMJ. 347:f6471.
[16] Masco et al. (2003) Systematic and Applied Microbiology, 26:557-563.
[17] Srůtková et al. (2011) J. Microbiol. Methods, 87(1):10-6.
[18] Tang, et al. (2017) J Am Heart Assoc, 6(10).
[19] Wang et al. (2015) PNAS 112(9):2583-2858
[20] Psaty et al. (2003) JAMA, 289(19):2534-44
[21] Lancet. (1995) 346(8991-8992):1647-53
[22] Abdanipour, Schluesener, Tiraihi, & Noori-Zadeh, 2015 Neurol Res, 37(3), 223-228. doi:10.1179/1743132814Y.0000000438
[23] Leoni et al. (2005) Mol. Med., 11(1-12):1-15.
[24] Glauben, et al. (2006) Journal of Immunology, 176(8): 5015-5022
[25] Gasche et al 200 Inflammatory Bowel Diseases 6: 8-15
[26] Fahy (2009) ProcAm Thorac Soc 6.256-259
[27] Reddy et al (2008) J Clin. Invest. 118:2562-73
[28] Leng et al (2006) Exp. Hematol. 34:776-787
[29] Tao et al (2007) Nat. Med. 13:1299-1307
[30] Johansson and Ekman (2004) Blood 104:5075
[31] Song et al (2005) APMIS 113: 264-268
[32] Zimmerman et al (2007) Cancer Res 67: 9074-54
[33] Zhang et al (2005) Breast Cancer Res Treat 94: 11-16
[34] Abbas and Gupta (2008) Epigenetics 3: 300-309
[35] Minamiya et al (2011) Lung Cancer 74: 300-4
[36] Jung et al (2012) J Cell Biochem 113: 2167-2177
[37] Quint et al 2011 Virchows Arch 459: 129-139
[38] Cook et al (2014) Hum Mol Genet 23:104-106
[39] Simoes-Pires et al (2013) Mol Neurodegen 8: 7
[40] Miyamoto-Shinohara et al. (2008) J. Gen. Appl. Microbiol., 54, 9-24.
[41] Cryopreservation and Freeze-Drying Protocols, ed. by Day and McLellan, Humana Press.
[42] Leslie et al. (1995) Appl. Environ. Microbiol. 61, 3592-3597.
[43] Mitropoulou et al. (2013) J Nutr Metab. (2013) 716861.
[44] Kailasapathy et al. (2002) Curr Issues Intest Microbiol. 3(2):39-48.
[45] Handbook of Pharmaceutical Excipients, 2nd Edition, (1994), Edited by A Wade and PJ Weller
[46] Remington's Pharmaceutical Sciences, Mack Publishing Co. (A. R. Gennaro edit. 1985)
*[47]* Handbook of Microbiological Media, Fourth Edition (2010) Ronald Atlas, CRC Press.
*[48]* Maintaining Cultures for Biotechnology and Industry (1996) Jennie C. Hunter-Cevera, Academic Press
[49] Strobel (2009) Methods Mol Biol. 581:247-61.
[50] Gennaro (2000) Remington: The Science and Practice of Pharmacy. 20th edition, ISBN: 0683306472.
[51] Molecular Biology Techniques: An Intensive Laboratory Course, (Ream et al., eds., 1998, Academic Press).
*[52]* Methods In Enzymology (S. Colowick and N. Kaplan, eds., Academic Press, Inc.)
[53] Handbook of Experimental Immunology, Vols. I-IV (D.M. Weir and C.C. Blackwell, eds, 1986, Blackwell Scientific Publications)
[54] Sambrook et al. (2001) Molecular Cloning: A Laboratory Manual, 3rd edition (Cold Spring Harbor Laboratory Press).
[55] Handbook of Surface and Colloidal Chemistry (Birdi, K.S. ed., CRC Press, 1997)
[56] Ausubel et al. (eds) (2002) Short protocols in molecular biology, 5th edition (Current Protocols).
*[57]* PCR (Introduction to Biotechniques Series), 2nd ed. (Newton & Graham eds., 1997, Springer Verlag)
[58] Current Protocols in Molecular Biology (F.M. Ausubel et al., eds., 1987) Supplement 30
[59] Smith & Waterman (1981) Adv. Appl. Math. 2: 482-489
[60]West and Johnstone, (2014) J Clin Invest, 124, 30-39
[61] Glauben et al (2006) J Immunol. 176, 5015-5022
[62] Angiolilli et al. (2017) Ann Rheum Dis. 76, 277-285
[63] Gonneaud et al (2014). J Inflamm. 11: 43
[64] Alenghat et al. (2013). Nature. 504: 153-157
[65] Felice et al (2015). Ailment Pharmacol Ther. 41: 26-38
[66] Matthewson et al (2016) Nature Immunology 15: 505-513
[67] Baas, T (2013) SciBX 6: doi:10.1038/scibx.2013.1310
[68] Johansson and Ekman (2004) Blood 104:5075
[69] Coquerelle et al. (2009) Gut. 58: 1363-1373.
[70] Andero et al. (2014) Prog Mol Biol Transl Sci. 122:169-92.
[71] Philips et al. (1991) Neuron. 7:695-702.
[72] Ng et al. (2019) Int J Mol Sci. 20: pii: E257.
[73] Ferrer et al. (2000) Brain Res. 866:257-61.

## Claims

1. A composition comprising a bacterial strain of the genus *Bariatricus,* for use in therapy.

2. The composition for use according to claim 1, for use in the treatment or prevention of a disease or condition mediated by elevated histone deacetylase (HDAC) activity.

3. The composition for use according to any preceding claim, for use in the treatment or prevention of a disease or condition mediated by elevated Class I HDAC activity.

4. The composition for use according to any preceding claim, for use in a method of selectively inhibiting Class I HDAC activity in the treatment of a condition mediated by elevated Class I HDAC activity.

5. The composition for use according to any preceding claim, wherein the composition is for use in selectively inhibiting HDAC1, HDAC2 or HDAC3 in a disease or condition mediated by elevated HDAC1, HDAC2 or HDAC3 activity.

6. The composition for use according to any preceding claim, for use in a patient with elevated HDAC activity.

7. The composition for use according to any preceding claim, for use in the treatment or prevention of a disease or condition selected from the list consisting of: a neurodegenerative disease, such as Alzheimer's disease, Huntington's disease or Parkinson's disease; brain injury, such as stroke; a behavioural or psychiatric disorder, such as attention deficit hyperactivity disorder, obsessive compulsive disorder, anxiety disorder, bipolar disorder, or post-traumatic stress disorder; an inflammatory or autoimmune disease, such as asthma, arthritis, psoriasis, multiple sclerosis, diabetes, allograft rejection, graft-versus-host disease, or an inflammatory bowel disease, such as Crohn's disease; or cancer, such as prostate cancer, colorectal cancer, breast cancer, lung cancer, liver cancer or gastric cancer.

8. A composition comprising a bacterial strain of the genus *Bariatricus,* for use in the treatment or prevention of a disease or condition selected from the list consisting of: a neurodegenerative disease, such as Alzheimer's disease, Huntington's disease or Parkinson's disease, brain injury, such as stroke, an inflammatory or autoimmune disease, such as asthma, arthritis, psoriasis, multiple sclerosis, diabetes, allograft rejection, graft-versus-host disease, or an inflammatory bowel disease, such as Crohn's disease or ulcerative colitis; or cancer, such as prostate cancer, colorectal cancer, breast cancer, lung cancer, liver cancer or gastric cancer.

9. The composition for use of any preceding claim, wherein the bacterial strain is of the species *Bariatricus massiliensis.*

10. The composition for use of any preceding claim, wherein the bacterial strain has a 16s rRNA gene sequence that is at least 95%, 96%, 97%, 98%, 99%, 99.5% or 99.9% identical to SEQ ID NO:1 or SEQ ID NO:2, or wherein the bacterial strain has the 16s rRNA gene sequence represented by SEQ ID NO:1 or SEQ ID NO:2.

11. The composition for use of any preceding claim, wherein the composition is for oral administration.

12. The composition for use of any preceding claim, wherein the bacterial strain is lyophilised.

13. A food product comprising the composition of any preceding claim, for the use of any preceding claim.

14. A cell of the *Bariatricus massiliensis* strain deposited under accession number NCIMB 43042.

15. A cell of the *Bariatricus massiliensis* strain deposited under accession number NCIMB 43042 for use in therapy, preferably for use in the treatment or prevention of a disease or condition as defined in one of claims 2-8.

16. A cell of the *Bariatricus massiliensis* strain deposited under accession number NCIMB 43171 for use in therapy, preferably for use in the treatment or prevention of a disease or condition as defined in one of claims 2-8.

## Patentansprüche

1. Zusammensetzung, die einen Bakterienstamm der Gattung *Bariatricus* umfasst, zur Verwendung in der Therapie.

2. Zusammensetzung zur Verwendung nach Anspruch 1 zur Verwendung bei der Behandlung oder Vorbeugung einer Krankheit oder eines Zustands, die bzw. der durch erhöhte Histondeacetylase(HDAC)-Aktivität vermittelt wird.

3. Zusammensetzung zur Verwendung nach einem vorhergehenden Anspruch zur Verwendung bei der Behandlung oder Vorbeugung einer Krankheit oder eines Zustands, die bzw. der durch erhöhte Aktivität von HDAC der Klasse I vermittelt wird.

4. Zusammensetzung zur Verwendung nach einem vorhergehenden Anspruch zur Verwendung in einem Verfahren zur selektiven Hemmung der Aktivität von HDAC der Klasse I bei der Behandlung eines Zustands, der durch erhöhte Aktivität von HDAC der Klasse I vermittelt wird.

5. Zusammensetzung zur Verwendung nach einem vorhergehenden Anspruch, wobei die Zusammensetzung zur Verwendung bei enem selektiven Hemmen von HDAC1, HDAC2 oder HDAC3 bei einer Krankheit oder einem Zustand bestimmt ist, die bzw. der durch erhöhte Aktivität von HDAC1, HDAC2 oder HDAC3 vermittelt wird.

6. Zusammensetzung zur Verwendung nach einem vorhergehenden Anspruch zur Verwendung bei einem Patienten mit erhöhter Aktivität von HDAC.

7. Zusammensetzung zur Verwendung nach einem vorhergehenden Anspruch zur Verwendung bei der Behandlung oder Vorbeugung einer Krankheit oder eines Zustands, ausgewählt aus der Liste bestehend aus: einer neurodegenerativen Krankheit wie Morbus Alzheimer, Morbus Huntington oder Morbus Parkinson; Hirnverletzung wie Schlaganfall; einer Verhaltens- oder psychiatrischen Störung, wie Aufmerksamkeitsdefizit-Hyperaktivitätsstörung, Zwangsstörung, Angststörung, bipolarer Störung oder posttraumatischer Belastungsstörung; einer entzündlichen oder Autoimmunkrankheit wie Asthma, Arthritis, Psoriasis, Multiple Sklerose, Diabetes, Abstoßung von Allotransplantaten, Transplantat-gegen-Wirt-Krankheit oder einer entzündlichen Darmerkrankung wie Morbus Crohn; oder Krebs, wie Prostatakrebs, Darmkrebs, Brustkrebs, Lungenkrebs, Leberkrebs oder Magenkrebs.

8. Zusammensetzung, die einen Bakterienstamm der Gattung *Bariatricus* umfasst, zur Verwendung bei der Behandlung oder Vorbeugung einer Krankheit oder eines Zustands ausgewählt aus der Liste bestehend aus: einer neurodegenerativen Krankheit wie Morbus Alzheimer, Morbus Huntington oder Morbus Parkinson; Hirnverletzung wie Schlaganfall; einer entzündlichen oder Autoimmunkrankheit wie Asthma, Arthritis, Psoriasis, Multiple Sklerose, Diabetes, Abstoßung von Allotransplantaten, Transplantat-gegen-Wirt-Krankheit oder einer entzündlichen Darmerkrankung wie Morbus Crohn oder Colitis ulcerosa; oder Krebs, wie Prostatakrebs, Darmkrebs, Brustkrebs, Lungenkrebs, Leberkrebs oder Magenkrebs.

9. Zusammensetzung zur Verwendung nach einem vorhergehenden Anspruch, wobei es sich um einen Bakterienstamm der Spezies *Bariatricus massiliensis* handelt.

10. Zusammensetzung zur Verwendung nach einem vorhergehenden Anspruch, wobei der Bakterienstamm eine 16s-rRNA-Gensequenz aufweist, die zu mindestens 95 %, 96 %, 97 %, 98 %, 99 %, 99,5 % oder 99,9 % identisch mit SEQ ID NO:1 oder SEQ ID NO:2 ist, oder wobei der Bakterienstamm die 16s-rRNA-Gensequenz aufweist, die durch SEQ ID NO:1 oder SEQ ID NO:2 dargestellt ist.

11. Zusammensetzung zur Verwendung nach einem vorhergehenden Anspruch, wobei die Zusammensetzung zur oralen Verabreichung bestimmt ist.

12. Zusammensetzung zur Verwendung nach einem vorhergehenden Anspruch, wobei der Bakterienstamm lyophilisiert ist.

13. Lebensmittelprodukt, das die Zusammensetzung nach einem vorhergehenden Anspruch umfasst, zur Verwendung nach einem vorhergehenden Anspruch.

14. Zelle des Stammes *Bariatricus massiliensis,* hinterlegt unter der *Zugangsnummer* NCIMB 43042.

15. Zelle des Stammes *Bariatricus massiliensis,* hinterlegt unter der Zugangsnummer NCIMB 43042 zur Verwendung in der Therapie, vorzugsweise zur Verwendung bei der Behandlung oder Vorbeugung einer Krankheit oder eines Zustands, wie in einem der Ansprüche 2-8 definiert.

16. Zelle des Stammes *Bariatricus massiliensis,* hinterlegt unter der Zugangsnummer NCIMB 43171 zur Verwendung in der Therapie, vorzugsweise zur Verwendung bei der Behandlung oder Vorbeugung einer Krankheit oder eines Zustands, wie in einem der Ansprüche 2-8 definiert.

## Revendications

1. Composition comprenant une souche bactérienne du genre *Bariatricus,* destinée à être utilisée en thérapie.

2. Composition destinée à être utilisée selon la revendication 1, pour une utilisation dans le traitement ou la prévention d'une maladie ou d'une affection induite par une activité élevée de l'histone désacétylase (HDAC).

3. Composition destinée à être utilisée selon une quelconque revendication précédente, pour une utilisation dans le traitement ou la prévention d'une maladie ou d'une affection induite par une activité élevée de l'HDAC de classe I.

4. Composition destinée à être utilisée selon une quelconque revendication précédente, pour une utilisation dans un procédé consistant à inhiber de manière sélective l'activité de l'HDAC de classe I dans le traitement d'une affection induite par une activité élevée de l'HDAC de classe I.

5. Composition destinée à être utilisée selon une quelconque revendication précédente, dans laquelle la composition est destinée à être utilisée pour inhiber de manière sélective l'HDAC1, l'HDAC2 ou l'HDAC3 dans une maladie ou une affection induite par une activité élevée de l'HDAC1, de l'HDAC2 ou de l'HDAC3.

6. Composition destinée à être utilisée selon une quelconque revendication précédente, pour une utilisation chez un patient ayant une activité élevée de l'HDAC.

7. Composition destinée à être utilisée selon une quelconque revendication précédente, pour une utilisation dans le traitement ou la prévention d'une maladie ou d'une affection sélectionnée parmi la liste constituée d'une maladie neurodégénérative, comme la maladie d'Alzheimer, la maladie d'Huntington ou la maladie de Parkinson ; d'une lésion cérébrale, comme un accident vasculaire cérébral ; d'un trouble comportemental ou psychiatrique comme un trouble du déficit de l'attention avec hyperactivité, un trouble obsessionnel compulsif, un trouble de l'anxiété, un trouble bipolaire, ou un trouble de stress post-traumatique ; d'une maladie inflammatoire ou auto-immune, comme l'asthme, l'arthrite, le psoriasis, la sclérose en plaques, le diabète, le rejet d'allogreffe, la réaction du greffon contre l'hôte, ou une maladie inflammatoire de l'intestin, comme la maladie de Crohn ; ou d'un cancer, comme un cancer de la prostate, un cancer colorectal, un cancer du sein, un cancer du poumon, un cancer du foie ou un cancer gastrique.

8. Composition comprenant une souche bactérienne du genre *Bariatricus,* pour une utilisation dans le traitement ou la prévention d'une maladie ou d'une affection sélectionnée parmi la lise constituée d'une maladie neurodégénérative, comme la maladie d'Alzheimer, la maladie d'Huntington ou la maladie de Parkinson ; d'une lésion cérébrale, comme un accident vasculaire cérébral ; d'une maladie inflammatoire ou auto-immune, comme l'asthme, l'arthrite, le psoriasis, la sclérose en plaques, le diabète, le rejet d'allogreffe, la réaction du greffon contre l'hôte, ou une maladie inflammatoire de l'intestin, comme la maladie de Crohn, ou la colite ulcéreuse ; ou d'un cancer, comme un cancer de la prostate, un cancer colorectal, un cancer du sein, un cancer du poumon, un cancer du foie ou un cancer gastrique.

9. Composition destinée à être utilisée selon une quelconque revendication précédente, dans laquelle la souche bactérienne est de l'espèce *Bariatricus massiliensis.*

10. Composition destinée à être utilisée selon une quelconque revendication précédente, dans laquelle la souche bactérienne a une séquence du gène ARNr 16s qui est au moins 95 %, 96 %, 97 %, 98 %, 99 %, 99,5 % ou 99,9 % identique à SEQ ID NO: 1 ou SEQ ID NO: 2, ou dans laquelle la souche bactérienne a une séquence du gène ARNr 16s représentée par SEQ ID NO: 1 ou SEQ ID NO: 2.

11. Composition destinée à être utilisée selon une quelconque revendication précédente, dans laquelle la composition est prévue pour une administration orale.

12. Composition destinée à être utilisée selon une quelconque revendication précédente, dans laquelle la souche bactérienne est lyophilisée.

13. Produit alimentaire comprenant la composition selon une quelconque revendication précédente, destiné à être utilisé selon une quelconque revendication précédente.

14. Cellule de la souche *Bariatricus massiliensis* déposée sous le numéro NCIMB 43042.

15. Cellule de la souche *Bariatricus massiliensis* déposée sous le numéro NCIMB 43042 destinée à être utilisée en thérapie, de préférence pour une utilisation dans le traitement ou la prévention d'une maladie ou d'une affection telle que définie dans l'une des revendications 2 à 8.

16. Cellule de la souche *Bariatricus massiliensis* déposée sous le numéro NCIMB 43171 destinée à être utilisée en thérapie, de préférence pour une utilisation dans le traitement ou la prévention d'une maladie ou d'une affection telle que définie dans l'une des revendications 2 à 8.


### SEQUENCE LISTING

<110> 4D Pharma Research Limited
<120> COMPOSITIONS COMPRISING BACTERIAL STRAINS
<130> P073035WO
<140> EP....
   <141> 2019-06-25
<150> EP18179641.8
   <151> 2018-06-25
<150> GB1814836.1
   <151> 2018-09-12
<150> GB1905000.4
   <151> 2019-04-09
<160> 2
<170> SeqWin2010, version 1.0
<210> 1
   <211> 1398
   <212> DNA
   <213> Bariatricus massiliensis
<400> 1
   agctccctcc tttcggttgg gtcactgact tcgggcgtta ctgactccca tggtgtgacg  60
   ggcggtgtgt acaagacccg ggaacgtatt caccgcgaca ttctgattcg cgattactag  120
   cgattccagc ttcatgtagt cgagttgcag actacaatcc gaactgagac gttatttttg  180
   ggatttgctc cgcctcgcgg cctcgcttcc ctttgtttac gccattgtag cacgtgtgta  240
   gccctgatca taaggggcat gatgatttga cgtcatcccc accttcctcc aggttatccc  300
   tggcagtctc tctagagtgc ccaccttaaa tgctggctac taaagataag ggttgcgctc  360
   gttgcgggac ttaacccaac atctcacgac acgagctgac gacaaccatg caccacctgt  420
   ctccagtgcc ccgaagggaa agtacattac atactctgtc accgggatgt caagaccagg  480
   taaggttctt cgcgttgctt cgaattaaac cacatgctcc accgcttgtg cgggtccccg  540
   tcaattcctt tgagtttcat tcttgcgaac gtactcccca ggtggaatac ttactgcgtt  600
   tgctgcggca ccgaatggct ttgccacccg acacctagta ttcatcgttt acggcgtgga  660
   ctaccagggt atctaatcct gtttgctccc cacgctttcg agcatcaacg tcagtcatcg  720
   tccagtaagc cgccttcgcc actggtgttc ctcccaatat ctacgcattt caccgctaca  780
   ctgggaattc cacttacctc tccgacactc tagctgcata gtttccaaag cagtcccggg  840
   gttgagcccc gggctttcac tccagactta cacagccgtc tacgctccct ttacacccag  900
   taaatccgga taacgcttgc accatacgta ttaccgcggc tgctggcacg tatttagccg  960
   gtgcttctta gtcaggtacc gtcattttct tccctgctga tagagcttta cataccgaaa  1020
   tacttcttcg ctcacgcggc gtcgctgcat cagggtttcc cccattgtgc aatattcccc  1080
   actgctgcct cccgtaggag tttgggccgt gtctcagtcc caatgtggcc ggtcaccctc  1140
   tcaggtcggc tactgatcgt cgccttggtg ggccgttacc ccgccaacca gctaatcaga  1200
   cgcgggtcca tctcatacca ccggagtttt taccaccgca ccatgcggtg ctgtggtctt  1260
   atacggtatt agcagccatt tctaactgtt atccccttgt atgaggcagg ttacccacgc  1320
   gttactcacc cgtccgccac tcagtcacga aaaccttcct tccgaagaaa tcgagttcta  1380
   agtgcttcgt cgactgca                                                1398
<210> 2
   <211> 1233
   <212> DNA
   <213> Bariatricus massiliensis
<400> 2
   acccgggaac gtattcaccg cgacattctg attcgcgatt actagcgatt ccagcttcat   60
   gtagtcgagt tgcagactac aatccgaact gagacgttat ttttgggatt tgctccgcct  120
   cgcggcctcg cttccctttg tttacgccat tgtagcacgt gtgtagccct gatcataagg  180
   ggcatgatga tttgacgtca tccccacctt cctccaggtt atccctggca gtctctctag  240
   agtgcccacc ttaaatgctg gctactaaag ataagggttg cgctcgttgc gggacttaac  300
   ccaacatctc acgacacgag ctgacgacaa ccatgcacca cctgtctcca gtgccccgaa  360
   gggaaagtac attacatact ctgtcaccgg gatgtcaaga ccaggtaagg ttcttcgcgt  420
   tgcttcgaat taaaccacat gctccaccgc ttgtgcgggt ccccgtcaat tcctttgagt  480
   ttcattcttg cgaacgtact ccccaggtgg aatacttact gcgtttgctg cggcaccgaa  540
   tggctttgcc acccgacacc tagtattcat cgtttacggc gtggactacc agggtatcta  600
   atcctgtttg ctccccacgc tttcgagcat caacgtcagt catcgtccag taagccgcct  660
   tcgccactgg tgttcctccc aatatctacg catttcaccg ctacactggg aattccactt  720
   acctctccga cactctagct gcatagtttc caaagcagtc ccggggttga gccccgggct  780
   ttcactccag acttacacag ccgtctacgc tccctttaca cccagtaaat ccggataacg  840
   cttgcaccat acgtattacc gcggctgctg gcacgtattt agccggtgct tcttagtcag  900
   gtaccgtcat tttcttccct gctgatagag ctttacatac cgaaatactt cttcgctcac  960
   gcggcgtcgc tgcatcaggg tttcccccat tgtgcaatat tccccactgc tgcctcccgt 1020
   aggagtttgg gccgtgtctc agtcccaatg tggccggtca ccctctcagg tcggctactg 1080
   atcgtcgcct tggtgggccg ttaccccgcc aaccagctaa tcagacgcgg gtccatctca 1140
   taccaccgga gtttttacca ccgcaccatg cggtgctgtg gtcttatacg gtattagcag 1200
   ccatttctaa ctgttatccc cttgtatgag gca                              1233